# EUROPEAN PATENT APPLICATION

(11) **EP 2 484 374 A1**
(43) Date of publication of application: **08.08.2012**
(21) Application number: 11186052.4
(22) Date of filing: 18.07.2005
(51) Int. Cl.: A61K 39/00, A61P 35/00

(54) **Immuno stimulating complex and oligonucleotide formulations for inducing enhanced interferon-gamma responses**

(30) Priority: 18.07.2004 US 589259 P
(62) Divisional of application: 05858509.2
(71) Applicant: CSL Limited, Parkville, VIC 3437 (AU); Coley Pharmaceutical Group, Ltd., Ottawa, Ontario K2K 3A2 (CA)
(72) Inventor: Davis, Heather, L., Durobin, Ontario K0A 1T0 (CA); McCluskie, Michael, J., Ottawa, Ontario K1K 4V3 (CA); Drane, Deborah, P., Sunbury, Victoria 3429 (AU)
(74) Representative: Pohlman, Sandra M.

(57) **Abstract**

Vaccine compositions comprising (a) an oligonucleotide, (b) and immune stimulating complex and (c) an antigen induce a strong interferon-gamma immune response. Both oligonucleotides containing immune stimulatory motifs and oligonucleotides lacking immune stimulatory motifs contribute to an interferon-gamma response when administered with an immune stimulating complex.

## Description

### CROSS-REFERENCE TO RELATED PATENT APPLICATIONS

This application claims benefit of U.S. patent application No. 60/589,259, filed 07/18/2004, which is incorporated in its entirety herein by reference.

### FIELD OF THE INVENTION

The present invention relates to formulations comprising immune stimulating complexes and immunostimulatory oligonucleotides, and to the use of such formulations in vaccine therapies.

### BACKGROUND OF THE INVENTION

Bacterial DNA has immune stimulatory effects to activate B cells and natural killer cells, but vertebrate DNA does not (Tokunaga, T., et al., 1988. Jpn. J. Cancer Res. 79:682-686; Tokunaga, T., et al., 1984, JNCI 72:955-962; Messina, J.P., et al., 1991, J. Immunol. 147:1759-1764; and reviewed in Krieg, 1998, In: Applied Oligonucleotide Technology, C.A. Stein and A.M. Krieg, (Eds.), John Wiley and Sons, Inc., New York, NY, pp. 431-448). It is now understood that these immune stimulatory effects of bacterial DNA result from the presence of unmethylated CpG dinucleotides in particular base contexts (CpG motifs). Such motifs are common in bacterial DNA, but are methylated and underrepresented in vertebrate DNA (Krieg et al, 1995 Nature 374:546-549; Krieg, 1999 Biochim. Biophys. Acta 93321:1-10). The immune stimulatory effects of bacterial DNA can be mimicked with synthetic oligonucleotides (ODN) containing CpG motifs. Such CpG ODN have highly stimulatory effects on human and murine leukocytes, such as inducing B cell proliferation, cytokine and immunoglobulin secretion, natural killer (NK) cell lytic activity, and IFN-γ secretion; and activating dendritic cells (DCs) and other antigen presenting cells to express co-stimulatory molecules and to secrete cytokines, especially the Th1-like cytokines that are important in promoting Th1-like T cell responses. These immune stimulatory effects of native phosphodiester backbone CpG ODN are highly CpG specific, in that the effects are dramatically reduced if the cytosine residue of the CpG motif is methylated or if the CpG motif is changed to a GpC or otherwise eliminated or altered (Krieg et al, 1995 Nature 374:546-549; Hartmann et al, 1999 Proc. Natl. Acad. Sci USA 96:9305-10).

CpG immunostimulatory oligonucleotides have also been reported to enhance the effects of adjuvants in a vaccine setting. U.S. Patent No. 6,406,705 B 1 described the combined use of CpG oligonucleotides, non-nucleic acid adjuvants and an antigen to induce an antigen-specific immune response. The non-nucleic acid adjuvants included adjuvants that create depot effects, adjuvants that stimulate the immune system and adjuvants having both of those activities. The patent did not teach or suggest the use of non-CpG oligonucleotides with non-nucleic acid adjuvants.

### SUMMARY OF THE INVENTION

The invention is based in part on the unexpected finding that a combination of previously identified immunostimulatory oligonucleotides (such as but not limited to CpG oligonucleotides) and immune stimulating complexes (e.g., ISCOM^{®} and ISCOMATRIX^{®} adjuvant) stimulate far greater levels of interferon-gamma (IFN-gamma) than combinations of immunostimulatory oligonucleotide with other non-nucleic acid adjuvants. Production of IFN-gamma is useful both per se and as a stage in the process of generating antigen-specific immune responses, particularly in the treatment of infectious disease and cancer. Consequently, the invention provides methods and compositions relating to induction of IFN-gamma mediated antigen-specific immune responses via formulations that (a) comprise immunostimulatory oligonucleotides and immune stimulating complexes and (b) are administered in conjunction with antigens.

The invention is further based in part on the observation that oligonucleotides previously reported not to be immunostimulatory actually have immunostimulatory capability when combined with immune stimulating complexes in a vaccine therapy. This observation suggests that the lack of immunostimulation previously observed was due to inefficient delivery of the oligonucleotides to target cells and receptors (e.g., the TLR family of receptors). Thus, the invention transforms a number of previously-characterized immunologically inert oligonucleotides into immunostimulatory oligonucleotides.

In one aspect, therefore, the invention provides a medicament for an antigen-specific immune response, preferably an enhanced interferon-gamma response. The medicament comprises an oligonucleotide component, an immune stimulating complex component and an antigen component. In certain embodiments, the oligonucleotide component comprises one or more CpG motifs. In other embodiments, the oligonucleotide components comprise one or more non-CpG motifs. In still other embodiments, the oligonucleotide components lack any CpG motifs or other known immunostimulatory motifs. Preferably, the immune stimulating complex comprises saponin and sterol. Also preferably, at least two components of the medicament are mixed together prior to administration of the medicament.

In another aspect, the invention provides a method for inducing an antigen-specific immune response, preferably an enhanced interferon-gamma response by administering the inventive medicament. The method may include steps of (a) obtaining an oligonucleotide component, an immune stimulating complex component and an antigen component, and (b) administering the three components, separately or in any combination thereof, to a patient. The method may further include a step of measuring the patient's interferon-gamma response. Patients include any vertebrate subjects receiving the vaccine. Preferably, the patient is a human, but could be a dog, cat, horse, cow, pig, turkey, goat, fish, monkey, chicken, rat, mouse or sheep.

In preferred embodiments, the antigen-specific immune response induced by the invention comprises enhanced IFN-gamma production. The antigen-specific immune response may include a cellular immune response, and therefore may include induction of CD8+ cytotoxic T lymphocytes. The antigen-specific immune response also may comprise a humoral response, and therefore may include induction of antigen-specific Th1- or Th2-induced immunoglobulin.

In some embodiments, the oligonucleotide is incorporated into the immune stimulating complex. In other embodiments, the oligonucleotide is simply associated (e.g., non-covalently and non-ionically) with the complex.

In some embodiments, the antigen is incorporated into the immune stimulating complex. In other embodiments, the antigen is simply associated with the complex.

As used herein, a formulation comprising an oligonucleotide and an immune stimulating complex is referred to as an oligonucleotide/immune stimulating complex formulation. As used herein, a formulation comprising an antigen and an immune stimulating complex is referred to as an immune stimulating complex/antigen formulation. As used herein, a formulation comprising an oligonucleotide, an antigen and an immune stimulating complex is referred to as an oligonucleotide/immune stimulating complex/antigen formulation.

In some embodiments, a formulation of the medicament is made by mixing together the oligonucleotide and the immune stimulating complex. The antigen may also be included. In some embodiments, the oligonucleotide comprises a moiety that is incorporated within the immune stimulating complex, such as a sterol (e.g., cholesterol), a lipidated tag (e.g. palmitic, oleic) or a saponin. The oligonucleotide may then be incorporated into the complex by virtue of the moiety that forms part of the immune stimulating complex

Thus, the oligonucleotide immune stimulating complex (ISC) and antigen may themselves be formulated together, or alternatively they may be formulated apart. If formulated together, the antigen immune stimulating complex and oligonucleotide may each be present in a proportion of complex (e.g., at least 1%, at least 10%, at least 25%, at least 40%, at least 50%, at least 75%, at least 80%, at least 90%, at least 95%, at least 99%, or all complexes). If formulated apart, they may be administered at the same location or at different locations. If administered at different locations, the locations preferably lead to the same draining lymph node. In some embodiments, the components, either together, separate or partially combined are administered intramuscularly or subcutaneously. In other embodiments, the components are administered mucosally such as but not limited to orally, sublingual, intranasally, intrapulmonary, rectally and intravaginally.

Preferably, the three components of the inventive medicament are administered simultaneously.

Antigens of the invention may be provided as isolated antigens, cell extracts (e.g., bacterial cell extracts, viral extracts, fungal extracts, mycobacterial extracts), attenuated whole cell vaccines, whole inactivated cell vaccines, dendritic cell vaccines or DNA vaccines. Isolated antigens may be peptide, lipid, glycolipid or carbohydrate in nature, or combinations thereof, although they are not so limited.

In some embodiments, the oligonucleotide is an immunostimulatory oligonucleotide. In preferred embodiments, the immunostimulatory oligonucleotide is a CpG oligonucleotide, a T-rich oligonucleotide, a poly-G oligonucleotide, or a phosphorothioate oligonucleotide. CpG oligonucleotides comprise an unmethylated CpG dinucleotide motif. CpG oligonucleotides may be A class CpG oligonucleotides, B class CpG oligonucleotides, or C class CpG oligonucleotides.

The oligonucleotide may be a non-CpG immunostimulatory oligonucleotide. Non-CpG oligonucleotides lack an unmethylated CpG dinucleotide motif. Thus, non-CpG oligonucleotides include nucleotides that comprise a methylated CpG motif, T-rich oligonucleotides, poly-G oligonucleotides and phosphorothioate oligonucleotides.

The oligonucleotide also may be an oligonucleotide that lacks known immunostimulatory motifs, such as those recited above, and thus would have been considered immunologically inert prior to the present invention. Such oligonucleotides are referred to herein as "inert oligonucleotides."

In some embodiments, the oligonucleotide has a partially or wholly modified phosphate backbone, such as a backbone that is partially or wholly phosphorothioate.

Methods of the invention can be directed to various vaccine settings, including subjects having or at risk of having various conditions or diseases. In some embodiments, the patient has or is at risk of developing a cancer. Such a patient might be administered a cancer antigen and/or a microbial antigen, depending on whether the antigen-specific immune response is intended to treat the cancer or an infectious disease in the subject. Opportunistic infectious diseases are common in immunocompromised patients, such as cancer patients undergoing anti-cancer treatment. In some embodiments, the cancer is a carcinoma or a sarcoma.

The cancer may be selected from the group consisting of biliary tract cancer, breast cancer, cervical cancer, choriocarcinoma, colon cancer, endometrial cancer, gastric cancer, intraepithelial neoplasm, liver cancer, lung cancer (e.g. small cell and non-small cell cancer), lymphoma, melanoma, neuroblastoma, oral cancer, ovarian cancer, pancreatic cancer, prostate cancer, rectal cancer, renal cancer and thyroid cancer. In some important embodiments, the cancer is selected from the group consisting of bone cancer, brain and CNS cancer, connective tissue cancer, esophageal cancer, eye cancer, Hodgkin's lymphoma, larynx cancer, oral cavity cancer, skin cancer and testicular cancer. The cancer also may be selected from the group consisting of melanoma, prostate cancer, breast cancer and colorectal cancer, and in some related embodiments, the cancer antigen is a melanoma antigen (e.g., the MAGE family of antigen), a prostate cancer antigen (e.g., PSMA), a breast cancer antigen (e.g., HER2) or a colorectal cancer antigen (e.g., APC), respectively.

Cancer antigens include MART-1/Melan-A, gp100, adenosine deaminase-binding protein (ADAbp), FAP, cyclophilin b, colorectal associated antigen (CRC)-C017-1A/GA733, carcinoembryonic antigen (CEA), CAP-1, CAP-2, etv6, AML1, prostate specific antigen (PSA), PSA-1, PSA-2, PSA-3, prostate-specific membrane antigen (PSMA), T-cell receptor/CD3-zeta chain, or CD20.

Cancer antigens also include MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4 and MAGE-C5.

Cancer antigens further include GAGE-1, GAGE-2, GAGE-3, GAGE-4, GAGE-5, GAGE-6, GAGE-7, GAGE-8 and GAGE-9.

Cancer antigens still further include BAGE, RAGE, LAGE-1, NAG, GnT-V, MUM-1, CDK4, tyrosinase, p53, MUC family, HER2/neu, p21ras, RCAS1, α-fetoprotein, E-cadherin, α-catenin, β-catenin, γ-catenin, p120ctn, gp100^{Pmel117}, PRAME, NY-ESO-1, cdc27, adenomatous polyposis coli protein (APC), fodrin, Connexin 37, Ig-idiotype, p15, gp75, GM2 ganglioside, GD2 ganglioside, human papilloma virus proteins, Smad family of tumor antigens, lmp-1, P1A, EBV-encoded nuclear antigen (EBNA)-1, brain glycogen phosphorylase, SSX-1, SSX-2 (HOM-MEL-40), SSX-1, SSX-4, SSX-5, SCP-1 and CT-7 and c-erbB-2.

Cancer antigens also CD20, CD22, CD52, CD33, CD10 (gp100), CD3/T-cell receptor (TCR), CD79/B-cell receptor (BCR), CD26, Human leukocyte antigen (HLA)-DR, HLA-DP, and HLA-DQ, RCAS 1, Prostate specific membrane antigen (PSMA), PSA, EGFR/HER1/erbB1, EGFRvIII, erbB2/HER2/neu), erbB3/HER3, erbB4/HER4m Tyrosinase, Melan-A/MART-1, tyrosinase related protein (TRP)-1/gp75, Polymorphic epithelial mucin (PEM), Human epithelial mucin (MUC1), α-fetoprotein, Kallikreins 6 and 10, Gastrin-releasing peptide/bombesin, Prostate specific antigen, and a cancer testis (CT) antigen.

In some embodiments of the invention, the patient has or is at risk of developing an infection. The infection may be selected from the group consisting of a bacterial infection, a viral infection, a fungal infection, a parasitic infection and a mycobacterial infection. In one embodiment, the infection is a chronic viral infection such as but not limited to hepatitis B infection, hepatitis C infection, HIV infection, HSV infection or HPV infection. In some embodiments, the parasite infection is an intracellular parasite infection. In other embodiments, the parasite infection is a non-helminthic parasite infection. Other examples of microbial infections are recited herein. Depending on the subject to be treated, the antigen is a bacterial antigen, a viral antigen, a fungal antigen, a parasitic antigen or a mycobacterial antigen. In some embodiments, the antigen is a prion.

In other embodiments, the patient has or is at risk of developing an allergy or asthma and the antigen is an allergen.

Methods of the invention may be performed in conjunction with a therapeutic regimen, such as surgery, radiation or chemotherapy. Chemotherapy may be but is not limited to anti-cancer agents, anti-bacterial agents, anti-viral agents, anti-fungal agents, anti-parasite agents, anti-mycobacterial agents, anti-allergy agents and anti-asthma agents. In embodiments directed toward treatment of subjects having or at risk of developing cancer, the methods may further comprise administration of interferon-alpha, either within or separate from formulations of the invention.

In another aspect, the invention provides a method of inducing an antigen-specific immune response comprising contacting an immune cell with a medicament of the invention in an amount effective to activate the immune cell. The immune cell may be a lymphocyte, such as a B or T cell, an antigen presenting cell, such as a dendritic cell, or a natural killer (NK) cell. The activation can be performed *in vivo, in vitro* or ex *vivo,* i.e., by isolating an immune cell from the subject, contacting the immune cell with the formulations, and re-administering the activated immune cell to the subject.

Each aspect of the invention can encompass various limitations and embodiments. Thus, each limitation of the invention involving any one element or combination of elements can be included in other aspects and embodiments of the invention. This invention is not limited in its application to the details of construction and the arrangement of components set forth herein. Rather, the invention includes embodiments not specifically detailed, and can be practiced in various ways.

The phraseology and terminology used herein is for the purpose of description and should not be regarded as limiting. The use herein of "including", "comprising", "having", "containing", "involving", and variations thereof, is meant to encompass the items listed thereafter and equivalents thereof as well as additional items.

### BRIEF DESCRIPTION OF THE DRAWINGS

The Figures are illustrative only and are not required for enablement of the invention disclosed herein.

FIG. 1 is a bar graph depicting the effect of different adjuvants on interferon-gamma (IFN-g) levels (SC immunization).

FIG. 2 is a bar graph depicting the effect of different adjuvants on interferon-gamma (IFN-g) levels (IM immunization).

FIG. 3 is a bar graph depicting the effect of different oligonucleotides on interferon-gamma (IFN-g) levels (SC immunization).

FIG. 4 is a bar graph depicting the effect of different oligonucleotides on interferon-gamma (IFN-g) levels (SC immunization).

FIG. 5 is a graph depicting the effect of different adjuvants on total IgG titers of anti-HBs (SC immunization).

FIG. 6 is a graph depicting the effect of different adjuvants on HBsAg specific CTL response (SC immunization).

FIG. 7 is a bar graph depicting the effect of different adjuvants on interferon-gamma (IFN-g) levels in the presence and absence of alum.

FIG. 8 is a bar graph depicting the effect of different adjuvants on interferon-gamma (IFN-g) levels in the presence and absence of alum.

FIG. 9 is a diagram showing results of vaccine therapy for B16-OVA melanoma using oligonucleotides formulated with an immune stimulating complex.

FIG. 10 is a diagram showing results of immunotherapy of B16-OVA melanoma.

FIG. 11 is a graph showing induction of OVA-specific CTL in vaccinated animals.

FIG. 12 is a graph showing OVA-specific IFN-gamma secretion by splenocytes in vaccinated animals.

FIG. 13 is a graph showing OVA-specific CD8+ T cells in splenocytes using OVA-loaded pentamers.

FIG. 14 is a graph showing survival data for animals with induced cervical cell carcinoma and vaccinated with E6/E7 peptide antigens.

FIG. 15 is a graph showing tumor volume data for animals with induced cervical cell carcinoma and vaccinated with E6/E7 peptide antigens.

### DETAILED DESCRIPTION OF THE PREFERRED EMBODIMENTS

The invention relates broadly to particular formulations as unexpectedly efficient delivery vehicles for oligonucleotides and antigens. The formulations comprise immune stimulating complexes (e.g., ISCOM^{®} or ISCOMATRIX^{®} adjuvant). The invention is premised in part on the unexpected discovery that immune stimulating complexes are a particularly effective vehicle for delivery of oligonucleotides previously characterized as immunostimulatory as well as those previously characterized as immunologically inert. Although not intending to be bound by any particular mechanism, it is postulated that the immune stimulating complexes enhance delivery of both types of oligonucleotide to particular receptors (e.g., the TLR family of receptors) and/or to particular cells irrespective of receptor involvement. This has resulted in the observed enhancement of Thl-biased antigen-specific immune responses when the oligonucleotide formulation is administered in a vaccine setting.

The synergy observed for the oligonucleotide and immune stimulating complex combination (in a vaccine setting) was much greater than the level of synergy previously observed for immunostimulatory oligonucleotide and other non-nucleic acid adjuvants (in a vaccine setting). This difference was completely unexpected. It was further unexpected that use of immune stimulating complexes could essentially transform previously-characterized immunologically inert oligonucleotides into immunostimulatory oligonucleotides. This latter observation broadens the genus of oligonucleotides that can be used for immunostimulatory purposes to include oligonucleotides with no previously characterized immunostimulatory motif.

As described in the Examples in greater detail, it has been observed according to the invention that in a murine vaccine model, co-administration of an immune stimulating complex (ISCOMATRIX^{®} adjuvant) with a CpG immunostimulatory oligonucleotide having the sequence TCG TCG TTT TGT CGT TTT GTC GTT (SEQ ID NO: 1) (ODN 7909) or with an "inert" oligonucleotide having the sequence TGC TGC TTT TGT GCT TTT GTG CTT (SEQ ID NO: 2) (ODN 2137) resulted in much higher levels of IFN-gamma than either oligonucleotide or the complex tested alone. A synergy between immune stimulating complexes and immunostimulatory or "inert" oligonucleotides was observed after subcutaneous (SC) and intramuscular (IM) injection.

Subcutaneous (SC) injection of a CpG immunostimulatory oligonucleotide (having sequence TCG TCG TTT TGT CGT TTT GTC GTT (SEQ ID NO: 1) (ODN 7909) and an immune stimulating complex and Hepatitis B surface antigen (HbsAg) gave significantly greater anti-HBs IgG levels compared to either adjuvant alone and to the combination of the CpG oligonucleotide with other non-nucleic acid adjuvants. The addition of immune stimulating complexes to a non-CpG oligonucleotide (having sequence TGC TGC TTT TGT GCT TTT GTG CTT (SEQ ID NO: 2) (ODN 2137) that was previously characterized as low or non-stimulatory also significantly increased anti-HBs IgG levels compared to either adjuvant alone.

Co-administration of immune stimulating complexes with CpG immunostimulatory oligonucleotides also greatly enhanced the cytolytic T lymphocyte (CTL) activity compared to either adjuvant alone and compared to combinations of CpG immunostimulatory oligonucleotides with other non-nucleic acid adjuvants. A similar result was observed for "inert" oligonucleotides.

Thus, the addition of immune stimulating complexes to immunostimulatory or "inert" oligonucleotides results in the induction of strong Thl-biased antigen-specific immune responses, as indicated by IFN-gamma production and CTL activation. These findings are unexpected at least in part because of the immunologically "inert" character of some of the oligonucleotides tested. These findings are also unexpected because the observed synergy was greatest with the combination of oligonucleotides and immune stimulating complexes, as compared to other adjuvant combinations.

These findings indicate that formulations comprising immune stimulating complexes and oligonucleotides are useful in optimizing vaccine therapies, such as but not limited to those directed to infectious disease, cancers, allergy and asthma.

### Immune stimulating complexes:

Immune stimulating complexes are particles having a diameter ranging in size from 10 nm to 100 nm, and more commonly from 30 nm to 50 nm, and comprised of glycosides and sterols, which form a matrix on which antigens may multimerize. The complexes can function as adjuvants as well as antigen delivery systems. As adjuvants, they are capable of stimulating the immune system. Using animal models, these complexes have been shown to enhance cellular and humoral immune responses to a number of antigens, including influenza virus, hepatitis C virus and human papilloma virus antigens.

Immune stimulating complexes contain glycosides such as Quillaia saponins, sterols (such as cholesterol) and preferably phospholipids (such as but not limited to phosphatidylcholine and phosphatidyl ethanolamine). Preferably, the glycoside is ISCOPREP® saponin which is a purified saponin fraction derivable from Quil A, which is obtained form the bark of the *Quillaja saponaria* tree. Immune stimulating complex formation is described in greater detail in EP 109942 A and EP 231039 A. Immune stimulating complexes can also be prepared as described in U.S. Patent No. 5,178,860. The entire contents of those references are incorporated herein by reference.

The invention embraces the use of a range of immune stimulating complexes including antigen-containing and non-antigen-containing complexes. ISCOM® and ISCOMATRIX® adjuvant are examples of immune stimulating complexes that can be prepared at research scale using well known techniques described in the literature (Morein et al., 1989, In: Vaccines: Recent trends and Progress, Gergoriadis et al. (Eds.), Plenum Press, New York, pp. 153-161; Cox et al., 1997, In: Vaccine Design: The Role of Cytokine Networks, Gergoriadis et al, (Eds.), Plenum Press, New York, pp. 33-49; Coulter et al., 1998, Vaccine 16:1243-1253). ISCOM® and ISCOMATRIX® adjuvant can also be prepared at large scale using well known techniques described in the literature (Kersten et al., 2004, In: Novel Vaccination Startegies, Kaufmann (Ed.), WILEY-VCH, Germany).

### Formulations:

Oligonucleotides and antigens, together or separately, can be formulated with immune stimulating complexes in any number of ways. For example, the oligonucleotides can simply be mixed with the immune stimulating complexes in the presence or absence of an antigen. Alternatively, the oligonucleotides can themselves be part of the matrix of the complex, for example by contributing one or more of the components of the matrix. As an example, the oligonucleotide may be conjugated to a sterol such as cholesterol. The oligonucleotide, by connection to the sterol, then can become part of the matrix of the complex. The oligonucleotide may also be conjugated to other substances, such as hydrophobic molecules (e.g., palmitic acid, oleic acid, linoleic acid, and the like). Oligonucleotides conjugated in this manner may then be incorporated into the complex with the amphilphilic molecule contributing to the matrix of the complex.

Antigens may similarly be mixed or incorporated into the complexes.

With respect to oligonucleotide formulations, the ratio of oligonucleotide to immune stimulating complex can range from 100:1 1 to 1:100. In preferred embodiments, the ratio is 1:1, 3:1, 10:1 or 20:1.

With respect to antigen formulations, the ratio of antigen to immune stimulating complex can range from 100:1 1 to 1:100. In preferred embodiments, the ratio is 1:10 to 10:1.

### Immunostimulatory Oligonucleotides Generally:

The invention embraces the use of a broad range of oligonucleotides. Some of these have been previously characterized as immunostimulatory and/or contain previously characterized immunostimulatory motifs (as described below). In general, Immunostimulatory oligonucleotides are oligonucleotides that demonstrate immunostimulatory potential even in the absence of an immune stimulating complex. Examples include CpG immunostimulatory oligonucleotides containing unmethylated as well as methylated CpG dinucleotide motifs, T-rich and poly-T immunostimulatory oligonucleotides, poly-G immunostimulatory oligonucleotides and phosphorothioate immunostimulatory oligonucleotides. Each of these is discussed in greater detail below.

A second category of oligonucleotide embraced by the invention is oligonucleotides previously characterized as immunologically inert, particularly in a vaccine setting. Examples include oligonucleotides that do not comprise any known immunostimulatory motif and oligonucleotides that have not previously been observed to have Th1 immunostimulatory potential. As used herein, this latter category of oligonucleotides is sometimes referred to herein as "inert" oligonucleotides. Combination of this class of oligonucleotides with immune stimulating complexes transforms them into immunostimulatory oligonucleotides. The change in activity may be due to efficient delivery and uptake of these oligonucleotides to and by particular receptors and cells.

As mentioned above, the immunostimulatory oligonucleotides contain specific sequences previously demonstrated to elicit an immune response. These specific sequences are referred to as "immunostimulatory motifs," and the oligonucleotides that contain at least one immunostimulatory motif are referred to as "immunostimulatory oligonucleotides." In some embodiments, the immunostimulatory motif is preferably an "internal immunostimulatory motif." The term "internal immunostimulatory motif" refers to the position of the motif sequence within a longer nucleic acid sequence, which is longer in length than the motif sequence by at least one nucleotide linked to both the 5' and 3' ends of the immunostimulatory motif sequence.

The invention embraces oligonucleotides that are DNA or RNA in nature. As a result, the term "oligonucleotides" refers to both oligodeoxynucleotides (DNA) and oligoribodeoxynucleotides (RNA).

### CpG Oligonucleotides:

Immunostimulatory oligonucleotides, in some instances, include unmethylated CpG immunostimulatory motifs. Such oligonucleotides are referred to as CpG oligonucleotides. A CpG oligonucleotide as used herein refers to an immunostimulatory CpG oligonucleotide, and accordingly these terms are used interchangeably unless otherwise indicated. Methylation status of the CpG immunostimulatory motif generally refers to the cytosine residue in the dinucleotide. An immunostimulatory oligonucleotide containing at least one unmethylated CpG dinucleotide is an oligonucleotide which contains a 5' unmethylated cytosine linked by a phosphate bond to a 3' guanine, and which activates the immune system. An immunostimulatory oligonucleotide containing at least one methylated CpG dinucleotide is an oligonucleotide which contains a 5' methylated cytosine linked by a phosphate bond to a 3' guanine, and which activates the immune system. CpG immunostimulatory oligonucleotides may comprise palindromes that in turn may encompass the CpG dinucleotide.

CpG oligonucleotides have been described in a number of issued patents, published patent applications, and other publications, including U.S. Patent Nos. 6,194,388; 6,207,646; 6,214,806; 6,218,371; 6,239,116; and 6,339,068.

In other embodiments, the immunostimulatory oligonucleotides are free of CpG dinucleotides.

Immunostimulatory oligonucleotides that are free of unmethylated CpG dinucleotides are referred to as "non-CpG immunostimulatory oligonucleotides," and they have non-CpG immunostimulatory motifs or may lack any known immunostimulatory motif.

Immunostimulatory oligonucleotides of the invention further can include any combination of methylated and unmethylated CpG and non-CpG immunostimulatory motifs.

### Different Classes of CpG Oligonucleotides:

Different classes of CpG immunostimulatory oligonucleotides have recently been identified. These are referred to as A, B and C class, and are described in greater detail below. Methods of the invention embrace the use of these different classes of CpG immunostimulatory oligonucleotides.

The "A class" CpG immunostimulatory oligonucleotides are characterized functionally by the ability to induce high levels of interferon-alpha and inducing NK cell activation while having minimal effects on B cell activation. Structurally, this class typically has stabilized poly-G sequences at 5' and 3' ends. It also has a palindromic phosphodiester CpG dinucleotide-containing sequence of at least 6 nucleotides, but it does not necessarily contain one of the following hexamer palindromes GACGTC (SEQ ID NO: 3), AGCGCT (SEQ ID NO: 4), or AACGTT (SEQ ID NO: 5) described by Yamamoto and colleagues. Yamamoto S et al. J Immunol 148:4072-6 (1992). A class CpG immunostimulatory oligonucleotides and exemplary sequences of this class have been described in U.S. Non-Provisional Patent Application Serial No. 09/672,126 and published PCT application PCT/US00/26527 (WO 01/22990), both filed on September 27, 2000.

The "B class" CpG immunostimulatory oligonucleotides are characterized functionally by the ability to activate B cells but is relatively weak in inducing IFN-α and NK cell activation. Structurally, this class typically is fully stabilized and includes an unmethylated CpG dinucleotide, optionally within certain preferred base contexts.

In one embodiment, the invention provides a B class CpG oligonucleotide represented by at least the formula:

5' X₁X₂CGX₃X₄ 3'

wherein X_{1,} X₂, X₃, and X₄ are nucleotides. In one embodiment, X₂ is adenine, guanine, or thymine. In another embodiment, X₃ is cytosine, adenine, or thymine.

In another embodiment, the invention provides an isolated B class CpG oligonucleotide represented by at least the formula:

5' N₁X₁X₂CGX₃X₄N₂ 3'

wherein X₁, X₂, X₃, and X₄ are nucleotides and N is any nucleotide and N₁ and N₂ are nucleic acid sequences composed of from about 0-25 N's each. In one embodiment, X₁X₂ is a dinucleotide selected from the group consisting of GpT, GpG, GpA, ApA, ApT, ApG, CpT, CpA, CpG, TpA, TpT and TpG; and X₃X₄ is a dinucleotide selected from the group consisting of TpT, ApT, TpG, ApG, CpG, TpC, ApC, CpC, TpA, ApA and CpA. Preferably X₁X₂ is GpA or GpT and X₃X₄ is TpT. In other embodiments, X₁ or X₂ or both are purines and X₃ or X₄ or both are pyrimidines or X₁X₂ is GpA and X₃ or X₄ or both are pyrimidines. In one preferred embodiment, X₁X₂ is a dinucleotide selected from the group consisting of TpA, ApA, ApC, ApG and GpG. In yet another embodiment, X₃X₄ is a dinucleotide selected from the group consisting of TpT, TpA, TpG, ApA, ApG, GpA and CpA. X₁X₂, in another embodiment, is a dinucleotide selected from the group consisting of TpT, TpG, ApT, GpC, CpC, CpT, TpC, GpT and CpG; X₃ is a nucleotide selected from the group consisting of A and T, and X₄ is a nucleotide, but when X₁X₂ is TpC, GpT or CpG, X₃Xₐ is not TpC, ApT or ApC.

In another preferred embodiment, the CpG oligonucleotide has the sequence 5' TCN₁TX₁X₂CGX₃X₄ 3' (SEQ ID NO: 6). The CpG oligonucleotides of the invention, in some embodiments, include X₁X₂ selected from the group consisting of GpT, GpG, GpA and ApA and X₃X₄ selected from the group consisting of TpT, CpT and TpC.

The B class CpG oligonucleotide sequences of the invention are those broadly described above as well as disclosed in published PCT Patent Applications PCT/US95/01570 and PCT/US97/19791, and in USPs 6,194,388, 6,207,646, 6,214,806, 6,218,371, 6,239,116 and 6,339,068. Exemplary sequences include but are not limited to those disclosed in these latter applications and patents.

The "C class" of CpG immunostimulatory oligonucleotides is characterized functionally by the ability to activate B cells and NK cells and induce IFN-α. Structurally, this class typically includes a B class-type immunostimulatory motif sequence, and a GC-rich palindrome or near-palindrome. Some of these oligonucleotides have both a traditional "stimulatory" CpG sequence and a "GC-rich" or "B-cell neutralizing" motif. These combination motif oligonucleotides have immune stimulating effects that fall somewhere between the effects associated with traditional B class CpG oligonucleotides (i.e., strong induction of B cell activation and dendritic cell (DC) activation), and the effects associated with A class CpG ODN (i.e., strong induction of IFN-α and NK cell activation but relatively poor induction of B cell and DC activation). Krieg AM et al. (1995) Nature 374:546-9; Ballas ZK et al. (1996) J Immuno/ 157:1840-5; Yamamoto S et al. (1992) J Immunol 148:4072-6. Moreover, while preferred B class CpG oligonucleotides often have phosphorothioate backbones and preferred A class CpG oligonucleotides have mixed or chimeric backbones, the C class of combination motif immune stimulatory oligonucleotides may have either stabilized, e.g., phosphorothioate, chimeric, or phosphodiester backbones, and in some preferred embodiments, they have semi-soft backbones. This class has been described in U.S. patent application US 10/224,523 filed on August 19, 2002.

One stimulatory domain or motif of the C class CpG oligonucleotide is defined by the formula: 5' X₁DCGHX₂ 3'. D is a nucleotide other than C. C is cytosine, G is guanine. H is a nucleotide other than G. X₁ and X₂ are any nucleic acid sequence 0 to 10 nucleotides long. X₁ may include a CG, in which case there is preferably a T immediately preceding this CG. In some embodiments, DCG is TCG. X₁ is preferably from 0 to 6 nucleotides in length. In some embodiments, X₂ does not contain any poly G or poly A motifs. In other embodiments, the immunostimulatory oligonucleotide has a poly-T sequence at the 5' end or at the 3' end. As used herein, "poly-A" or "poly-T" shall refer to a stretch of four or more consecutive A's or T's respectively, e.g., 5' AAAA 3' or 5' TTTT 3'. As used herein, "poly-G end" shall refer to a stretch of four or more consecutive G's, e.g., 5' GGGG 3', occurring at the 5' end or the 3' end of a nucleic acid. As used herein, "poly-G oligonucleotide" shall refer to an oligonucleotide having the formula 5' X₁X₂GGGX₃X₄ 3' wherein X₁, X₂, X₃, and X₄ are nucleotides and preferably at least one of X₃ and X₄ is a G. Some preferred designs for the B cell stimulatory domain under this formula comprise TTTTTCG (SEQ ID NO: 7), TCG (SEQ ID NO: 8), TTCG (SEQ ID NO: 9), TTTCG (SEQ ID NO: 10), TTTTCG (SEQ ID NO: 11), TCGT (SEQ ID NO: 12), TTCGT (SEQ ID NO: 13), TTTCGT (SEQ ID NO: 14), TCGTCGT (SEQ ID NO: 15).

The second motif of the C class CpG oligonucleotide is referred to as either P or N and is positioned immediately 5' to X₁ or immediately 3' to X₂.

N is a B cell neutralizing sequence that begins with a CGG trinucleotide and is at least 10 nucleotides long. A B cell neutralizing motif includes at least one CpG sequence in which the CG is preceded by a C or followed by a G (Krieg AM et al. (1998) Proc Natl Acad Sci USA 95:12631-12636) or is a CG containing DNA sequence in which the C of the CG is methylated. Neutralizing motifs or sequences have some degree of immunostimulatory capability when present in an otherwise non-stimulatory motif, but when present in the context of other immunostimulatory motifs serve to reduce the immunostimulatory potential of the other motifs.

P is a GC-rich palindrome containing sequence at least 10 nucleotides long. As used herein, "palindrome" and equivalently "palindromic sequence" shall refer to an inverted repeat, i.e., a sequence such as ABCDEE'D'C'B'A' in which A and A', B and B', etc., are bases capable of forming the usual Watson-Crick base pairs.

As used herein, "GC-rich palindrome" shall refer to a palindrome having a base composition of at least two-thirds G's and C's. In some embodiments the GC-rich domain is preferably 3' to the "B cell stimulatory domain". In the case of a 10-base long GC-rich palindrome, the palindrome thus contains at least 8 G's and C's. In the case of a 12-base long GC-rich palindrome, the palindrome also contains at least 8 G's and C's. In the case of a 14-mer GC-rich palindrome, at least ten bases of the palindrome are G's and C's. In some embodiments the GC-rich palindrome is made up exclusively of G's and C's.

In some embodiments the GC-rich palindrome has a base composition of at least 81 % G's and C's. In the case of such a 10-base long GC-rich palindrome, the palindrome thus is made exclusively of G's and C's. In the case of such a 12-base long GC-rich palindrome, it is preferred that at least ten bases (83 %) of the palindrome are G's and C's. In some preferred embodiments, a 12-base long GC-rich palindrome is made exclusively of G's and C's. In the case of a 14-mer GC-rich palindrome, at least twelve bases (86 %) of the palindrome are G's and C's. In some preferred embodiments, a 14-base long GC-rich palindrome is made exclusively of G's and C's. The C's of a GC-rich palindrome can be unmethylated or they can be methylated.

In general this domain has at least 3 Cs and Gs, more preferably 4 of each, and most preferably 5 or more of each. The number of Cs and Gs in this domain need not be identical. It is preferred that the Cs and Gs are arranged so that they are able to form a self-complementary duplex, or palindrome, such as CCGCGCGG (SEQ ID NO: 16). This may be interrupted by As or Ts, but it is preferred that the self-complementarity is at least partially preserved as for example in the motifs CGACGTTCGTCG (SEQ ID NO: 17) or CGGCGCCGTGCCG (SEQ ID NO: 18). When complementarity is not preserved, it is preferred that the non-complementary base pairs be TG. In a preferred embodiment there are no more than 3 consecutive bases that are not part of the palindrome, preferably no more than 2, and most preferably only 1. In some embodiments, the GC-rich palindrome includes at least one CGG trimer, at least one CCG trimer, or at least one CGCG tetramer. In other embodiments, the GC-rich palindrome is not CCCCCCGGGGGG (SEQ ID NO: 19) or GGGGGGCCCCCC (SEQ ID NO: 20), CCCCCGGGGG (SEQ ID NO: 21) or GGGGGCCCCC (SEQ ID NO: 22).

At least one of the G's of the GC rich region may be substituted with an inosine (I). In some embodiments, P includes more than one I.

In certain embodiments, the immunostimulatory oligonucleotide has one of the following formulas 5' NX₁DCGHX₂ 3', 5' X₁DCGHX₂N 3', 5' PX₁DCGHX₂ 3', 5' X₁DCGHX₂P 3',5' X₁DCGHX₂PX₃ 3', 5' X₁DCGHPX₃ 3', 5' DCGHX₂PX₃ 3', 5' TCGHX₂PX₃ 3', 5' DCGHPX₃ 3' or 5' DCGHP 3'.

The invention provides other immune stimulatory oligonucleotides defined by a formula 5' N₁PyGN₂P 3'. N₁ is any sequence 1 to 6 nucleotides long. Py is a pyrimidine. G is guanine. N₂ is any sequence 0 to 30 nucleotides long. P is a GC-rich palindrome containing a sequence at least 10 nucleotides long.

N₁ and N₂ may contain more than 50% pyrimidines, and more preferably more than 50% T. N₁ may include a CG, in which case there is preferably a T immediately preceding this CG. In some embodiments, N₁PyG is TCG (SEQ ID NO: 8), and most preferably a TCGN₂, where N₂ is not G.

N₁PyCN₂P may include one or more inosine (I) nucleotides. Either the C or the G in N1 may be replaced by inosine, but the CpI is preferred to the IpG. For inosine substitutions such as IpG, the optimal activity may be achieved with the use of a "semi-soft" or chimeric backbone, where the linkage between the IG or the CI is phosphodiester. N₁ may include at least one CI, TCI, IG or TIG motif.

In certain embodiments N₁PyGN₂ is a sequence selected from the group consisting of TTTTTCG (SEQ ID NO: 7), TCG (SEQ ID NO: 8), TTCG (SEQ ID NO: 9), TTTCG (SEQ ID NO: 10), TTTTCG (SEQ ID NO: 11), TCGT (SEQ ID NO: 12), TTCGT (SEQ ID NO: 13), TTTCGT (SEQ ID NO: 14), and TCGTCGT (SEQ ID NO: 15).

Some non-limiting examples of C-Class oligonucleotides include:

T*C_G*C_G*T*C_G*T*T*C_G*G*C*G*C_G*C*G*C*C*G (SEQ ID NO:22)

T*C_G*T*C_G*A*C_G*T*T*C_G*G*C*G*C_G*C*G*C*C*G (SEQ ID NO:23)

T*C_G*G*A*C_G*T*T*C_G*G*C*G*C_G*C*G*C*C*G (SEQ ID NO:24)

T*C_G*G*A*C_G*T*T*C_G*G*C*G*C*G*C*C*G (SEQ ID NO:25)

T*C_G*G*A*C_G*T*T*C_G*G*C*G*C*G*C*C*G (SEQ ID NO:26)

T*C_G*G*A*C_G*T*T*C_G*G*C*G*C*G*C*C*G (SEQ ID NO:27)

T*C_G*G*A*C_G*T*T*C_G*G*C*G*C*G*C*C*G (SEQ ID NO:28)

T*C_G*G*A*C_G*T*T*C_G*G*C*G*C*G*C*C*G (SEQ ID NO:29)

T*C_G*G*A*C_G*T*T*C_G*G*C*G*C*G*C*C*G (SEQ ID NO:30)

wherein *refers to a phosphorothioate bond and- refers to a phosphodiester bond.

### Other Immunostimulatory Oligonucleotides:

Other immunostimulatory oligonucleotides are T-rich and/or possess poly-T motifs. These are described in greater detail in U.S. Patent Application Publication US 2003/0212026 Al. Poly-T motifs generally are characterized by four or more consecutive thymine residues. Still other immunostimulatory oligonucleotides possess poly-G motifs. These are described in greater detail in published PCT application WO 00/14217, published March 16, 2000. Poly-G motifs generally are characterized by four or more consecutive guanine residues.

As noted above, some embodiments of the invention employ non-CpG oligonucleotides. A non-CpG oligonucleotide refers to an oligonucleotide, whether immunostimulatory or not, that lacks an unmethylated CpG motif. Accordingly, T-rich, poly-T, poly-C, methylated CpG and other CpG-like motifs may be present in the genus of non-CpG oligonucleotides.

### TLR Ligands:

Oligonucleotides of the invention may be TLR ligands. As used herein, a TLR ligand is a molecule that binds to a TLR (i.e., a Toll-like receptor). There are a number of TLR identified to date including TLR1, TLR2, TLR3, TLR4, TLR5, TLR6, TLR7, TLR8, TLR9, TLR1O and TLR11. There are similarly a number of TLR ligands identified to date, some of which have been observed to be immunostimulatory (e.g., CpG oligonucleotides). The invention embraces TLR ligands that have been previously identified as being TLR ligands but which have also been observed to be immunologically inert. As used herein, an immunologically inert TLR ligand is one which has been observed to have no or low immunostimulatory potential. The invention also intends to embrace compounds that according to the invention are tested in the presence and absence of an immune stimulating complex (in a vaccine setting) and found to be transformed from an inert compound to an immunostimulatory compound. In some embodiments, the TLR ligands are oligonucleotides that do not possess previously characterized immunostimulatory motifs such as but not limited to unmethylated CpG motifs, methylated CpG motifs, poly T motifs, T-rich motifs, poly-G motifs and the like. Examples of immunostimulatory motifs are described in greater detail in U.S. Patent Application Publication Nos. US 2003/018406 and A1 US 2003/0212026 Al, published September 25, 2003 and November 13, 2003, respectively, the contents of which are incorporated herein by reference in their entirety.

Screening assays for TLR ligands have been described in U.S. Patent Application Publication No. US 2003/0104523, published June 5, 2003, the entire contents of which are incorporated herein in their entirety. The invention embraces the use of compounds that are shown to be TLR ligands (e.g., via radiolabeled ligand-receptor assays) but which when compared to, for example, immunostimulatory oligonucleotides appear to be inert because their relative immunostimulatory potential is negligible or therapeutically non-useful in comparison.

One category of such inert TLR ligands is those which in the absence of an immune stimulating complex have no or low immunostimulatory potential but which when formulated with an immune stimulating complex demonstrate at least a 2-fold, at least a 3-fold, at least a 4-fold, at least a 5-fold, at least a 10-fold, at least a 20-fold, at least a 50-fold, or more increase in immunostimulatory potential, as measured by assays known in the art.

Some inert TLR ligands demonstrate an activity in the absence of an immune stimulating complex that is about that of a true negative control (e.g., saline or a compound that demonstrates no increase in immunostimulatory potential over background levels when in the presence of an immune stimulating complex). They may demonstrate an immunostimulatory potential that is within 5%, within 10%, within 25%, within 50%, or within 75% of a negative control.

In some important embodiments, the TLR ligands are TLR3 ligands, TLR7 ligands, TLR8 ligands and TLR9 ligands.

It is possible that many agents previously screened and characterized as non-TLR ligands are in fact TLR ligands that simply were not immunostimulatory in particular screening assays (e.g., assays that used readouts of TLR signaling rather than TLR binding). The invention embraces various of these previously disregarded compounds, provided that when combined with immune stimulating complexes they readout as immunostimulatory.

### Backbone Modifications, Chimeric etc.:

Oligonucleotides of the invention preferably are partially resistant to degradation (e.g., are stabilized). A "stabilized oligonucleotide molecule" refers to an oligonucleotide that is relatively resistant to *in vivo* degradation (e.g. via an exo- or endo-nuclease). Nucleic acid stabilization can be accomplished via backbone modifications. Oligonucleotides having phosphorothioate linkages provide maximal activity and protect the oligonucleotide from degradation by intracellular exo- and endo-nucleases. Other modified oligonucleotides include phosphodiester modified oligonucleotides, combinations of phosphodiester and phosphorothioate oligonucleotide, methylphosphonate, methylphosphorothioate, phosphorodithioate, p-ethoxy, and combinations thereof.

The immunostimulatory oligonucleotides may have a chimeric backbone. For purposes of the instant invention, a chimeric backbone refers to a partially stabilized backbone, wherein at least one internucleotide linkage is phosphodiester or phosphodiester-like, and wherein at least one other internucleotide linkage is a stabilized internucleotide linkage, wherein the at least one phosphodiester or phosphodiester-like linkage and the at least one stabilized linkage are different. Since boranophosphonate linkages have been reported to be stabilized relative to phosphodiester linkages, for purposes of the chimeric nature of the backbone, boranophosphonate linkages can be classified either as phosphodiester-like or as stabilized, depending on the context. For example, a chimeric backbone according to the instant invention could, in some embodiments, includes at least one phosphodiester (phosphodiester or phosphodiester-like) linkage and at least one boranophosphonate (stabilized) linkage. In other embodiments, a chimeric backbone according to the instant invention could include boranophosphonate (phosphodiester or phosphodiester-like) and phosphorothioate (stabilized) linkages. A "stabilized internucleotide linkage" shall mean an internucleotide linkage that is relatively resistant to *in vivo* degradation (e.g., via an exo- or endo-nuclease), compared to a phosphodiester internucleotide linkage. Preferred stabilized internucleotide linkages include, without limitation, phosphorothioate, phosphorodithioate, methylphosphonate, and methylphosphorothioate. Other stabilized internucleotide linkages include, without limitation, peptide, alkyl, dephospho, and others as described above.

Modified backbones such as phosphorothioates may be synthesized using automated techniques employing either phosphoramidate or H-phosphonate chemistries. Aryl- and alkyl-phosphonates can be made, e.g., as described in U.S. Patent No. 4,469,863; and alkylphosphotriesters (in which the charged oxygen moiety is alkylated as described in U.S. Patent No. 5,023,243 and European Patent No. 092,574) can be prepared by automated solid phase synthesis using commercially available reagents. Methods for making other DNA backbone modifications and substitutions have been described. Uhlmann E et al. (1990) Chem Rev 90:544; Goodchild J (1990) Bioconjugate Chem 1:165. Methods for preparing chimeric oligonucleotides are also known. For instance patents issued to Uhlmann et al have described such techniques.

Mixed backbone modified ODN may be synthesized using a commercially available DNA synthesizer and standard phosphoramidite chemistry. (F. E. Eckstein, "Oligonucleotides and Analogues - A Practical Approach" IRL Press. Oxford. UK, 1991, and M. D. Matteucci and M. H. Caruthers, Tetrahedron Lett. 21, 719 (1980)) After coupling, PS linkages are introduced by sulfurization using the Beaucage reagent (R. P. Iyer, W. Egan, J. B. Regan and S. L. Beaucage, J. Am. Chem. Soc. 112, 1253 (1990)) (0.075 M in acetonitrile) or phenyl acetyl disulfide (PADS) followed by capping with acetic anhydride, 2,6-lutidine in tetrahydrofurane (1:1:8; v:v:v) and N-methylimidazole (16 % in tetrahydrofurane). This capping step is performed after the sulfurization reaction to minimize formation of undesired phosphodiester (PO) linkages at positions where a phosphorothioate linkage should be located. In the case of the introduction of a phosphodiester linkage, e.g. at a CpG dinucleotide, the intermediate phosphorous-III is oxidized by treatment with a solution of iodine in water/pyridine. After cleavage from the solid support and final deprotection by treatment with concentrated ammonia (15 hrs at 50°C), the ODN are analyzed by HPLC on a Gen-Pak Fax column (Millipore-Waters) using a NaCl-gradient (e.g. buffer A: 10 mM NaH₂PO₄ in acetonitrile/water =1:4/v:v pH 6.8; buffer B: 10 mM NaH₂PO₄, 1.5 M NaCl in acetonitrile/water = 1:4/v:v; 5 to 60 % B in 30 minutes at 1 ml/min) or by capillary gel electrophoresis. The ODN can be purified by HPLC or by FPLC on a Source High Performance column (Amersham Pharmacia). HPLC-homogeneous fractions are combined and desalted *via* a C18 column or by ultrafiltration. The ODN was analyzed by MALDI-TOF mass spectrometry to confirm the calculated mass.

The oligonucleotides of the invention can also include other modifications. These include nonionic DNA analogs, such as alkyl- and aryl-phosphates (in which the charged phosphonate oxygen is replaced by an alkyl or aryl group), phosphodiester and alkylphosphotriesters, in which the charged oxygen moiety is alkylated. Nucleic acids which contain diol, such as tetraethyleneglycol or hexaethyleneglycol, at either or both termini have also been shown to be substantially resistant to nuclease degradation.

### Soft and Semi-Soft ODN:

In some embodiments of the invention, the oligonucleotides may be soft or semi-soft oligonucleotides. A soft oligonucleotide is an immunostimulatory oligonucleotide having a partially stabilized backbone, in which phosphodiester or phosphodiester-like internucleotide linkages occur only within and immediately adjacent to at least one internal pyrimidine -purine dinucleotide (YZ). Preferably YZ is YG, a pyrimidine-guanosine (YG) dinucleotide. The at least one internal YZ dinucleotide itself has a phosphodiester or phosphodiester-like internucleotide linkage. A phosphodiester or phosphodiester-like internucleotide linkage occurring immediately adjacent to the at least one internal YZ dinucleotide can be 5', 3', or both 5' and 3' to the at least one internal YZ dinucleotide.

In particular, phosphodiester or phosphodiester-like internucleotide linkages involve "internal dinucleotides." An internal dinucleotide in general shall mean any pair of adjacent nucleotides connected by an internucleotide linkage, in which neither nucleotide in the pair of nucleotides is a terminal nucleotide, i.e., neither nucleotide in the pair of nucleotides is a nucleotide defining the 5' or 3' end of the oligonucleotide. Thus a linear oligonucleotide that is n nucleotides long has a total of n-1 dinucleotides and only n-3 internal dinucleotides. Each internucleotide linkage in an internal dinucleotide is an internal internucleotide linkage. Thus a linear oligonucleotide that is n nucleotides long has a total of n-1 internucleotide linkages and only n-3 internal internucleotide linkages. The strategically placed phosphodiester or phosphodiester-like internucleotide linkages, therefore, refer to phosphodiester or phosphodiester-like internucleotide linkages positioned between any pair of nucleotides in the nucleic acid sequence. In some embodiments the phosphodiester or phosphodiester-like internucleotide linkages are not positioned between either pair of nucleotides closest to the 5' or 3' end.

Preferably a phosphodiester or phosphodiester-like internucleotide linkage occurring immediately adjacent to the at least one internal YZ dinucleotide is itself an internal internucleotide linkage. Thus for a sequence N₁ YZ N₂, wherein N₁ and N₂ are each, independent of the other, any single nucleotide, the YZ dinucleotide has a phosphodiester or phosphodiester-like internucleotide linkage, and in addition (a) N₁ and Y are linked by a phosphodiester or phosphodiester-like internucleotide linkage when N₁ is an internal nucleotide, (b) Z and N₂ are linked by a phosphodiester or phosphodiester-like internucleotide linkage when N₂ is an internal nucleotide, or (c) N₁ and Y are linked by a phosphodiester or phosphodiester-like internucleotide linkage when N₁ is an internal nucleotide and Z and N₂ are linked by a phosphodiester or phosphodiester-like interucleotide linkage when N₂ is an internal nucleotide.

Soft oligonucleotides are believed to be relatively susceptible to nuclease cleavage compared to completely stabilized oligonucleotides. Without meaning to be bound to a particular theory or mechanism, it is believed that soft oligonucleotides of the invention are cleavable to fragments with reduced or no immunostimulatory activity relative to full-length soft oligonucleotides. Incorporation of at least one nuclease-sensitive internucleotide linkage, particularly near the middle of the oligonucleotide, is believed to provide an "off switch" which alters the pharmacokinetics of the oligonucleotide so as to reduce the duration of maximal immunostimulatory activity of the oligonucleotide. This can be of particular value in tissues and in clinical applications in which it is desirable to avoid injury related to chronic local inflammation or immunostimulation, e.g., the kidney.

Semi-soft oligonucleotides are immunostimulatory oligonucleotides having a partially stabilized backbone, in which phosphodiester or phosphodiester-like internucleotide linkages occur only within at least one internal pyrimidine-purine (YZ) dinucleotide. Semi-soft oligonucleotides generally possess increased immunostimulatory potency relative to corresponding fully stabilized immunostimulatory oligonucleotides. Due to the greater potency of semi-soft oligonucleotides, semi-soft oligonucleotides may be used, in some instances, at lower effective concentations and have lower effective doses than conventional fully stabilized immunostimulatory oligonucleotides in order to achieve a desired biological effect.

It is believed that the foregoing properties of semi-soft oligonucleotides generally increase with increasing "dose" of phosphodiester or phosphodiester-like internucleotide linkages involving internal YZ dinucleotides. Thus it is believed, for example, that generally for a given oligonucleotide sequence with five internal YZ dinucleotides, an oligonucleotide with five internal phosphodiester or phosphodiester-like YZ internucleotide linkages is more immunostimulatory than an oligonucleotide with four internal phosphodiester or phosphodiester-like YG internucleotide linkages, which in turn is more immunostimulatory than an oligonucleotide with three internal phosphodiester or phosphodiester-like YZ internucleotide linkages, which in turn is more immunostimulatory than an oligonucleotide with two internal phosphodiester or phosphodiester-like YZ internucleotide linkages, which in turn is more immunostimulatory than an oligonucleotide with one internal phosphodiester or phosphodiester-like YZ internucleotide linkage. Importantly, inclusion of even one internal phosphodiester or phosphodiester-like YZ internucleotide linkage is believed to be advantageous over no internal phosphodiester or phosphodiester-like YZ internucleotide linkage. In addition to the number of phosphodiester or phosphodiester-like internucleotide linkages, the position along the length of the nucleic acid can also affect potency.

The soft and semi-soft oligonucleotides will generally include, in addition to the phosphodiester or phosphodiester-like internucleotide linkages at preferred internal positions, 5' and 3' ends that are resistant to degradation. Such degradation-resistant ends can involve any suitable modification that results in an increased resistance against exonuclease digestion over corresponding unmodified ends. For instance, the 5' and 3' ends can be stabilized by the inclusion there of at least one phosphate modification of the backbone. In a preferred embodiment, the at least one phosphate modification of the backbone at each end is independently a phosphorothioate, phosphorodithioate, methylphosphonate or methylphosphorothioate internucleotide linkage. In another embodiment, the degradation-resistant end includes one or more nucleotide units connected by peptide or amide linkages at the 3' end.

A phosphodiester internucleotide linkage is the type of linkage characteristic of nucleic acids found in nature. The phosphodiester internucleotide linkage includes a phosphorus atom flanked by two bridging oxygen atoms and bound also by two additional oxygen atoms, one charged and the other uncharged. Phosphodiester internucleotide linkage is particularly preferred when it is important to reduce the ' tissue half-life of the oligonucleotide.

A phosphodiester-like internucleotide linkage is a phosphorus-containing bridging group that is chemically and/or diastereomerically similar to phosphodiester. Measures of similarity to phosphodiester include susceptibility to nuclease digestion and ability to activate RNAse H. Thus for example phosphodiester, but not phosphorothioate, oligonucleotides are susceptible to nuclease digestion, while both phosphodiester and phosphorothioate oligonucleotides activate RNAse H. In a preferred embodiment the phosphodiester-like internucleotide linkage is boranophosphate (or equivalently, boranophosphonate) linkage. U.S. Patent No. 5,177,198; U.S. Patent No. 5,859,231; U.S. Patent No. 6,160,109; U.S. Patent No. 6,207,819; Sergueev et al., (1998) J Am Chem Soc 120:9417-27. In another preferred embodiment the phosphodiester-like internucleotide linkage is diasteromerically pure Rp phosphorothioate. It is believed that diasteromerically pure Rp phosphorothioate is more susceptible to nuclease digestion and is better at activating RNAse H than mixed or diastereomerically pure Sp phosphorothioate. Stereoisomers of CpG oligonucleotides are the subject of co-pending U.S. patent application 09/361,575 filed July 27, 1999, and published PCT application PCT/US99/17100 (WO 00/06588). It is to be noted that for purposes of the instant invention, the term "phosphodiester-like internucleotide linkage" specifically excludes phosphorodithioate and methylphosphonate internucleotide linkages.

As described above the soft and semi-soft oligonucleotides may have phosphodiester like linkages between C and G. One example of a phosphodiester-like linkage is a phosphorothioate linkage in an Rp conformation. Oligonucleotide p-chirality can have apparently opposite effects on the immune activity of a CpG oligonucleotide, depending upon the time point at which activity is measured. At an early time point of 40 minutes, the Rₚ but not the Sₚ stereoisomer of phosphorothioate CpG oligonucleotide induces JNK phosphorylation in mouse spleen cells. In contrast, when assayed at a late time point of 44 hr, the Sₚ but not the Rₚ stereoisomer is active in stimulating spleen cell proliferation. This difference in the kinetics and bioactivity of the Rₚ and Sₚ stereoisomers does not result from any difference in cell uptake, but rather most likely is due to two opposing biologic roles of the p-chirality. First, the enhanced activity of the Rp stereoisomer compared to the Sp for stimulating immune cells at early time points indicates that the Rp may be more effective at interacting with the CpG receptor, TLR9, or inducing the downstream signaling pathways. On the other hand, the faster degradation of the Rp PS-oligonucleotides compared to the Sp results in a much shorter duration of signaling, so that the Sp PS-oligonucleotides appear to be more biologically active when tested at later time points.

A surprisingly strong effect is achieved by the p-chirality at the CpG dinucleotide itself. In comparison to a stereo-random CpG oligonucleotide, the congener in which the single CpG dinucleotide was linked in Rp was slightly more active, while the congener containing an Sp linkage was nearly inactive for inducing spleen cell proliferation.

### Size, Synthesis, Modified Bases and Other Oligonucleotide Properties

The size of the immunostimulatory oligonucleotide (i.e., the number of nucleotide residues along the length of the oligonucleotide) also may contribute to the stimulatory activity of the oligonucleotide.. For facilitating uptake into cells, immunostimulatory oligonucleotides preferably have a minimum length of 6 nucleotide residues. Oligonucleotides of any size greater than 6 nucleotides (even many kb long) are capable of inducing an immune response if sufficient immunostimulatory motifs are present, because larger oligonucleotides are degraded inside cells. It is believed that semi-soft oligonucleotides as short as 4 nucleotides can also be immunostimulatory if they can be delivered to the interior of a cell. In certain embodiments, the immunostimulatory oligonucleotides are 4 to 100 nucleotides long, 6 to 100 nucleotides long, or 8 to 100 nucleotides long. In typical embodiments the immunostimulatory oligonucleotides are 4 to 40 nucleotides long, 6 to 40 nucleotides long, 8 to 40 nucleotides long, 4 to 20 nucleotides long, 6 to 20 nucleotides long, 8 to 20 nucleotides long, 4 to 10 nucleotides, long, 6 to 10 nucleotides long or 8 to 10 nucleotides long. In important embodiments, nucleic acids and oligonucleotides of the invention are not plasmids or expression vectors.

The term oligonucleotide also encompasses oligonucleotides with substitutions or modifications, such as in the bases and/or sugars. For example, they include oligonucleotides having backbone sugars that are covalently attached to low molecular weight organic groups other than a hydroxyl group at the 2' position and other than a phosphate group or hydroxy group at the 5' position. Thus modified oligonucleotides may include a 2'-O-alkylated ribose group. In addition, modified oligonucleotides may include sugars such as arabinose or 2'-fluoroarabinose instead of ribose. Thus the oligonucleotides may be heterogeneous in backbone composition thereby containing any possible combination of polymer units linked together such as peptide-nucleic acids (which have an amino acid backbone with nucleic acid bases). The foregoing applies equally to nucleic acids disclosed herein.

The immunostimulatory oligonucleotides can encompass various chemical modifications and substitutions, in comparison to natural RNA and DNA, involving a phosphodiester internucleotide bridge, a β-D-ribose unit and/or a natural nucleotide base (adenine, guanine, cytosine, thymine, uracil). Examples of chemical modifications are known to the skilled person and are described, for example, in Uhlmann E et al. (1990) Chem Rev 90:543; "Protocols for Oligonucleotides and Analogs" Synthesis and Properties & Synthesis and Analytical Techniques, S. Agrawal, Ed, Humana Press, Totowa, USA 1993; Crooke ST et al. (1996) Annu Rev Pharmacol Toxicol 36:107-129; and Hunziker J et al. (1995) Mod Synth Methods 7:331-417. An oligonucleotide may have one or more modifications, wherein each modification is located at a particular phosphodiester internucleotide bridge and/or at a particular β-D-ribose unit and/or at a particular natural nucleotide base position in comparison to an oligonucleotide of the same sequence which is composed of natural DNA or RNA.

For example, the invention relates to an oligonucleotide which may comprise one or more modifications and wherein each modification is independently selected from
a) the replacement of a phosphodiester internucleotide bridge located at the 3' and/or the 5' end of a nucleotide by a modified internucleotide bridge,
b) the replacement of phosphodiester bridge located at the 3' and/or the 5' end of a nucleotide by a dephospho bridge,
c) the replacement of a sugar phosphate unit from the sugar phosphate backbone by another unit,
d) the replacement of a beta-D-ribose unit by a modified sugar unit, and
e) the replacement of a natural nucleotide base by a modified nucleotide base.

More detailed examples for the chemical modification of an oligonucleotide are as follows.

A phosphodiester internucleotide bridge located at the 3' and/or the 5' end of a nucleotide can be replaced by a modified internucleotide bridge, wherein the modified internucleotide bridge is for example selected from phosphorothioate, phosphorodithioate, NR¹R²-phosphoramidate, boranophosphate, α-hydroxybenzyl phosphonate, phosphate-(C₁-C₂₁)-O-alkyl ester, phosphate-[(C₆-C₁₂)aryl-(C₁-C₂₁)-O-alkyl]ester, (C₁-C₈)alkylphosphonate and/or (C₆-C₁₂)arylphospbonate bridges, (C₇-C₁₂)-α-hydroxymethyl-aryl (e.g., disclosed in WO 95/01363), wherein (C₆-C₁₂)aryl, (C₆-C₂₀)aryl and (C₆-C₁₄)aryl are optionally substituted by halogen, alkyl, alkoxy, nitro, cyano, and where R¹ and R² are, independently of each other, hydrogen, (C₁-C₁₈)-alkyl, (C₆-C₂₀)-aryl, (C₆-C₁₄)-aryl-(C₁-C₈)-alkyl, preferably hydrogen, (C₁-C₈)-alkyl, preferably (C₁-C₄)-alkyl and/or methoxyethyl, or R¹ and R² form, together with the nitrogen atom carrying them, a 5-6-membered heterocyclic ring which can additionally contain a further heteroatom from the group O, S and N.

The replacement of a phosphodiester bridge located at the 3' and/or the 5' end of a nucleotide by a dephospho bridge (dephospho bridges are described, for example, in Uhlmann E and Peyman A in "Methods in Molecular Biology", Vol. 20, "Protocols for Oligonucleotides and Analogs", S. Agrawal, Ed., Humana Press, Totowa 1993, Chapter 16, pp. 355 ff), wherein a dephospho bridge is for example selected from the dephospho bridges formacetal, 3'-thioformacetal, methylhydroxylamine, oxime, methylenedimethyl-hydrazo, dimethylenesulfone and/or silyl groups.

A sugar phosphate unit (i.e., a (3-D-ribose and phosphodiester internucleotide bridge together forming a sugar phosphate unit) from the sugar phosphate backbone (i.e., a sugar phosphate backbone is composed of sugar phosphate units) can be replaced by another unit, wherein the other unit is for example suitable to build up a "morpholino-derivative" oligomer (as described, for example, in Stirchak EP et al. (1989) Nucleic Acids Res 17:6129-41), that is, e.g., the replacement by a morpholino-derivative unit; or to build up a polyamide nucleic acid ("PNA"; as described for example, in Nielsen PE et al. (1994) Bioconjug Chem 5:3-7), that is, e.g., the replacement by a PNA backbone unit, e.g., by 2-aminoethylglycine.

A β-ribose unit or a β-D-2'-deoxyribose unit can be replaced by a modified sugar unit, wherein the modified sugar unit is for example selected from β-D-ribose, α-D-2'-deoxyribose, L-2'-deoxyribose, 2'-F-2'-deoxyribose, 2'-F-arabinose, 2'-O-(C₁-C₆)alkyl-ribose, preferably 2'-O-(C)-C₆)alkyl-ribose is 2'-O-methylribose, 2'-O-(C₂-C₆)alkenyl-ribose, 2'-[O-(C₁-C₆)alkyl-O-(C₁-C₆)alky]-ribose, 2'-NH₂-2'-deoxyribose, β-D-xylo-furanose, α-arabinofuranose, 2,4-dideoxy-β-D-erythro-hexopyranose, and carbocyclic (described, for example, in Froehler J (1992) Am Chem Soc 114:8320) and/or open-chain sugar analogs (described, for example, in Vandendriessche et al. (1993) Tetrahedron 49:7223) and/or bicyclosugar analogs (described, for example, in Tarkov M et al. (1993) Helv Chim Acta 76:481).

In some preferred embodiments, the sugar is 2'-O-methylribose, particularly for one or both nucleotides linked by a phosphodiester or phosphodiester-like internucleotide linkage.

Oligonucleotides also include substituted purines and pyrimidines such as C-5 propyne pyrimidine and 7-deaza-7-substituted purine modified bases. Wagner RW et al. (1996) Nat Biotechnol 14:840-4. Besides the more common naturally occurring bases of adenine, cytosine, guanine, thymine, and uracil, the oligonucleotides may also comprise other naturally and non-naturally occurring bases, substituted and unsubstituted aromatic moieties. A modified base is any base which is chemically ' distinct from the naturally occurring bases typically found in DNA and RNA such as thymine, adenine, cytosine, guanine and uracil, but which share basic chemical structures with these naturally occurring bases. Modified nucleotide bases include, for example, 5-(C₂-C₆)-alkenylcytosine, 5-(C₂-C₆)-alkenyluracil, N4-alkylcytosine, e.g., N4-ethylcytosine, N4-alkyldeoxycytidine, e.g., N4-ethyldeoxycytidine, 5-(C₁-C₆)-alkylcytosine, 5-(C₁-C₆)-alkyluracil, 5-(C₂-C₆)-alkynylcytosine, 5-(C₂-C₆)-alkynyluracil, 2-amino-6-chloropurine, 2-aminopurine, 5-aminouracil, 8-azapurine, 5-bromocytosine, 5-bromouracil, 5-chlorocytosine, 5-chlorouracil, deoxyribonucleotides of nitropyrrole, diaminopurine e.g., 2,4-diaminopurine and 2,6-diaminopurine, dihydrouracil, N²-dimethylguanine, 5-fluorocytosine, 5-fluorouracil, 5-hydroxycytosine, 5-hydroxydeoxycytidine, 5-hydroxymethylcytosine, 5-hydroxymethyldeoxycytidine, 5-hydroxymethyluracil, hypoxanthine, inosine, 5-mothylcytosine, C5-propynylpyrimidine, pseudouracil, a substituted 7-deazapurine, preferably 7-deaza-7-substituted and/or 7-deaza-8-substituted purine, 6-thiodeoxyguanosine, 2-thiouracil, 4-thiouracil, uracil, etc. This list is exemplary and is not limiting. Other such modifications are known to those of skill in the art.

In particular formulas described herein a set of modified bases is defined. For instance, the letter Y is used to refer to a nucleotide containing a cytosine or a modified cytosine. A modified cytosine as used herein is a naturally occurring or non-naturally occurring pyrimidine base analog of cytosine which can replace this base without impairing the immunostimulatory activity of the oligonucleotide. Modified cytosines include but are not limited to 5-substituted cytosines (e.g. 5-methyl-cytosine, 5-fluoro-cytosine, 5-chloro-cytosine, 5-bromo-cytosine, 5-iodo-cytosine, 5-hydroxy-cytosine, 5-hydroxymethyl-cytosine, 5-difluoromethyl-cytosine, and unsubstituted or substituted 5-alkynyl-cytosine), 6-substituted cytosines, N4-substituted cytosine (e.g. N4-ethyl-cytosine), 5-aza-cytosine, 2-mercapto-cytosine, isocytosine, pseudo-isocytosine, cytosine analogs with condensed ring systems (e.g. N,N'-propylene cytosine or phenoxazine), and uracil and its derivatives.(e.g. 5-fluorouracil, 5-bromo-uracil, 5-bromovinyl-uracil, 4-thio-uracil, 5-hydroxy-uracil, 5-propynyl-uracil). Some of the preferred cytosines include 5-methylcytosine, 5-fluorocytosine, 5-hydroxycytosine, 5-hydroxymethyl-cytosine, and N4-ethylcytosine. In another embodiment, the cytosine base is substituted by a universal base (e.g. 3-nitropyrrole, P-base), an aromatic ring system (e.g. fluorobenzene or difluorobenzene) or a hydrogen atom (dSpacer).

The letter Z is used to refer to guanine or a modified guanine base. A modified guanine as used herein is a naturally occurring or non-naturally occurring purine base analog of guanine which can replace this base without impairing the immunostimulatory activity of the oligonucleotide. Modified guanines include but are not limited to 7-deazaguanine, 7-deaza-7-substituted guanine (such as 7-deaza-7-(C2-C6)alkynylguanine), 7-deaza-8-substituted guanine, hypoxanthine, N2-substituted guanines (e.g. N2-methyl-guanine), 5-amino-3-methyl-3H,6H-thiazolo[4,5-d]pyrimidine-2,7-dione, 2,6-diaminopurine, 2-aminopurine, purine, indole, adenine, substituted adenines (e.g. N6-methyl-adenine, 8-oxo-adenine) 8-substituted guanine (e.g. 8-hydroxyguanine and 8-bromoguanine), and 6-thioguanine. In another embodiment of the invention, the guanine base is substituted by a universal base (e.g. 4-methyl-indole, 5-nitro-indole, and K-base), an aromatic ring system (e.g. benzimidazole or dichloro- benzimidazole, 1-methyl-1H-[1,2,4]triazole-3-carboxylic acid amide) or a hydrogen atom (dSpacer).

The oligonucleotides may have one or more accessible 5' ends. It is possible to create modified oligonucleotides having two such 5' ends. This may be achieved, for instance by attaching two oligonucleotides through a 3'-3' linkage to generate an oligonucleotide having one or two accessible 5' ends. The 3'3'-linkage may be a phosphodiester, phosphorothioate or any other modified internucleotide bridge. Methods for accomplishing such linkages are known in the art. For instance, such linkages have been described in Seliger, H.; et al., Oligonucleotide analogs with terminal 3'-3'- and 5'-5'-internucleotidic linkages as antisense inhibitors of viral gene expression, Nucleotides & Nucleotides (1991), 10(1-3), 469-77 and Jiang, et al., Pseudo-cyclic oligonucleotides: in vitro and in vivo properties, Bioorganic & Medicinal Chemistry (1999), 7(12), 2727-2735.

Additionally, 3'3'-linked oligonucleotides where the linkage between the 3'-terminal nucleotides is not a phosphodiester, phosphorothioate or other modified bridge, can be prepared using an additional spacer, such as tri- or tetra-ethylenglycol phosphate moiety (Durand, M. et al, Triple-helix formation by an oligonucleotide containing one (dA)12 and two (dT)12 sequences bridged by two hexaethylene glycol chains, Biochemistry (1992), 31(38), 9197-204, US Patent No. 5658738, and US Patent No. 5668265). Alternatively, the non-nucleotidic linker may be derived from ethanediol, propanediol, or from an abasic deoxyribose (dSpacer) unit (Fontanel, Marie Laurence et al., Sterical recognition by T4 polynucleotide kinase of non-nucleosidic moieties 5'-attached to oligonucleotides; Nucleic Acids Research (1994), 22(11), 2022-7) using standard phosphoramidite chemistry. The non-nucleotidic linkers can be incorporated once or multiple times, or combined with each other allowing for any desirable distance between the 3'-ends of the two ODNs to be linked.

### Branched ODN and Dendrimers

The immunostimulatory oligonucleotides may also contain one or more unusual linkages between the nucleotide or nucleotide-analogous moieties. The usual internucleoside linkage is a 3'5'-linkage. All other linkages are considered to be unusual internucleoside linkages, such as 2'5'-, 5'5'-, 3'3'-, 2'2'-, 2'3'-linkages. The nomenclature 2' to 5' is chosen according to the carbon atom of ribose. However, if unnatural sugar moieties arc employed, such as ring-expanded sugar analogs (e.g. hexanose, cylohexene or pyranose) or bi- or tricyclic sugar analogs, then this nomenclature changes according to the nomenclature of the monomer. In 3'-deoxy-β-D-ribopyranose analogs (also called *p*-DNA), the mononucleotides are e.g. connected via a 4'2' -linkage.

If the oligonucleotides contains one 3'3'-linkage, then this oligonucleotide may have two unlinked 5'-ends. Similarly, if the oligonucleotide contains one 5'5'-linkage, then this oligonucleotide may have two unlinked 3'-ends. The accessibility of unlinked ends of nucleotides may be better accessible by their receptors. Both types of unusual linkages (3'3'- and 5'5') were described by Ramalho Ortigao et al. (Antisense Research and Development (1992) 2, 129-46), whereby oligonucleotides having a 3'3'-linkage were reported to show enhanced stability towards cleavage by nucleases.

Different types of linkages can also be combined in one molecule which may lead to branching of the oligomer. If one part of the oligonucleotide is connected at the 3'-end via a 3'3'-linkage to a second oligonucleotide part and at the 2'-end via a 2'3'-linkage to a third part of the molecule, this results e.g. in a branched oligonucleotide with three 5'-ends (3'3'-, 2'3'-branched).

In principle, linkages between different parts of an oligonucleotide or between different oligonucleotides, respectively, can occur via all parts of the molecule, as long as this does not negatively interfere with the recognition by its receptor. According to the nature of the oligonucleotide, the linkage can involve the sugar moiety (Su), the heterocyclic nucleobase (Ba) or the phosphate backbone (Ph). Thus, linkages of the type Su-Su, Su-Ph, Su-Ba, Ba-Ba, Ba-Su; Ba-Ph, Ph-Ph, Ph-Su, and Ph-Ba are possible. If the oligonucleotides are further modified by certain non-nucleotidic substituents, the linkage can also occur via the modified parts of the oligonucleotides. These modifications also include modified oligonucleotides, e.g. PNA, LNA, or Morpholino Oligonucleotide analogs.

The linkages are preferably composed of C, H, N, O, S, B, P, and Halogen, containing 3 to 300 atoms. An example with 3 atoms is an acetal linkage (ODN1-3'-O-CH₂-O-3'-ODN2) connecting e.g. the 3'-hydroxy group of one nucleotide to the 3'-hydroxy group of a second oligonucleotide. An example with about 300 atoms is PEG-40 (tetraconta polyethyleneglycol). Preferred linkages are phosphodiester, phosphorothioate, methylphosphonate, phosphoramidate, boranophosphonate, amide, ether, thioether, acetal , thioacetal, urea, thiourea, sulfonamide, Schiff Base and disulfide linkages. It is also possible to use the Solulink BioConjugation System available at the "trilinkbiotech" website.

If the oligonucleotide is composed of two or more sequence parts, these parts can be identical or different. Thus, in an oligonucleotide with a 3'3'-linkage, the sequences can be identical 5'-ODN1-3'3'-ODN1-5' or different 5'-ODN1-3'3'-ODN2-5'. Furthermore, the chemical modification of the various oligonucleotide parts as well as the linker connecting them may be different. Since the uptake of short oligonucleotides appears to be less efficient than that of long oligonucleotides, linking of two or more short sequences results in improved immune stimulation. The length of the short oligonucleotides is preferably 2-20 nucleotides, more preferably 3-16 nucleotides, but most preferably 5-10 nucleotides. Preferred are linked oligonucleotides which have two or more unlinked 5'-ends.

The oligonucleotide partial sequences may also be linked by non-nucleotidic linkers, in particular abasic linkers (dSpacers), trietyhlene glycol units or hexaethylene glycol units. Further preferred linkers are alkylamino linkers, such as C3, C6, C12 aminolinkers, and also alkylthiol linkers, such as C3 or C6 thiol linkers. The oligonucleotides can also be linked by aromatic residues which may be further substituted by alkyl or substituted alkyl groups. The oligonucleotides may also contain a Doubler or Trebler unit as described at the "glenres" website, in particular those oligonucleotides with a 3'3'-linkage. Branching of the oligonucleotides by multiple doubler, trebler, or other multiplier units leads to dendrimers which are a further embodiment of this invention. The oligonucleotides may also contain linker units resulting from peptide modifying reagents or oligonucleotide modifying reagents as described at the "glenres" website. Furthermore, it may contain one or more natural or unnatural amino acid residues which are connected by peptide (amide) linkages.

Another possibility for linking oligonucleotides is via crosslinking of the heterocyclic bases (Verma and Eckstein; Annu. Rev. Biochem. (1998) 67: 99-134; page 124). A linkage between the sugar moiety of one sequence part with the heterocyclic base of another sequence part (Iyer et al. Curr. Opin. Mol. Therapeutics (1999) 1: 344-358; page 352) may also be used.

The different oligonucleotides are synthesized by established methods and can be linked together on-line during solid-phase synthesis. Alternatively, they may be linked together post-synthesis of the individual partial sequences.

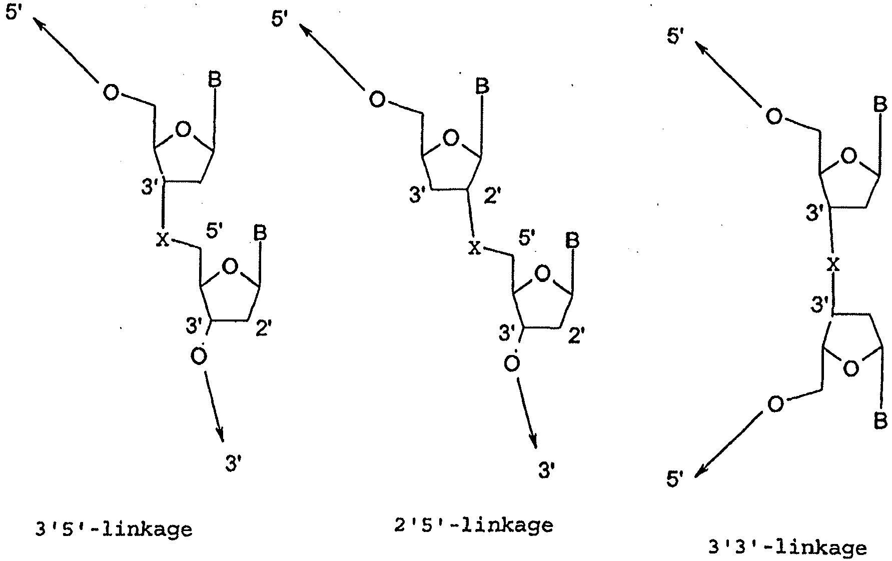

X is e.g.: 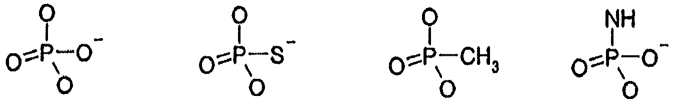 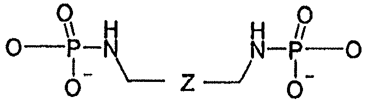 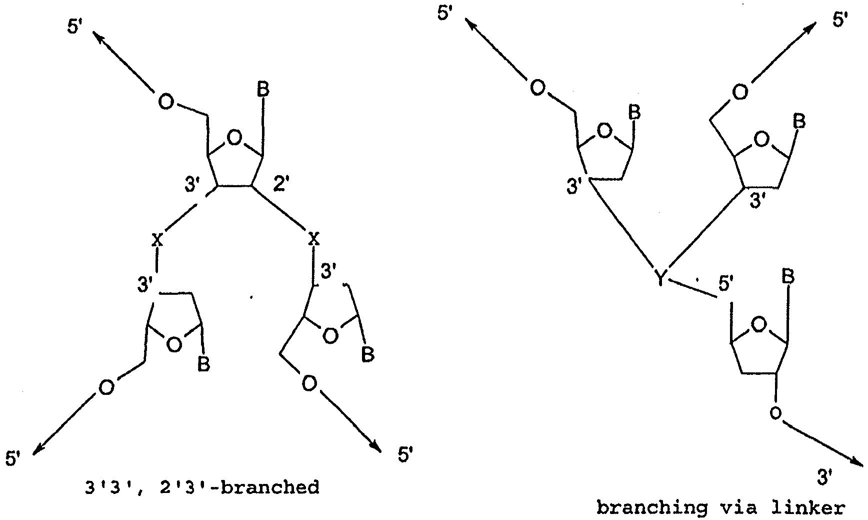 x is e.g.: 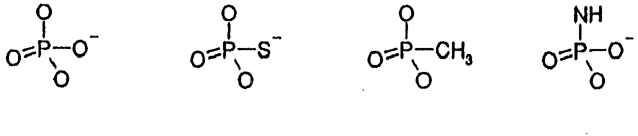 Y is e.g.:

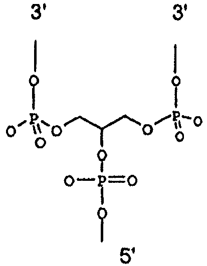

### Antigens:

Antigens of the invention may be formulated with an immune stimulating complex, as with oligonucleotides. The antigen and oligonucleotide need not be formulated together. The antigen may in some embodiments be conjugated to the oligonucleotide (e.g., by covalent means).

Antigen administration to a subject is known in the art and is generally referred to as active vaccination. The antigen and/or oligonucleotide may be administered locally or systemically. According to the invention, the antigen formulation may be administered to the same site as the oligonucleotide formulation. In some preferred embodiments, the antigen and oligonucleotide at least drain to the same lymph node, even if they are administered to different sites of the body. Some preferred routes of administration include intramuscular (IM) and subcutaneous (SC) administration. The antigen and/or oligonucleotide may also be administered by mucosal routes such as oral, sublingual, intranasal, intratracheal, intrapulmonary, intrarectally and intravaginally. In still other embodiments, the antigen and/or oligonucleotide may be administered topically (e.g., to the skin or to an exposed mucosal surface).

The antigen formulation is administered to the subject substantially simultaneously with the oligonucleotide formulation. Substantially simultaneously means that the antigen is administered within minutes of the oligonucleotide. The antigen may be administered before, at the same time as, or after the formulation.

The antigen formulation may be administered multiple times. In these instances, the first administration of antigen formulation is referred to as a prime dose and subsequent administrations are referred to as a boost dose. The oligonucleotide formulation may be administered with either or both the prime and boost dose. The prime and boost doses preferably are both formulated with immune stimulating complexes.

The invention embraces the administration of one or more antigens in a given vaccination protocol. Accordingly, any given antigen formulation may comprise one or more antigens, and additionally, subsequent antigen formulations may comprise the same or different antigens from prior or subsequent antigen formulations.

An antigen, as used herein, is a molecule capable of provoking an immune response. In most instances, an antigen provokes such a response when presented by an antigen presenting cell in the context of an antigen presenting molecule such as an MHC or CD1 molecule, Antigens include cells, cell extracts, proteins, polypeptides, peptides, polysaccharides, polysaccharide conjugates, peptide and non-peptide mimics of polysaccharides and other molecules, small molecules, .lipids, glycolipids, carbohydrates, viruses and viral extracts, multicellular organisms such as helminth parasites, and allergens. The term broadly includes any type of molecule that is recognized by a host immune system as being foreign. Antigens include cancer antigens, microbial antigens, and allergens.

A cancer antigen, as used herein, is a molecule expressed by a cancer cell that is capable of provoking an immune response when presented by an antigen presenting cell in the context of an antigen presenting molecules such as an MHC or CD 1 molecule. As used herein, the terms "cancer antigen" and "tumor antigen" are used interchangeably. Cancer antigens can be prepared from cancer cells either by preparing crude extracts of cancer cells, for example, as described in Cohen, et al., 1994, Cancer Research, 54:1055, by partially purifying the antigens, by recombinant technology, or by de novo synthesis of known antigens. Cancer antigens can be isolated from naturally occurring sources or prepared recombinantly or by any other means known in the art.

Some cancer antigens are encoded, although not necessarily expressed, by normal cells. These antigens can be characterized as those which are normally silent (i.e., not expressed) in normal cells, those that are expressed only at certain stages of normal cell differentiation, or those that are normally temporally expressed such as at embryonic or fetal stages of development. Other cancer antigens are encoded by mutant cellular genes, such as oncogenes (e.g., activated ras oncogene), suppressor genes (e.g., mutant p53), fusion proteins resulting from internal deletions or chromosomal translocations. Still other cancer antigens can be encoded by viral genes such as those carried on RNA and DNA tumor viruses.

In some embodiments, the cancer antigen is selected from the group consisting of MART-1/Melan-A, gp100, adenosine deaminase-binding protein (ADAbp), FAP, cyclophilin b, colorectal associated antigen (CRC)--C017-1A/GA733, carcinoembryonic antigen (CEA), CAP-1, CAP-2, etv6, AML1, prostate specific antigen (PSA), PSA-1, PSA-2, PSA-3, prostate-specific membrane antigen (PSMA), T-cell receptor/CD3-zeta chain, and CD20.

In other embodiments, the cancer antigen is selected from the group consisting of MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5).

In still other embodiments, the cancer antigen is selected from the group consisting of GAGE-1, GAGE-2, GAGE-3, GAGE-4, GAGE-5, GAGE-6, GAGE-7, GAGE-8, GAGE-9.

In yet other embodiments, the cancer antigen is selected from the group consisting of BAGE, RAGE, LAGE-1, NAG, GnT-V, MUM-1, CDK4, tyrosinase, p53, MUC family, HER2/neu, p21ras, RCAS1, α-fetoprotein, E-cadherin, α-catenin, β-catenin, γ-catenin, p120ctn, gp100^{Pmel117}, PRAME, NY-ESO-1, cdc27, adenomatous polyposis coli protein (APC), fodrin, Connexin 37, Ig-idiotype, p15, gp75, GM2 ganglioside, GD2 ganglioside, human papilloma virus proteins, Smad family of tumor antigens, lmp-1, PIA, EBV-encoded nuclear antigen (EBNA)-1, brain glycogen phosphorylase, SSX-1, SSX-2 (HOM-MEL-40), SSX-1, SSX-4, SSX-5, SCP-1 and CT-7, and c-erbB-2.

In still other embodiments, the cancer antigen is selected from the group consisting of CD20, CD22, CD52, CD33, CD10 (gp100), CD3/T-cell receptor (TCR), CD79/B-cell receptor (BCR), CD26, Human leukocyte antigen (HLA)-DR, HLA-DP, and HLA-DQ, RCAS1, Prostate specific membrane antigen (PSMA), PSA, EGFR/HER1/erbB1, EGFRvIII, erbB2/HER2/neu), erbB3/HER3, erbB4/HER4m Tyrosinase, Melan-A/MART-1, tyrosinase related protein (TRP)-1/gp75, Polymorphic epithelial mucin (PEM), Human epithelial mucin (MUC1), α-fetoprotein, KaMikreins 6 and 10, Gastrin-releasing peptide/bombesin, Prostate specific antigen, and a cancer testis (CT) antigen.

A microbial antigen, as used herein, is a molecule deriving from an infectious pathogen including but not limited to bacteria, viruses, fungi, parasites and mycobacteria and capable of provoking an immune response when presented by an antigen presenting cell in the context of an antigen presenting molecules such as an MHC or CD1 molecule. Such antigens include the intact microbe and natural isolates and fragments or derivatives thereof, as well as synthetic compounds which are identical or similar to natural microbial antigens and induce an immune response specific for that microbe. A compound is similar to a natural microbial antigen if it induces an immune response (humoral and/or cellular) to a natural microbial antigen. Such antigens are used routinely in the art and are known to those of ordinary skill in the art.

Categories of viruses that have been found in humans and which can be used as antigens or sources of viral antigens according to the invention include *Retroviridae* (e.g. human immunodeficiency viruses, such as HIV-1 (also referred to as HDTV-III, LAVE or HTLV-III/LAV, or MIV-III; and other isolates, such as HIV-LP; *Picornaviridae* (e.g. polio viruses, hepatitis A virus; enteroviruses, human Coxsackie viruses, rhinoviruses, echoviruses); *Calciviridae* (e.g. strains that cause gastroenteritis); *Togaviridae* (e.g. equine encephalitis viruses, rubella viruses); *Flaviridae* (e.g. dengue viruses, encephalitis viruses, yellow fever viruses); *Coronoviridae* (e.g. coronaviruses); *Rhabdoviradae* (e.g. vesicular stomatitis viruses, rabies viruses); *Filoviridae* (e.g. ebola viruses); *Paramyxoviridae* (e.g. parainfluenza viruses, mumps virus, measles virus, respiratory syncytial virus); *Orthomyxoviridae* (e.g. influenza viruses); *Bungaviridae* (e.g. Hantaan viruses, bunga viruses, phleboviruses and Nairo viruses); Arena viridae (hemorrhagic fever viruses); *Reoviridae* (e.g. reoviruses, orbiviurses and rotaviruses); *Birnaviridae*; *Hepadnaviridae* (Hepatitis B virus); *Parvovirida* (parvoviruses); *Papovaviridae* (papilloma viruses, polyoma viruses); *Adenoviridae* (most adenoviruses); *Herpesviridae* (herpes siniplex virus (HSV) 1 and 2, varicella zoster virus, cytomegalovirus (CMV), herpes virus; *Poxviridae* (variola viruses, vaccinia viruses, pox viruses); and *Iridoviridae* (e.g. African swine fever virus); and unclassified viruses (e.g. the agent of delta hepatitis (thought to be a defective satellite of hepatitis B virus), the agents of non-A, non-B hepatitis (class 1 = internally transmitted; class 2 = parenterally transmitted (i.e. Hepatitis C); Norwalk and related viruses, and astroviruses).

Both gram negative and gram positive bacteria serve as antigens or sources of antigens in vertebrate animals. Such gram positive bacteria include *Pasteurella* species, *Staphylococci* species, and *Streptococcus* species. Gram negative bacteria include *Escherichia coli, Pseudomonas* species, and *Salmonella* species. Specific examples of infectious bacteria include *Helicobacter pyloris, Borelia burgdorferi, Legionella pneumophilia, Mycobacteria sps* (e.g. *M. tuberculosis, M. avium, M. intracellulare, M. kansaii, M. gordonae*), *Staphylococcus aureus, Neisseria gonorrhoeae, Neisseria meningitidis, Listeria monocytogenes, Streptococcus pyogenes* (Group A Streptococcus), *Streptococcus agalactiae* (Group B Streptococcus), *Streptococcus* (viridans group), *Streptococcus faecalis, Streptococcus bovis, Streptococcus* (anaerobic sps.), *Streptococcus pneumoniae,* pathogenic *Campylobacter sp., Enterococcus sp., Haemophilus influenzae, Bacillus antracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, Rickettsia,* and *Actinomyces israelli.*

Polypeptide antigens of bacterial pathogens include an iron-regulated outer membrane protein, (IROMP), an outer membrane protein (OMP), and an A-protein of Aeromonis salmonicida which causes furunculosis, p57 protein of Renibacterium salmoninarum which causes bacterial kidney disease (BKD), major surface associated antigen (msa), a surface expressed cytotoxin (mpr), a surface expressed hemolysin (ish), and a flagellar antigen of Yersiniosis; an extracellular protein (ECP), an iron-regulated outer membrane protein (IROMP), and a structural protein of Pasteurellosis; an OMP and a flagellar protein of Vibrosis anguillarum and V. ordalii; a flagellar protein, an OMP protein, aroA, and purA of Edwardsiellosis ictaluri and E. tarda; and surface antigen of Ichthyophthirius; and a structural and regulatory protein of Cytophaga columnari; and a structural and regulatory protein of Rickettsia.

Examples of fungi that act as antigens or as antigen sources may include *Cryptococcus neoformans, Histoplasma capsulatum, Coccidioides immitis, Blastomyces dermatitidis, Chlamydia trachomatis,* and *Candida albicans.*

Other infectious organisms that are antigens or antigen sources include *Plasmodium spp.* such as *Plasmodium falciparum, Plasmodium malariae, Plasmodium ovale,* and *Plasmodium* vivax and *Toxoplasma gondii.* Blood-borne and/or tissues parasites include *Plasmodium* spp., *Babesia microti, Babesia divergens, Leishmania tropica, Leishmania* spp., *Leishmania braziliensis, Leishmania donovani, Trypanosoma gambiense* and *Trypanosoma rhodesiense* (African sleeping sickness), *Trypanosoma cruzi* (Chagas' disease), and *Toxoplasma gondii.*

Mycobacteria that act as antigens or antigen sources include *Mycobacterium leprae* and *Mycobacterium tuberculosis.*

Other medically relevant microorganisms have been described extensively in the literature, e.g., see C.G.A Thomas, Medical Microbiology, Bailliere Tindall, Great Britain 1983, the entire contents of which is hereby incorporated by reference.

An allergen is an antigen that can induce an allergic or asthmatic response in a susceptible subject. The list of allergens is enormous and includes pollens, insect venoms, animal dander dust, fungal spores and drugs (e.g. penicillin). Examples of natural, animal and plant allergens include proteins specific to the following genera: *Canine (Canis familiaris)*; *Dermatophagoides* (e.g. *Dermatophagoides farinae*); *Felis* (*Felis domesticus*); *Ambrosia* (*Ambrosia artemiisfolia*; *Lolium* (e.g. *Lolium perenne* or *Lolium multiflorum*); *Cryptomeria* (*Cryptomeria japonica*); *Alternaria* (*Altenaria a*/*ternata*); *Alder*; *Alnus* (*Ainus gultinoasa*); *Betula* (*Betula verrucosa*); *Quercus* (*Quercus alba*); *Olea* (*Olea europa*); *Artemisia* (*Artemisia vulgaris*); *Plantago* (e.g. *Plantago lanceo*/*ata*); *Parietaria* (e.g. *Parietaria offcinalis* or *Parietaria judaica*); *Blattella* (e.g. *Blattella germanica*); *Apis* (e.g. *Apis multiflorum*); *Cupressus* (e.g. *Cupressus sempervirens, Cupressus arizonica* and *Cupressus macrocarpa*); *Juniperus* (e.g. *Juniperus sabinoides, Juniperus virginiana, Juniperus communis* and *Junipenus ashei*); *Thuya* (e.g. *Thuya orientalis); Chamaecyparis* (e.g. *Chamaecyparis obtusa*); *Periplaneta* (e.g. *Periplaneta americana*); *Agropyron* (e.g. *Agropyron repens*); *Secale* (e.g. *Secale cereale*); *Triticum* (e.g. *Triticum aestivum*); *Dactylis* (e.g. *Dactylis glomerata*); *Festuca* (e.g. *Festuca elatior*); *Poa* (e.g. *Poa pratensis* or *Poa compressa*); *Avena* (e.g. *Avena sativa*); *Holcus* (e.g. *Holcus lanatus*); *Anthoxanthum* (e.g. *Anthoxanthum odoratum*); *Arrhenatherum* (e.g. *Arrhenatherum elatius*); *Agrostis* (e.g. *Agrostis alba*); *Phleum* (e.g. *Phleum pratense*); *Phalaris* (e.g. *Phalaris arundinacea*); *Paspalum* (e.g. *Paspalum notatum*); *Sorghum* (e.g. *Sorghum halepensis*); and *Bromus* (e.g. *Bromus inermis*).

The antigen may be encoded by a nucleic acid vector or it may not be encoded by a nucleic acid vector. In the former case the nucleic acid vector is administered to the subject and the antigen is expressed in vivo. In the latter case the antigen may be administered directly to the subject. An antigen not encoded in a nucleic acid vector refers to any type of antigen that is not a nucleic acid. For instance, in some aspects of the invention the antigen not encoded in a nucleic acid vector is a polypeptide. Minor modifications of the primary amino acid sequences of polypeptide antigens may also result in a polypeptide which has substantially equivalent antigenic activity as compared to the unmodified counterpart polypeptide. Such modifications may be deliberate, as by site-directed mutagenesis, or may be spontaneous. All of the polypeptides produced by these modifications are included herein as long as antigenicity still exists. The polypeptide may be, for example, a viral polypeptide.

The invention also utilizes polynucleotides encoding the antigenic polypeptides. It is envisioned that the antigen may be delivered to the subject in a nucleic acid molecule which encodes for the antigen such that the antigen must be expressed in vivo. Such antigens delivered to the subject in a nucleic acid vector are referred to as antigens encoded by a nucleic acid vector. The nucleic acid encoding the antigen is operatively linked to a gene expression sequence which directs the expression of the antigen nucleic acid within a eukaryotic cell. The gene expression sequence is any regulatory nucleotide sequence, such as a promoter sequence or promoter-enhancer combination, which facilitates the efficient transcription and translation of the antigen nucleic acid to which it is operatively linked. The gene expression sequence may, for example, be a mammalian or viral promoter, such as a constitutive or inducible promoter. Constitutive mammalian promoters include, but are not limited to, the promoters for the following genes: hypoxanthine phosphoribosyl transferase (HPTR), adenosine deaminase, pyruvate kinase, b-actin promoter and other constitutive promoters. Exemplary viral promoters which function constitutively in eukaryotic cells include, for example, promoters from the cytomegalovirus (CMV), simian virus (e.g., SV40), papilloma virus, adenovirus, human immunodeficiency virus (HIV), Rous sarcoma virus, cytomegalovirus, the long terminal repeats (LTR) of Moloney leukemia virus and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art.

In general, the gene expression sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operably joined antigen nucleic acid. The gene expression sequences optionally include enhancer sequences or upstream activator sequences as desired.

The antigen nucleic acid is operatively linked to the gene expression sequence. As used herein, the antigen nucleic acid sequence and the gene expression sequence are said to be operably linked when they are covalently linked in such a way as to place the expression or transcription and/or translation of the antigen coding sequence under the influence or control of the gene expression sequence. Two DNA sequences are said to be operably linked if induction of a promoter in the 5' gene expression sequence results in the transcription of the antigen sequence and if the nature of the linkage between the two DNA sequences does not (1) result in the introduction of a frame-shift mutation, (2) interfere with the ability of the promoter region to direct the transcription of the antigen sequence, or (3), interfere with the ability of the corresponding RNA transcript to be translated into a protein. Thus, a gene expression sequence would be operably linked to an antigen nucleic acid sequence if the gene expression sequence were capable of effecting transcription of that antigen nucleic acid sequence such that the resulting transcript is translated into the desired protein or polypeptide.

The antigen-encoding nucleic acids may be delivered to the immune system alone or in association with a vector. In its broadest sense, a vector is any vehicle capable of facilitating the transfer of the antigen-encoding nucleic acid to the cells of the immune system so that the antigen can be expressed and presented on the surface of the immune cell. The vector generally transports the nucleic acid to the immune cells with reduced degradation relative to the extent of degradation that would result in the absence of the vector. The vector optionally includes the above-described gene expression sequence to enhance expression of the antigen nucleic acid in immune cells. In general, the vectors useful in the invention include, but are not limited to, plasmids, phagemids, viruses, other vehicles derived from viral or bacterial sources that have been manipulated by the insertion or incorporation of the antigen nucleic acid sequences. Viral vectors are a preferred type of vector and include, but are not limited to, nucleic acid sequences from the following viruses: retrovirus, such as Moloney murine leukemia virus, Harvey murine sarcoma virus, murine mammary tumor virus, and Rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known in the art.

Preferred viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, M., Gene Transfer and Expression, A Laboratory Manual W. H. Freeman C.O., New York (1990) and Murry, E. J. Methods in Molecular Biology, vol. 7, Humana Press, Inc., Cliffton, New Jersey (1991).

A preferred virus for certain applications is the adeno-associated virus, a double-stranded DNA virus. The adeno-associated virus can be engineered to be replication-deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well-known to those of skill in the art. See e.g., Sambrook et al., Molecular Cloning: A Laboratory Manual, Second Edition, Cold Spring Harbor Laboratory Press, 1989. In the last few years, plasmid vectors have been found to be particularly advantageous for delivering genes to cells in vivo because of their inability to replicate within and integrate into a host genome. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well-known to those of ordinary skill in the art. Additionally, plasmids may be custom designed using restriction enzymes and ligation reactions to remove and add specific fragments of DNA.

It has recently been discovered that gene carrying plasmids can be delivered to the immune system using bacteria. Modified forms of bacteria such as Salmonella can be transfected with the plasmid and used as delivery vehicles. The bacterial delivery vehicles can be administered to a host subject orally or by other administration means. The bacteria deliver the plasmid to immune cells, e.g. B cells, dendritic cells, likely by passing through the gut barrier. High levels of immune protection have been established using this methodology. Such methods of delivery are useful for the aspects of the invention utilizing systemic delivery of antigen, Immunostimulatory nucleic acid and/or other therapeutic agent.

### Isolated:

Antigens of the invention are commonly used in their isolated forms. An isolated form is one in which the substance has been physically separated from the components with which it is normally exists or can be found. For example, an antigen from a tumor is said to be isolated if it is physically separated from the tumor from which it derived, possibly from the cells which express the antigen, and possibly also from other components of such cells.

The term substantially purified, as used herein, refers to a substance that is substantially free of proteins, lipids, carbohydrates or other materials with which it is naturally associated. One skilled in the art can purify viral or bacterial polypeptides using standard techniques for protein purification. A substantially pure polypeptide will often yield a single major band on a non-reducing polyacrylamide gel. In the case of partially glycosylated polypeptides or those that have several start codons, there may be several bands on a non-reducing polyacrylamide gel, but these will form a distinctive pattern for that polypeptide. The purity of the viral or bacterial polypeptide can also be determined by amino-terminal amino acid sequence analysis.

The oligonucleotides of the invention are also commonly used in their isolated forms. An isolated oligonucleotide is an oligonucleotide that is physically separated from those substances with which it is normally associated. If the oligonucleotide is produced from naturally occurring sources, then it is isolated if it is physically separated from other components of that naturally occurring source such as cells, proteins, nuclei, chromosomes, etc.

### Disease Treatment:

As used herein, the terms treat, treated, or treating when used with respect to a disorder, such as an infectious disease, cancer or allergy, refers to prophylactic treatment which increases the resistance of a subject to development of the disease (e.g., to infection with a pathogen) or, in other words, decreases the likelihood that the subject will develop the disease (e.g., become infected with the pathogen) as well as a therapeutic treatment after the subject has developed the disease in order to fight the disease (e.g., reduce or eliminate the infection) or prevent the disease from becoming worse.

The medicaments described herein are useful therapeutically and prophylactically for stimulating the immune system to form antigen-specific immune responses necessary to treat cancer, infectious disease, allergy, asthma and other, disorders. The medicaments demonstrate unexpectedly better immune stimulatory effects as compared to other adjuvant combinations. For example, the medicaments induce unexpectedly high levels of IFN-gamma, activate CTLs and enhance Th-1-induced immunoglobulin production, indicating they will be more effective than originally expected (as well as more effective than other combinations including adjuvants having depot effects and immune stimulating activity) for vaccination.

### Subjects:

The terms "subject" and "patient" are used interchangeably herein, and refer to a human or other vertebrate animal including but not limited to, a dog, cat, horse, cow, pig, sheep, goat, turkey, chicken, primate, e.g., monkey, and fish (aquaculture species), e.g. salmon. The invention can be used to treat cancer and tumors, infections, and allergy/asthma in human and non-human subjects. Cancer is one of the leading causes of death in companion animals (e.g., cats and dogs).

A subject at risk, as used herein, is a subject who has a higher than normal risk of developing an infection, or a cancer, or an allergy.

A subject at risk of developing an infection may be a subject who is planning to travel to an area where a particular type of infectious agent is prevalent or it may be a subject who through lifestyle or medical procedures is exposed to bodily fluids which may contain infectious organisms or directly to the organism or even any subject living in an area where an infectious organism has been identified. Subjects at risk of developing infection also include general populations to which a medical agency recommends vaccination with a particular microbial antigen.

A subject having an infection is a subject that has been exposed to an infectious pathogen and has acute or chronic detectable levels of the pathogen in the body. An infectious disease, as used herein, is a disease arising from the presence of a foreign microorganism in the body. It is particularly important to develop effective vaccine strategies and treatments to protect the body's mucosal surfaces, which are the primary site of pathogenic entry.

The infectious disease may be a bacterial infection, a viral infection, a fungal infection, a parasitic infection, or a mycobacterial infection, although it is not so limited. Examples of these are listed herein and supplemented below.

The bacterial infection may be but is not limited to an Actinomyces infection, an anthrax infection, a Bacteriodes infection, a Borrelia infection, a Campylobacter infection, a Citrobacter infection, a Clostridium difficile infection, a Corynebacterium infection, an E. coli infection, an Enterobacter infection, a Gardnerella infection, a Haemophilus infection, an H. pylori infection, a Klebsiella infection, a Legionella infection, a Listeria infection, a Neisseria infection, a Nocardia infection, a Pasteurella infection, a Pneumococcus infection, a Proteus infection, a Pseudomonas infection,a Salmonella infection, a Shigella infection, a Spirillum infection, a Spirochaeta infection, a Staphylococcal infection, a Streptobacillus infection, a Streptococcal infection, and a Treponema infection.

The viral infection may be but is not limited to an adenovirus infection, a retrovirus infection, a rotavirus infection, etc. It may be but is not limited to a cytomegalovirus infection, an Epstein Barr virus infection, a hepatitis A virus infection, a hepatitis B virus infection, a hepatitis C virus infection, a Herpes simplex virus 1 infection, a Herpes simplex virus 2 infection, an HIV infection, a human papilloma virus infection, an influenza A virus infection, a monkey pox infection, a respiratory syncytial virus infection, a SARS infection a small pox infection, a varicella-zoster virus infection. In some embodiments, the infectious disease is a chronic infectious disease such as a chronic viral infection. Examples include hepatitis virus infection, human papilloma virus infection, HIV infection, and Herpes simplex virus infection.

Infectious viruses of both human and non-human vertebrates, include retroviruses, RNA viruses and DNA viruses. This group of retroviruses includes both simple retroviruses and complex retroviruses. The simple retroviruses include the subgroups of B-type retroviruses, C-type retroviruses and D-type retroviruses. An example of a B-type retrovirus is mouse mammary tumor virus (MMTV). The C-type retroviruses include subgroups C-type group A (including Rous sarcoma virus (RSV), avian leukemia virus (ALV), and avian myeloblastosis virus (AMV)) and C-type group B (including feline leukemia virus (FeLV), gibbon ape leukemia virus (GALV), spleen necrosis virus (SNV), reticuloendotheliosis virus (RV) and simian sarcoma virus (SSV)). The D-type retroviruses include Mason-Pfizer monkey virus (MPMV) and simian retrovirus type 1 (SRV-1). The complex retroviruses include the subgroups of lentiviruses, T-cell leukemia viruses and the foamy viruses. Lentiviruses include HIV-1, but also include HIV-2, SIV, Visna virus, feline immunodeficiency virus (FIV), and equine infectious anemia virus (EIAV). The T-cell leukemia viruses include HTLV-1, HTLV-II, simian T-cell leukemia virus (STLV), and bovine leukemia virus (BLV). The foamy viruses include human foamy virus (HFV), simian foamy virus (SFV) and bovine foamy virus (BFV).

Examples of other RNA viruses that are antigens in vertebrate animals include, but are not limited to, members of the family Reoviridae, including the genus Orthoreovirus (multiple serotypes of both mammalian and avian retroviruses), the genus Orbivirus (Bluetongue virus, Eugenangee virus, Kemerovo virus, African horse sickness virus, and Colorado Tick Fever virus), the genus Rotavirus (human rotavirus, Nebraska calf diarrhea virus, simian rotavirus, bovine or ovine rotavirus, avian rotavirus); the family Picornaviridae, including the genus Enterovirus (poliovirus, Coxsackie virus A and B, enteric cytopathic human orphan (ECHO) viruses, hepatitis A virus, Simian enteroviruses, Murine encephalomyelitis (ME) viruses, Poliovirus muris, Bovine enteroviruses, Porcine enteroviruses, the genus Cardiovirus (Encephalomyocarditis virus (EMC), Mengovirus), the genus Rhinovirus (Human rhinoviruses including at least 113 subtypes; other rhinoviruses), the genus Apthovirus (Foot and Mouth disease (FMDV); the family Calciviridae, including Vesicular exanthema of swine virus, San Miguel sea lion virus, Feline picornavirus and Norwalk virus; the family Togaviridae, including the genus Alphavirus (Eastern equine encephalitis virus, Semliki forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (Measles virus, subacute sclerosing panencephalitis virus, distemper virus, Rinderpest virus), the genus Pneumovirus (respiratory syncytial virus (RSV), Bovine respiratory syncytial virus and Pneumonia virus); the family Rhabdoviridae, including the genus Vesiculovirus (VSV), Chandipura virus, Flanders-Hart Park virus), the genus Lyssavirus (Rabies virus), fish Rhabdoviruses, and two probable Rhabdoviruses (Marburg virus and Ebola virus); the family Arenaviridae, including Lymphocytic choriomeningitis virus (LCM), Tacaribe virus complex, and Lassa virus; the family Coronoaviridae, including Infectious Bronchitis Virus (IBV), Hepatitis virus, Human enteric corona virus, and Feline infectious peritonitis (Feline coronavirus).

Illustrative DNA viruses that are antigens in vertebrate animals include, but are not limited to, the family Poxyiridae, including the genus Orthopoxyirus (Variola major, Variolaminor, Monkey pox Vaccinia, Cowpox, Buffalopox, Rabbitpox, Ectromelia), the genus Leporipoxyirus (Myxoma, Fibroma), the genus Avipoxyirus (Fowlpox, other avian poxyirus), the genus Capripoxyirus (sheeppox, goatpox), the genus Suipoxyirus (Swinepox), the genus Parapoxyirus (contagious postular dermatitis virus, pseudocowpox, bovine papular stomatitis virus); the family Iridoviridae (African swine fever virus, Frog viruses 2 and 3, Lymphocystis virus of fish); the family Herpesviridae, including the alpha-Herpesviruses (Herpes Simplex Types 1 and 2, Varicella-Zoster, Equine abortion virus, Equine herpes virus 2 and 3, pseudorabies virus, infectious bovine keratoconjunctivitis virus, infectious bovine rhinotracheitis virus, feline rhinotracheitis virus, infectious laryngotracheitis virus) the Beta-herpesviruses (Human cytomegalovirus and cytomegaloviruses of swine and monkeys); the gamma-herpesviruses (Epstein-Barr virus (EBV), Marek's disease virus, Herpes saimiri, Herpesvirus ateles, Herpesvirus sylvilagus, guinea pig herpes virus, Lucke tumor virus); the family Adenoviridae, including the genus Mastadenovirus (Human subgroups A,B,C,D,E and ungrouped; simian adenoviruses (at least 23 serotypes), infectious canine hepatitis, and adenoviruses of cattle, pigs, sheep, frogs and many other species, the genus Aviadenovirus (Avian adenoviruses); and non-cultivatable adenoviruses; the family Papoviridae, including the genus Papillomavirus (Human papilloma viruses, bovine papilloma viruses, Shope rabbit papilloma virus, and various pathogenic papilloma viruses of other species), the genus Polyomavirus (polyomavirus, Simian vacuolating agent (SV-40), Rabbit vacuolating agent (RKV), K virus, BK virus, JC virus, and other primate polyoma viruses such as Lymphotrophic papilloma virus); the family Parvoviridae including the genus Adeno-associated viruses, the genus Parvovirus (Feline panleukopenia virus, bovine parvovirus, canine parvovirus, Aleutian mink disease virus, etc).

The fungal infection may be but is not limited to aspergillosis, blastomycosis, candidiasis, chromomycosis, crytococcosis, histoplasmosis, mycetoma infections, paracoccidioidomycosis, pseudallescheriasis, ringworm, and tinea versicolor infection.

The parasitic infection may be but is not limited to amebiasis, Echinococcus infections, Fascioliasis, Hymenolepsis infection, Leishmaniasis, Onchocerciasis, Necator americanus infection, neurocysticercosis, Paragonimiasis, Plasmodium infections, Pneumocystis infection, Schistosomiasis, Taenia infection, Trichomonas vaginalis infection, Trichuris trichuria infection, Trypanosoma brucei infection and Trypanosoma cruzi infection.

The mycobacterial infection may be but is not limited to *M. tuberculosis* and *M. leprae.*

The methods for inducing antigen-specific immune responses are well suited for treatment of birds such as hens, chickens, turkeys, ducks, geese, quail, and pheasant. Birds are prime targets for many types of infections.

Hatching birds are exposed to pathogenic microorganisms shortly after birth. Although these birds are initially protected against pathogens by maternal derived antibodies, this protection is only temporary, and the bird's own immature immune system must begin to protect the bird against the pathogens. It is often desirable to prevent infection in young birds when they are most susceptible. It is also desirable to prevent against infection in older birds, especially when the birds are housed in closed quarters, leading to the rapid spread of disease. Thus, it is desirable to administer the formulations of the invention to birds to enhance an antigen-specific immune response.

An example of a common infection in chickens is chicken infectious anemia virus (CIAV). CIAV was first isolated in Japan in 1979 during an investigation of a Marek's disease vaccination break (Yuasa et al., 1979, Avian Dis. 23:366-385). Since that time, CIAV has been detected in commercial poultry in all major poultry producing countries (van Bulow et al., 1991, pp.690-699) in Diseases of Poultry, 9th edition, Iowa State University Press).

CIAV infection results in a clinical disease, characterized by anemia, hemorrhage and immunosuppression, in young susceptible chickens. Atrophy of the thymus and of the bone marrow and consistent lesions of CIAV-infected chickens are also characteristic of CIAV infection. Lymphocyte depletion in the thymus, and occasionally in the bursa of Fabricius, results in immunosuppression and increased susceptibility to secondary viral, bacterial, or fungal infections which then complicate the course of the disease. The immunosuppression may cause aggravated disease after infection with one or more of Marek's disease virus (MDV), infectious bursal disease virus, reticuloendotheliosis virus, adenovirus, or reovirus. It has been reported that pathogenesis of MDV is enhanced by CIAV (DeBoer et al., 1989, p. 28 In Proceedings of the 38th Western Poultry Diseases Conference, Tempe, Ariz.). Further, it has been reported that CIAV aggravates the signs of infectious bursal disease (Rosenberger et al., 1989, Avian Dis. 33:707-713). Chickens develop an age resistance to experimentally induced disease due to CAA. This is essentially complete by the age of 2 weeks, but older birds are still susceptible to infection (Yuasa, N. et al., 1979 supra; Yuasa, N, et al., Arian Diseases 24, 202-209, 1980). However, if chickens are dually infected with CAA and an immunosuppressive agent (IBDV, MDV etc.), age resistance against the disease is delayed (Yuasa, N. et al., 1979 and 1980 supra; Bulow von V. et al., J. Veterinary Medicine 33, 93-116, 1986). Characteristics of CIAV that may potentiate disease transmission include high resistance to environmental inactivation and some common disinfectants. The economic impact of CIAV infection on the poultry industry is clear from the fact that 10% to 30% of infected birds in disease outbreaks die.

Vaccination of birds, like other vertebrate animals can be performed at any age. Normally, vaccinations are performed at up to 12 weeks of age for a live microorganism and between 14-18 weeks for an inactivated microorganism or other type of vaccine. For in ovo vaccination, vaccination can be performed in the last quarter of embryo development. Thus, the formulations provided herein can be administered to birds and other non-human vertebrates using routine vaccination schedules and the antigen can be administered after an appropriate time period as described herein.

Cattle and livestock are also susceptible to infection. Diseases which affect these animals can produce severe economic losses, especially amongst cattle. The methods of the invention can be used to protect against infection in livestock, such as cows, horses, pigs, sheep, and goats.

Cows can be infected by bovine viruses. Bovine viral diarrhea virus (BVDV) is a small enveloped positive-stranded RNA virus and is classified, along with hog cholera virus (HOCV) and sheep border disease virus (BDV), in the pestivirus genus. Although, pestiviruses were previously classified in the togaviridae family, some studies have suggested their reclassification within the flaviviridae family along with the flavivirus and hepatitis C virus (HCV) groups (Francki, et al., 1991).

BVDV, which is an important pathogen of cattle can be distinguished, based on cell culture analysis, into cytopathogenic (CP) and noncytopathogenic (NCP) biotypes. The NCP biotype is more widespread although both biotypes can be found in cattle. If a pregnant cow becomes infected with an NCP strain, the cow can give birth to a persistently infected and specifically immunotolerant calf that will spread virus during its lifetime. The persistently infected cattle can succumb to mucosal disease and both biotypes can then be isolated from the animal. Clinical manifestations can include abortion, teratogenesis, and respiratory problems, mucosal disease and mild diarrhea. In addition, severe thrombocytopenia, associated with herd epidemics, that may result in the death of the animal has been described and strains associated with this disease seem more virulent than the classical BVDVs.

Equine herpes viruses (EHV) comprise a group of antigenically distinct biological agents which cause a variety of infections in horses ranging from subclinical to fatal disease. These include Equine herpesvirus-1 (EHV-1), a ubiquitous pathogen in horses. EHV-1 is associated with epidemics of abortion, respiratory tract disease, and central nervous system disorders. Primary infection of upper respiratory tract of young horses results in a febrile illness which lasts for 8 to 10 days. Immunologically experienced mares may be re-infected via the respiratory tract without disease becoming apparent, so that abortion usually occurs without warning. The neurological syndrome is associated with respiratory disease or abortion and can affect animals of either sex at any age, leading to lack of coordination, weakness and posterior paralysis (Telford, E. A. R. et al., Virology 189, 304-316,1992). Other EHV's include EHV-2, or equine cytomegalovirus, EHV-3, equine coital exanthema virus, and EHV-4, previously classified as EHV-1 subtype 2.

Sheep and goats can be infected by a variety of dangerous microorganisms including visna-maedi.

Primates such as monkeys, apes and macaques can be infected by simian immunodeficiency virus. Inactivated cell-virus and cell-free whole simian immunodeficiency vaccines have been reported to afford protection in macaques (Stott et al. (1990) Lancet 36:1538-1541; Desrosiers et al. PNAS USA (1989) 86:6353-6357; Murphey-Corb et al. (1989) Science 246:1293-1297; and Carlson et al. (1990) ADS Res. Human Retroviruses 6:1239-1246). A recombinant HIV gp120 vaccine has been reported to afford protection in chimpanzees (Berman et al. (1990) Nature 345:622-625).

Cats, both domestic and wild, are susceptible to infection with a variety of microorganisms. For instance, feline infectious peritonitis is a disease which occurs in both domestic and wild cats, such as lions, leopards, cheetahs, and jaguars. When it is desirable to prevent infection with this and other types of pathogenic organisms in cats, the methods of the invention can be used to vaccinate cats to protect them against infection.

Domestic cats may become infected with several retroviruses, including but not limited to feline leukemia virus (FeLV), feline sarcoma virus (FeSV), endogenous type Concornavirus (RD-114), and feline syncytia-forming virus (FeSFV). Of these, FeLV is the most significant pathogen, causing diverse symptoms, including lymphoreticular and myeloid neoplasms, anemias, immune mediated disorders, and an immunodeficiency syndrome which is similar to human acquired immune deficiency syndrome (AIDS). Recently, a particular replication-defective FeLV mutant, designated FeLV-AIDS, has been more particularly associated with immunosuppressive properties.

The discovery of feline T-lymphotropic lentivirus (also referred to as feline immunodeficiency) was first reported in Pedersen et al. (1987) Science 235:790-793. Characteristics of FIV have been reported in Yamamoto et al. (1988) Leukemia, December Supplement 2:204S-215S; Yamamoto et al. (1988) Am. J. Vet. Res. 49:1246-1258; and Ackley et al. (1990) J. Virol. 64:5652-5655. Cloning and sequence analysis of FIV have been reported in Ohnsted et al. (1989) Proc. Natl. Acad. Sci. USA 86:2448-2452 and 86;4355-4360.

Feline infectious peritonitis (FIP) is a sporadic disease occurring unpredictably in domestic and wild Felidae. While FIP is primarily a disease of domestic cats, it has been diagnosed in lions, mountain lions, leopards, cheetahs, and the jaguar. Smaller wild cats that have been afflicted with FIP include the lynx and caracal, sand cat, and pallas cat. In domestic cats, the disease occurs predominantly in young animals, although cats of all ages are susceptible. A peak incidence occurs between 6 and 12 months of age. A decline in incidence is noted from 5 to 13 years of age, followed by an increased incidence in cats 14 to 15 years old.

Viral, bacterial, and parasitic diseases in fin-fish, shellfish or other aquatic life forms pose a serious problem for the aquaculture industry. Owing to the high density of animals in the hatchery tanks or enclosed marine farming areas, infectious diseases may eradicate a large proportion of the stock in, for example, a fin-fish, shellfish, or other aquatic life forms facility. Prevention of disease is a more desired remedy to these threats to fish than intervention once the disease is in progress. Vaccination of fish is the only preventative method which may offer long-term protection through immunity. Nucleic acid based vaccinations are described in U.S. Pat. No. 5,780,448 issued to Davis.

The fish immune system has many features similar to the mammalian immune system, such as the presence of B cells, T cells, lymphokines, complement, and immunoglobulins. Fish have lymphocyte subclasses with roles that appear similar in many respects to those of the B and T cells of mammals. Vaccines can be administered by immersion or orally.

Aquaculture species include but are not limited to fin-fish, shellfish, and other aquatic animals. Fin-fish include all vertebrate fish, which may be bony or cartilaginous fish, such as, for example, salmonids, carp, catfish, yellowtail, seabream, and seabass. Salmonids are a family of fin-fish which include trout (including rainbow trout), salmon, and Arctic char. Examples of shellfish include, but are not limited to, clams, lobster, shrimp, crab, and oysters. Other cultured aquatic animals include, but are not limited to eels, squid, and octopi.

Polypeptides of viral aquaculture pathogens include but are not limited to glycoprotein (G) or nucleoprotein (N) of viral hemorrhagic septicemia virus (VHSV); G or N proteins of infectious hematopoietic necrosis virus (1HNV); VP1, VP2, VP3 or N structural proteins of infectious pancreatic necrosis virus (IPNV); G protein of spring viremia of carp (SVC); and a membrane-associated protein, tegumin or capsid protein or glycoprotein of channel catfish virus (CCV).

Typical parasites infecting horses are Gasterophilus spp.; Eimeria leuckarti, Giardia spp.; Tritrichomonas equi; Babesia spp. (RBC's), Theileria equi; Trypanosoma spp.; Klossiella equi; Sarcocystis spp.

Typical parasites infecting swine include Eimeria bebliecki, Eimeria scabra, Isospora suis, Giardia spp.; Balantidium coli, Entamoeba histolytica; Toxoplasma gondii and Sarcocystis spp., and Trichinella spiralis.

The major parasites of dairy and beef cattle include Eimeria spp., Cryptosporidium sp., Giardia spp.; Toxoplasma gondii; Babesia bovis (RBC), Babesia bigemina (RBC), Trypanosoma spp. (plasma), Theileria spp. (RBC); Theileria parva (lymphocytes); Tritrichomonas foetus; and Sarcocystis spp.

The major parasites of raptors include Trichomonas gallinae; Coccidia (Eimeria spp.); Plasmodium relictum, Leucocytozoon danilewskyi (owls), Haemoproteus spp., Trypanosoma spp.; Histomonas; Cryptosporidium meleagridis, Cryptosporidium baileyi, Giardia, Eimeria; Toxoplasma.

Typical parasites infecting sheep and goats include Eimeria spp., Cryptosporidium sp., Giardia sp.; Toxoplasma gondii; Babesia spp. (RBC), Trypanosoma spp. (plasma), Theileria spp. (RBC); and Sarcocystis spp.

Typical parasitic infections in poultry include coccidiosis caused by Eimeria acervulina, E. necatrix, E. tenella, Isospora spp. and Eimeria truncata; histomoniasis, caused by Histomonas meleagridis and Histomonas gallinarum; trichomoniasis caused by Trichomonas gallinae; and hexamitiasis caused by Hexamita meleagridis. Poultry can also be infected Emeria maxima, Emeria meleagridis, Eimeria adenoeides, Eimeria meleagrimitis, Cryptosporidium, Eimeria brunetti, Emeria adenoeides, Leucocytozoon spp., Plasmodium spp., Hemoproteus meleagridis, Toxoplasma gondii and Sarcocystis.

The methods of the invention can also be applied to the treatment and/or prevention of parasitic infection in dogs, cats, birds, fish and ferrets. Typical parasites of birds include Trichomonas gallinae; Eimeria spp., Isospora spp., Giardia; Cryptosporidium; Sarcocystis spp., Toxoplasma gondii, Haemoproteus/Parahaemoproteus, Plasmodium spp., Leucocytozoon/Akiba, Atoxoplasma, Trypanosoma spp. Typical parasites infecting dogs include Trichinella spiralis; Isopora spp., Sarcocystis spp., Cryptosporidium spp., Hammondia spp., Giardia duodenalis (canis); Balantidium coli, Entamoeba histolytica; Hepatozoon canis; Toxoplasma gondii, Trypanosoma cruzi; Babesia canis; Leishmania amastigotes; Neospora caninum.

Typical parasites infecting feline species include Isospora spp., Toxoplasma gondii, Sarcocystis spp., Hammondia hammondi, Besnoitia spp., Giardia spp.; Entamoeba histolytica; Hepatozoon canis, Cytauxzoon sp., Cytauxzoon sp., Cytauxzoon sp. (red cells, RE cells).

Typical parasites infecting fish include Hexamita spp., Eimeria spp.; Cryptobia spp., Nosema spp., Myxosoma spp., Chilodonella spp., Trichodina spp.; Plistophora spp., Myxosoma Henneguya; Costia spp., Ichthyophithirius spp., and Oodinium spp.

Typical parasites of wild mammals include Giardia spp. (carnivores, herbivores), Isospora spp. (carnivores), Eimeria spp. (carnivores, herbivores); Theileria spp. (herbivores), Babesia spp. (carnivores, herbivores), Trypanosoma spp. (carnivores, herbivores); Schistosoma spp. (herbivores); Fasciola hepatica (herbivores), Fascioloides magna (herbivores), Fasciola gigantica (herbivores), Trichinella spiralis (carnivores, herbivores).

Parasitic infections in zoos can also pose serious problems. Typical parasites of the bovidae family (blesbok, antelope, banteng, eland, gaur, impala, klipspringer, kudu, gazelle) include Eimeria spp. Typical parasites in the pinnipedae family (seal, sea lion) include Eimeria phocae. Typical parasites in the camelidae family (camels, llamas) include Eimeria spp. Typical parasites of the giraffidae family (giraffes) include Eimeria spp. Typical parasites in the elephantidae family (African and Asian) include Fasciola spp. Typical parasites of lower primates (chimpanzees, orangutans, apes, baboons, macaques, monkeys) include Giardia sp.; Balantidium coli, Entamoeba histolytica, Sarcocystis spp., Toxoplasma gondii; Plasmodim spp. (RBC), Babesia spp. (RBC), Trypanosoma spp. (plasma), Leishmania spp. (macrophages).

A subject at risk of developing allergy or asthma includes a subject that has been identified as having an allergy or asthma but that doesn't have the active disease during the immunostimulatory oligonucleotide treatment as well as a subject that is considered to be at risk of developing these diseases because of genetic or environmental factors. If the antigen is an allergen and the subject develops allergic responses to that particular antigen and the subject may be exposed to the antigen, e.g., during pollen season, then that subject is at risk of exposure to the allergen.

A subject having an allergy is a subject that has or is at risk of developing an allergic reaction in response to an allergen. An allergy refers to acquired hypersensitivity to a substance (allergen). Allergic conditions include but are not limited to eczema, allergic rhinitis or coryza, hay fever, conjunctivitis, bronchial asthma, urticaria (hives) and food allergies, and other atopic conditions.

A subject at risk of developing a cancer is one who has a higher than normal probability of developing cancer (i.e., higher than the probability in the general population). These subjects include, for instance, subjects having a genetic abnormality, the presence of which has been demonstrated to have a correlative relation to a higher than normal likelihood of developing a cancer and subjects exposed to cancer causing agents such as tobacco, asbestos, or other chemical toxins, or a subject who has previously been treated for cancer that is in apparent remission. When a subject at risk of developing a cancer is treated with an antigen formulation specific for the type of cancer to which the subject is at risk of developing and a oligonucleotide formulation, the subject may be able to mount an antigen-specific immune response against the cancer cells as they develop.

A subject having a cancer is a subject that has detectable cancerous cells.

Cancers may be carcinoma or sarcoma, but are not so limited. For example, the cancer may be basal cell carcinoma, biliary tract cancer, bladder cancer, bone cancer, brain cancer, breast cancer, cervical cancer, choriocarcinoma, CNS cancer, colon and rectum cancer, connective tissue cancer, cancer of the digestive system, endometrial cancer, esophageal cancer, eye cancer, cancer of the head and neck, gastric cancer, intra-epithelial neoplasm, kidney cancer, larynx cancer, leukemia, acute lymphoid leukemia, acute myeloid leukemia, chronic lymphoid leukemia, chronic myeloid leukemia, cutaneous T-cell leukemia, hairy cell leukemia, liver cancer, non-small cell lung cancer, small cell lung cancer, lymphoma, follicular lymphoma, Hodgkin's lymphoma, Non-Hodgkin's lymphoma, melanoma, myeloma, multiple myeloma, neuroblastoma, oral cavity cancer, ovarian cancer, pancreatic cancer, prostate cancer, rectal cancer, renal cancer, cancer of the respiratory system, retinoblastoma, rhabdomyosarcoma, skin cancer, squamous cell carcinoma, stomach cancer, testicular cancer, thyroid cancer, cancer of the urinary system and uterine cancer.

The invention can be used to treat cancer and tumors in non human subjects. Cancer is one of the leading causes of death in companion animals (i.e., cats and dogs). Cancer usually strikes older animals which, in the case of house pets, have become integrated into the family. Forty-five % of dogs older than 10 years of age, are likely to succumb to the disease. The most common treatment options include surgery, chemotherapy and radiation therapy. Others treatment modalities which have been used with some success are laser therapy, cryotherapy, hyperthermia and immunotherapy. The choice of treatment depends on type of cancer and degree of dissemination. Unless the malignant growth is confined to a discrete area in the body, it is difficult to remove only malignant tissue without also affecting normal cells.

Malignant disorders commonly diagnosed in dogs and cats include but are not limited to lymphosarcoma, osteosarcoma, mammary tumors, mastocytoma, brain tumor, melanoma, adenosquamous carcinoma, carcinoid lung tumor, bronchial gland tumor, bronchiolar adenocarcinoma, fibroma, myxochondroma, pulmonary sarcoma, neurosarcoma, osteoma, papilloma, retinoblastoma, Ewing's sarcoma, Wilm's tumor, Burkitt's lymphoma, microglioma, neuroblastoma, osteoclastoma, oral neoplasia, fibrosarcoma, osteosarcoma and rhabdomyosarcoma Other neoplasias in dogs include genital squamous cell carcinoma, transmissable veneral tumor, testicular tumor, seminoma, Sertoli cell tumor, hemangiopericytoma, histiocytoma, chloroma (granulocytic sarcoma), corneal papilloma, corneal squamous cell carcinoma, hemangiosarcoma, pleural mesothelioma, basal cell tumor, thymoma, stomach tumor, adrenal gland carcinoma, oral papillomatosis, hemangioendothelioma and cystadenoma. Additional malignancies diagnosed in cats include follicular lymphoma, intestinal lymphosarcoma, fibrosarcoma and pulmonary squamous cell carcinoma. The ferret, an ever-more popular house pet is known to develop insulinoma, lymphoma, sarcoma, neuroma, pancreatic islet cell tumor, gastric MALT lymphoma and gastric adenocarcinoma.

Neoplasias affecting agricultural livestock include leukemia, hemangiopericytoma and bovine ocular neoplasia (in cattle); preputial fibrosarcoma, ulcerative squamous cell carcinoma, preputial carcinoma, connective tissue neoplasia and mastocytoma (in horses); hepatocellular carcinoma (in swine); lymphoma and pulmonary adenomatosis (in sheep); pulmonary sarcoma, lymphoma, Rous sarcoma, reticulendotheliosis, fibrosarcoma, nephroblastoma, B-cell lymphoma and lymphoid leukosis (in avian species); retinoblastoma, hepatic neoplasia, lymphosarcoma (lymphoblastic lymphoma), plasmacytoid leukemia and swimbladder sarcoma (in fish), caseous lumphadenitis (CLA): chronic, infectious, contagious disease of sheep and goats caused by the bacterium Corynebacterium pseudotuberculosis, and contagious lung tumor of sheep caused by jaagsiekte.

Prion diseases include a number of fatal, neurodegenerative diseases believed to be caused by aggregates of normal protein that is present in an abnormal conformation. The normal prion protein is usually present in the cell membrane of many tissues, particularly neuronal tissue. The abnormally conformed prion protein is believed to be directly involved in converting normally conformed prion protein into more of the abnormally conformed prion protein, which then self-assembles into aggregates that are damaging to neuronal tissue anatomy and function.

At least some of the prion diseases are transmissible. However, unlike bacteria, viruses, fungi, parasites, and other replicating pathogens, transmissible prions are simply proteins; they are transmissible without any accompanying nucleic acid. For reasons that are not yet fully understood, the abnormally conformed prion proteins generally do not induce an immune response. Thus, exposure of a healthy individual to abnormally conformed prion protein can initiate a prion disease that can go unchecked by the immune system.

The formulations of the invention are useful in the treatment of prion diseases, including Creutzfeldt-Jakob disease (CJD), bovine spongiform encephalopathy (BSE), and scrapie. The CJD may be iatrogenic CJD (iCJD), variant CJD (vCJD) or sporadic CJD (sCJD). The formulations are also useful in the treatment of other neurologic diseases involving abnormal protein deposits or aggregates. Such diseases include Alzheimer's disease, which involves deposits of amyloid. The main component of amyloid plaques is amyloid-beta peptide (Abeta), a fibrillar 40-42 amino acid peptide that accumulates extracellularly and causes neuronal death. Further reference to prion diseases, subjects at risk thereof and diagnosis of subjects having prior disease can be found in published PCT Application WO 2004/007743, published January 22, 2004, the entire contents of which are recited herein in their entirety.

### Other therapies:

Subjects may be further administered other therapeutic agents or regimens. Examples include anti-microbial agents, anti-cancer agents, anti-allergy agents and anti-asthma agents. These other agents may be formulated together with or separately from the oligonucleotide/immune stimulating complex/antigen formulations of the invention.

An anti-microbial agent, as used herein, refers to a naturally-occurring or synthetic compound that is capable of killing or inhibiting infectious microorganisms. The type of anti-microbial agent useful according to the invention will depend upon the type of microorganism with which the subject is infected or at risk of becoming infected. Anti-microbial agents include but are not limited to anti-bacterial agents, anti-viral agents, anti-fungal agents, anti-parasitic agents, and anti-mycobacterial agents. Phrases such as. "anti-infective agent," "anti-bacterial agent," "anti-viral agent," "anti-fungal agent," "anti-parasitic agent," "parasiticide" and anti-mycobacterial agent" have established meanings to those of ordinary skill in the art and are defined in standard medical texts.

Anti-bacterial agents kill or inhibit bacteria, and include antibiotics as well as other synthetic or natural compounds having similar functions. Antibiotics are low molecular weight molecules which are produced as secondary metabolites by cells, such as microorganisms. In general, antibiotics interfere with one or more bacterial functions or structures which are specific for the microorganism and which are not present in host cells. Anti-viral agents can be isolated from natural sources or synthesized and are useful for killing or inhibiting viruses. Anti-fungal agents are used to treat superficial fungal infections as well as opportunistic and primary systemic fungal infections. Anti-parasite agents kill or inhibit parasites. Anti-mycobacterial agents kill or inhibit mycobacteria.

Anti-bacterial agents kill or inhibit the growth or function of bacteria. A large class of antibacterial agents is antibiotics. Antibiotics, which are effective for killing or inhibiting a wide range of bacteria, are referred to as broad spectrum antibiotics. Other types of antibiotics are predominantly effective against the bacteria of the class gram-positive or gram-negative. These types of antibiotics are referred to as narrow spectrum antibiotics. Other antibiotics which are effective against a single organism or disease and not against other types of bacteria, are referred to as limited spectrum antibiotics. Antibacterial agents are sometimes classified based on their primary mode of action. In general, antibacterial agents are cell wall synthesis inhibitors, cell membrane inhibitors, protein synthesis inhibitors, nucleic acid synthesis or functional inhibitors, and competitive inhibitors.

Anti-viral agents are compounds that prevent infection of cells by viruses or replication of the virus within the cell. There are many fewer antiviral drugs than antibacterial drugs because the process of viral replication is so closely related to DNA replication within the host cell, that non-specific antiviral agents would often be toxic to the host. There are several stages within the process of viral infection which can be blocked or inhibited by antiviral agents. These stages include, attachment of the virus to the host cell (immunoglobulin or binding peptides), uncoating of the virus (e.g. amantadine), synthesis or translation of viral mRNA (e.g. interferon), replication of viral RNA or DNA (e.g. nucleotide analogues), maturation of new virus proteins (e.g. protease inhibitors), and budding and release of the virus.

Anti-virals that are nucleotide analogues include, but are not limited to, acyclovir (used for the treatment of herpes simplex virus and varicella-zoster virus), gancyclovir (useful for the treatment of cytomegalovirus), idoxuridine, ribavirin (useful for the treatment of respiratory syncitial virus), dideoxyinosine, dideoxycytidine, zidovudine (azidothymidine), imiquimod, and resimiquimod.

Anti-viral agents useful in the invention include but are not limited to immunoglobulins, amantadine, interferons, nucleotide analogues, and protease inhibitors. Specific examples of anti-virals include but are not limited to Acemannan; Acyclovir; Acyclovir Sodium; Adefovir; Alovudine; Alvircept Sudotox; Amantadine Hydrochloride; Aranotin; Arildone; Atevirdine Mesylate; Avridine; Cidofovir; Cipamfylline; Cytarabine Hydrochloride; Delavirdine Mesylate; Desciclovir; Didanosine; Disoxaril; Edoxudine; Enviradene; Enviroxime; Famciclovir; Famotine Hydrochloride; Fiacitabine; Fialuridine; Fosarilate; Foscarnet Sodium; Fosfonet Sodium; Ganciclovir; Ganciclovir Sodium; Idoxuridine; Kethoxal; Lamivudine; Lobucavir; Memotine Hydrochloride; Methisazone; Nevirapine; Penciclovir; Pirodavir; Ribavirin; Rimantadine Hydrochloride; Saquinavir Mesylate; Somantadine Hydrochloride; Sorivudine; Statolon; Stavudine; Tilorone Hydrochloride; Trifluridine; Valacyclovir Hydrochloride; Vidarabine; Vidarabine Phosphate; Vidarabine Sodium Phosphate; Viroxime; Zalcitabine; Zidovudine; and Zinviroxime.

Anti-fungal agents are useful for the treatment and prevention of infective fungi. Anti-fungal agents are sometimes classified by their mechanism of action. Some anti-fungal agents function as cell wall inhibitors by inhibiting glucose synthase. These include, but are not limited to, basiungin/ECB. Other anti-fungal agents function by destabilizing membrane integrity. These include, but are not limited to, immidazoles, such as clotrimazole, sertaconzole, fluconazole, itraconazole, ketoconazole, miconazole, and voriconacole, as well as FK 463, amphotericin B, BAY 38-9502, MK 991, pradimicin, UK 292, butenafine, and terbinafine. Other anti-fungal agents function by breaking down chitin (e.g. chitinase) or immunosuppression (501 cream).

Anti-parasitic agents, also referred to as parasiticides, useful for human administration include but are not limited to albendazole, amphotericin B, benznidazole, bithionol, chloroquine HCl, chloroquine phosphate, clindamycin, dehydroemetine, diethylcarbamazine, diloxanide furoate, eflornithine, furazolidaone, glucocorticoids, halofantrine, iodoquinol, ivermectin, mebendazole, mefloquine, meglumine antimoniate, melarsoprol, metrifonate, metronidazole, niclosamide, nifurtimox, oxamniquine, paromomycin, pentamidine isethionate, piperazine, praziquantel, primaquine phosphate, proguanil, pyrantel pamoate, pyrimethanmine-sulfonamides, pyrimethanmine-sulfadoxine,quinacrine HCl, quinine sulfate, quinidine gluconate, spiramycin, stibogluconate sodium (sodium antimony gluconate), suramin, tetracycline, doxycycline, thiabendazole, tinidazole, trimethroprim-sulfamethoxazole, and tryparsamide some of which are used alone or in combination with others.

The inventive medicaments may also be administered in conjunction with an anti-cancer agent. An anti-cancer agent is an agent that is administered to a subject for the purpose of treating a cancer, and preferably is cytotoxic, particularly to proliferating cells. For the purpose of this specification, anti-cancer agents are classified as chemotherapeutic agents, immunotherapeutic agents, hormone therapy, and biological response modifiers.

The chemotherapeutic agent may be selected from the group consisting of methotrexate, vincristine, adriamycin, cisplatin, non-sugar containing chloroethylnitrosoureas, 5-fluorouracil, mitomycin C, bleomycin, doxorubicin, dacarbazine, taxol, fragyline, Meglamine GLA, valrubicin, carmustaine and poliferposan, MM1270, BAY 12-9566, RAS famesyl transferase inhibitor, farnesyl transferase inhibitor, MMP, MTA/LY231514, LY264618/Lometexol, Glamolec, CI-994, TNP-470, Hycamtin/Topotecan, PKC412, Valspodar/PSC833, Novantrone/Mitroxantrone, Metaret/Suramin, Batimastat, E7070, BCH-4556, CS-682, 9-AC, AG3340, AG3433, Incel/VX-710, VX-853, ZD0101, ISI641, ODN 698, TA 2516/Marmistat, BB2516/Marmistat, CDP 845, D2163, PD183805, DX8951f, Lemonal DP 2202, FK 317, Picibanil/OK-432, AD 32/Valrubicin, Metastron/strontium derivative, Temodal/Temozolomide, Evacet/liposomal doxorubicin, Yewtaxan/Paclitaxel, Taxol/Paclitaxel, Xeload/Capecitabine, Furtulon/Doxifluridine, Cyclopax/oral paclitaxel, Oral Taxoid, SPU-077/Cisplatin, HMR 1275/Flavopiridol, CP-358 (774)/EGFR, CP-609 (754)/RAS oncogene inhibitor, BMS-182751/oral platinum, UFT(Tegafur/Uracil), Ergamisol/Levamisole, Eniluracil/776C85/5FU enhancer, Campto/Levamisole, Camptosar/Irinotecan, Tumodex/Ralitrexed, Leustatin/Cladribine, Paxex/Paclitaxel, Doxil/liposomal doxorubicin, Caelyx/liposomal doxorubicin, Fludara/Fludarabine, Pharmarubicin/Epirubicin, DepoCyt, ZD1839, LU 79553/Bis-Naphtalimide, LU 103793/Dolastain, Caetyx/liposomal doxorubicin, Gemzar/Gemcitabine, ZD 0.473/Anormed, YM 116, Iodine seeds, CDK4 and CDK2 inhibitors, PARP inhibitors, D4809/Dexifosamide, lfes/Mesnex/lfosamide, Vumon/Teniposide, Paraplatin/Carboplatin, Plantinol/cisplatin, Vepeside/Etoposide, ZD 9331, Taxotere/Docetaxel, prodrug of guanine arabinoside, Taxane Analog, nitrosoureas, alkylating agents such as melphelan and cyclophosphamide, Aminoglutethimide, Asparaginase, Busulfan, Carboplatin, Chlorombucil, Cytarabine HCI Dactinomycin, Daunorubicin HCl, Estramustine phosphate sodium, Etoposide (VP16-213), Floxuridine, Fluorouracil (5-FU), Flutamide, Hydroxyurea (hydroxycarbamide), Ifosfamide, Interferon Alfa-2a, Alfa-2b, Leuprolide acetate (LHRH-releasing factor analogue), Lomustine (CCNU), Mechlorethamine HCl (nitrogen mustard), Mercaptopurine, Mesna, Mitotane (o.p'-DDD), Mitoxantrone HC1, Octreotide, Plicamycin, Procarbazine HCl, Streptozocin, Tamoxifen citrate, Thioguanine, Thiotepa, Vinblastine sulfate, Amsacrine (m-AMSA), Azacitidine, Erthropoietin, Hexamethylmelamine (HMM), Interleukin 2, Mitoguazone (methyl-GAG; methyl glyoxal bis-guanylhydrazone; MGBG), Pentostatin (2'deoxycoformycin), Semustine (methyl-CCNU), Teniposide (VM-26) and Vindesine sulfate, but it is not so limited.

The formulations may also be used with antibody therapy. Antibodies directed to cancer antigens include but are not limited to Ributaxin, Herceptin, Quadramet, Panorex, IDEC-Y2B8, BEC2, C225, Oncolym, SMART M195, ATRAGEN, Ovarex, Bexxar, LDP-03, ior t6, MDX-210, MDX-11, MDX-22, OV103, 3622W94, anti-VEGF, Zenapax, MDX-220, MDX-447, MELIMMUNB-2, MELIMMUNE-1, CEACIDE, Pretarget, NovoMAb-G2, TNT, Gliomab-H, GNI-250, EMD-72000, LymphoCide, CMA 676, Monopharm-C, 4B5, ior egf.r3, ior c5, BABS, anti-FLK-2, MDX-260, ANA Ab, SMART ID10 Ab, SMART ABL 364 Ab and ImmuRAIT-CEA.

Anti-asthma/allergy agents may be selected from the group consisting of PDE-4 inhibitor, bronchodilator/beta-2 agonist, K+ channel opener, VLA-4 antagonist, neurokin antagonist, TXA2 synthesis inhibitor, xanthanine, arachidonic acid antagonist, 5-lipoxygenase inhibitor, thromboxin A2 receptor antagonist, thromboxane A2 antagonist, inhibitor of 5-lipox activation protein, and protease inhibitor, but is not so limited. In some important embodiments, the asthma/allergy medicament is a bronchodilator/beta-2 agonist selected from the group consisting of salmeterol, salbutamol, terbutaline, D2522/formoterol, fenoterol, and orciprenaline.

The anti-asthma/allergy agent may also be anti-histamines and prostaglandin inducers. In one embodiment, the anti-histamine is selected from the group consisting of loratidine, cetirizine, buclizine, ceterizine analogues, fexofenadine, terfenadine, desloratadine, norastemizole, epinastine, ebastine, ebastine, astemizole, levocabastine, azelastine, tranilast, terfenadine, mizolastine, betatastine, CS 560, and HSR 609. In another embodiment, the prostaglandin inducer is S-5751.

The anti-asthma/allergy agents may also be steroids and immunomodulators. The immunomodulators may be selected from the group consisting of antiinflammatory agents, leukotriene antagonists, IL-4 muteins, soluble IL-4 receptors, immunosuppressants, anti-IL-4 antibodies, IL-4 antagonists, anti-IL-5 antibodies, soluble IL-13 receptor-Fc fusion proteins, anti-IL-9 antibodies, CCR3 antagonists, CCR5 antagonists, VLA-4 inhibitors, and downregulators of IgE, but are not so limited. In one embodiment, the downregulator of IgE is an anti-IgE. The steroid may be beclomethasone, fluticasone, tramcinolone, budesonide, and budesonide.

### With cytokines:

Subjects of the invention may also be co-administered cytokines (Bueler & Mulligan, 1996; Chow *et al.,* 1997; Geissler *et al.,* 1997; *Iwasaki et al.,* 1997; *Kim et al.,* 1997) or B-7 co-stimulatory molecules (Iwasaki *et al.,* 1997; Tsuji *et al.,* 1997), either together with or separate from the oligonucleotide/immune stimulating complex/antigen medicaments. The term cytokine is used as a generic name for a diverse group of soluble proteins and peptides which act as humoral regulators at nano- to picomolar concentrations and which, either under normal or pathological conditions, modulate the functional activities of individual cells and tissues. These proteins also mediate interactions between cells directly and regulate processes taking place in the extracellular environment. Examples of cytokines include, but are not limited to IL-1, IL-2, IL-4, IL-5, EL-6, IL-7, IL-10, IL-12, IL-15, IL-18, granulocyte-macrophage colony stimulating factor (GM-CSF), granulocyte colony stimulating factor (G-CSF), IFN-α, tumor necrosis factor (TNF), TGF-β, FLT-3 ligand, and CD40 ligand.

### With Other Adjuvants:

Medicaments of the invention may be used in combination with additional non-nucleic acid adjuvants. Non-nucleic acid adjuvants include, for instance, adjuvants that create a depo effect, immune stimulating adjuvants, and adjuvants that create a depo effect and stimulate the immune system.

An "adjuvant that creates a depo effect" is an adjuvant that causes the antigen to be slowly released in the body, thus prolonging the exposure of immune cells to the antigen. This class of adjuvants includes alum (e.g., aluminum hydroxide, aluminum phosphate); or emulsion-based formulations including mineral oil, non-mineral oil, water-in-oil or oil-in-water-in oil emulsion, oil-in-water emulsions such as Seppic ISA series of Montanide adjuvants (e.g., Montanide ISA 720, AirLiquide, Paris, France); MF-59 (a squalene-in-water emulsion stabilized with Span 85 and Tween 80; Chiron Corporation, Emeryville, CA; and PROVAX (an oil-in-water emulsion containing a stabilizing detergent and a micelle-forming agent; IDEC, Pharmaceuticals Corporation, San Diego, CA).

An "immune stimulating adjuvant" is an adjuvant that causes activation of a cell of the immune system. It may, for instance, cause an immune cell to produce and secrete cytokines. This class of adjuvants includes saponins purified from the bark of the Q. saponaria tree, such as QS21 (a glycolipid that elutes in the 2 1 st peak with HPLC fractionation; Aquila Biopharmaceuticals, Inc., Worcester, MA); poly[di(carboxylatophenoxy)phosphazene (PCPP polymer; Virus Research Institute, USA); derivatives of lipopolysaccharides such as monophosphoryl lipid A (MPL; Ribi ImmunoChem Research, Inc., Hamilton, MT), muramyl dipeptide (MDP; Ribi) andthreonyl-muramyl dipeptide (t-MDP; Ribi); OM-174 (a glucosamine disaccharide related to lipid A; OM Pharma SA, Meyrin, Switzerland); and Leishmania elongation factor (a purified Leishmania protein; Corixa Corporation, Seattle, WA).

"Adjuvants that create a depo effect and stimulate the immune system" are compounds that have both of the above- identified functions. This class of adjuvants includes but is not limited to SB-AS2 (SmithKline Beecham adjuvant system #2 which is an oil-in-water emulsion containing MPL and QS21: SmithKline Beecham Biologicals [SBB], Rixensart, Belgium); SB-AS4 (SmithKline Beecham adjuvant system #4 which contains alum and MPL; SBB, Belgium); non-ionic block copolymers that form micelles such as CRL 1005 (these contain a linear chain of hydrophobic polyoxpropylene flanked by chains ofpolyoxyethylene; Vaxcel, Inc., Norcross, GA); and Syntex Adjuvant Formulation (SAF, an oil-in-water emulsion containing Tween 80 and a nonionic block copolymer; Syntex Chemicals, Inc., Boulder, CO).

The oligonucleotide/immune stimulating complex/antigen medicaments may be administered simultaneously or sequentially with the other therapeutic agents and/or regimens. When the other therapeutic agents are administered substantially simultaneously with the formulations of the invention, they can be administered in the same or separate formulations, provided they are administered at substantially the same time (i.e., generally within minutes of each other, or within the time it takes a person of ordinary skill in the medical or pharmaceutical arts to administer the two substances). When other therapeutic agents are administered sequentially with the formulations of the invention, then the administration of the other therapeutic agents and the formulations is temporally separated. The separation in time between the administration of these compounds may be a matter of minutes, hours, days or longer.

### Formulations, Delivery Vehicles, Effective Amounts etc.:

The effective amount of a medicament refers to the amount necessary or sufficient to realize a desired biologic effect. For example, an effective amount of an oligonucleotide formulation administered with an antigen and an immune stimulating complex for inducing an antigen-specific immune response is that amount necessary to stimulate production of IFN-gamma or antigen-specific Th-1-induced immunoglobulin or activation of antigen-specific CTLs.

Combined with the teachings provided herein, by choosing among the various active compounds and weighing factors such as potency, relative bioavailability, patient body weight, severity of adverse side-effects and preferred mode of administration, an effective prophylactic or therapeutic treatment regimen can be planned which does not cause substantial toxicity and yet is entirely effective to treat the particular subject. The effective amount for any particular application can vary depending on such factors as the disease or condition being treated, the particular immunostimulatory oligonucleotide being administered, the dose of immune stimulating complex the size of the subject, or the severity of the disease or condition. One of ordinary skill in the art can empirically determine the effective amount of a particular oligonucleotide formulation and/or antigen formulation and/or other therapeutic agent without necessitating undue experimentation.

Subject doses of the compounds described herein for mucosal, local or parental delivery typically range from about 0.1 µg to 10 mg per administration, which depending on the application could be given for example daily, weekly, or any other amount of time therebetween. More typically mucosal, local or parental doses range from about 1 µg to 10 mg per administration, even more typically from about 10 µg to 5 mg per administration, still more typically from about 10 µg to 1 mg, and most typically from about 100 µg to 1 mg, with 2 - 4 administrations being spaced days or weeks apart.

For any compound described herein the therapeutically effective amount can be initially determined from animal models. A therapeutically effective dose can also be determined from human data for immunostimulatory oligonucleotides, antigens and complexes that have been tested individually in humans (human clinical trials have been initiated). The applied dose can be adjusted based on the relative bioavailability and potency of the administered compound. Adjusting the dose to achieve maximal efficacy based on the methods described above and other methods are known in the art and within the capabilities of the ordinarily skilled artisan.

Medicaments of the invention may be administered neat or in pharmaceutically acceptable solutions, which may in turn contain pharmaceutically acceptable concentrations of salt, buffering agents, preservatives, detergents, denaturants, compatible carriers, and optionally other therapeutic ingredients.

Oligonucleotides, immune stimulating complexes and antigens can be administered to a patient by any mode of administration either combined, separate or in any combination. Preferred routes of administration include but are not limited to parenteral administrations such as intramuscular and subcutaneous; and mucosal administrations such as oral, sublingual, intratracheal, intranasal, inhalation, intrapulmonary, vaginal and rectal.

For oral administration, the medicaments can be formulated readily by combining the active component(s) with pharmaceutically acceptable carriers well known in the art. Such carriers enable the components of the invention to be formulated as tablets, pills, dragees, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers, i.e. EDTA for neutralizing internal acid conditions or may be administered without any carriers.

Also specifically contemplated are oral dosage forms of the above components. The components maybe chemically modified so that oral delivery of the derivative is efficacious. Generally, the chemical modification contemplated is the attachment of at least one moiety to the component molecule itself, where said moiety permits (a) inhibition of proteolysis; and/or (b) uptake into the blood stream from the stomach or intestine. Also desired is the increase in overall stability of the components and increase in circulation time in the body. Examples of such moieties include polyethylene glycol, copolymers of ethylene glycol and propylene glycol, carboxymethyl cellulose, dextran, polyvinyl alcohol, polyvinyl pyrrolidone and polyproline. Abuchowski and Davis, 1981, "Soluble Polymer-Enzyme Adducts" In: Enzymes αs Drugs, Hocenberg and Roberts, eds., Wiley-Interscience, New York, NY, pp. 367-383; Newmark, et al., 1982, J. Appl. Biochem. 4:185-189. Other polymers that could be used are poly-1,3-dioxolane and poly-1,3,6-tioxocane. Preferred for pharmaceutical usage, as indicated above, are polyethylene glycol moieties.

The location of release may be the stomach, the small intestine (the duodenum, the jejunum, or the ileum), or the large intestine. One skilled in the art has available formulations which will not dissolve in the stomach, yet will release the material in the duodenum or elsewhere in the intestine. Preferably, the release will avoid the deleterious effects of the stomach environment, either by protection of the oligonucleotide or by release of the biologically active material beyond the stomach environment, such as in the intestine.

To ensure full gastric resistance a coating impermeable to at least pH 5.0 is essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and Shellac. These coatings may be used as mixed films.

A coating or mixture of coatings can also be used on tablets, which are not intended for protection against the stomach. This can include sugar coatings, or coatings which make the tablet easier to swallow. Capsules may consist of a hard shell (such as gelatin) for delivery of dry component i.e. powder; for liquid forms, a soft gelatin shell may be used. The shell material of cachets could be thick starch or other edible paper. For pills, lozenges, molded tablets or tablet triturates, moist massing techniques can be used.

The component can be included in the formulation as fine multi-particulates in the form of granules or pellets of particle size about 1 mm. The formulation of the material for capsule administration could also be as a powder, lightly compressed plugs or even as tablets. The component could be prepared by compression.

Colorants and flavoring agents may all be included. For example, the oligonucleotide and complex components may be contained within an edible product, such as a refrigerated beverage containing colorants and flavoring agents.

One may dilute or increase the volume of the components with an inert material. These diluents could include carbohydrates, especially mannitol, a-lactose, anhydrous lactose, cellulose, sucrose, modified dextrans and starch. Certain inorganic salts may be also be used as fillers including calcium triphosphate, magnesium carbonate and sodium chloride. Some commercially available diluents are Fast-Flo, Emdex, STA-Rx 1500, Emcompress and Avicell.

Disintegrants may be included in the formulation of the components into a solid dosage form. Materials used as disintegrates include but are not limited to starch, including the commercial disintegrant based on starch, Explotab. Sodium starch glycolate, Amberlite, sodium carboxymethylcellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants.

Binders may be used to hold the components together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic.

An anti-frictional agent may be included in the formulation to prevent sticking during the formulation process. Lubricants may be used as a layer between the therapeutic and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the formulation and to aid rearrangement during compression might be added. The glidants may include starch, talc, pyrogenic silica and hydrated silicoaluminate.

To aid dissolution of the components into the aqueous environment a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. The list of potential non-ionic detergents that could be included in the formulation as surfactants are lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation either alone or as a mixture in different ratios.

Pharmaceutical preparations that can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

For buccal administration, the formulations may take the form of tablets or lozenges formulated in conventional manner.

For administration by inhalation, the formulations may be conveniently delivered in the form of an aerosol spray presentation from pressurized packs or a nebulizer, with the use of a suitable propellant, *e.g.,* dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

Also contemplated herein is pulmonary delivery of the formulations. The formulation is delivered to the lungs of a mammal while inhaling and traverses across the lung epithelial lining to the blood stream. Other reports of inhaled molecules include Adjei et al., 1990, Pharmaceutical Research, 7:565-569; Adjei et al., 1990, International Journal of Pharmaceutics, 63:135-144 (leuprofide acetate); Braquet et al., 1989, Journal of Cardiovascular Pharmacology, 13(suppl. 5):143-146 (endothelin-1); Hubbard et al.,1989, Annals of Internal Medicine, Vol. m, pp. 206-212 (al-antitrypsin); Smith et al., 1989, J. Clin. Invest. 84:1145-1146 (a-1-proteinase); Oswein et al.,1990, "Aerosolization of Proteins", Proceedings of Symposium on Respiratory Drug Delivery II, Keystone, Colorado, March, (recombinant human growth hormone); Debs et al., 1988, J. Immunol. 140:3482-3488 (IFN-gamma and tumor necrosis factor alpha) and Platz et al., U.S. Patent No. 5,284,656 (granulocyte colony stimulating factor). A method and composition for pulmonary delivery of drugs for systemic effect is described in U.S. Patent No. 5,451,569, issued September 19,1995 to Wong et al.

Contemplated for use in the practice of this invention are a wide range of mechanical devices designed for pulmonary delivery of therapeutic products, including but not limited to nebulizers, metered dose inhalers, and powder inhalers, all of which are familiar to those skilled in the art.

Some specific examples of commercially available devices suitable for the practice of this invention are the Ultravent nebulizer, manufactured by Mallinckrodt, Inc., St. Louis, Missouri; the Acorn II nebulizer, manufactured by Marquest Medical Products, Englewood, Colorado; the Ventolin metered dose inhaler, manufactured by Glaxo Inc., Research Triangle Park, North Carolina; and the Spinhaler powder inhaler, manufactured by Fisons Corp., Bedford, Massachusetts.

Nasal delivery of a pharmaceutical composition of the present invention is also contemplated. Nasal delivery allows the passage of a pharmaceutical composition of the present invention to the blood stream directly after administering the therapeutic product to the nose, without the necessity for deposition of the product in the lung. Formulations for nasal delivery include those with dextran or cyclodextran.

For nasal administration, a useful device is a small, hard bottle to which a metered dose sprayer is attached. In one embodiment, the metered dose is delivered by drawing the pharmaceutical composition of the present invention solution into a chamber of defined volume, which chamber has an aperture dimensioned to aerosolize and aerosol formulation by forming a spray when a liquid in the chamber is compressed. The chamber is compressed to administer the pharmaceutical composition of the present invention. In a specific embodiment, the chamber is a piston arrangement. Such devices are commercially available.

Alternatively, a plastic squeeze bottle with an aperture or opening dimensioned to aerosolize an aerosol formulation by forming a spray when squeezed is used. The opening is usually found in the top of the bottle, and the top is generally tapered to partially fit in the nasal passages for efficient administration of the aerosol formulation. Preferably, the nasal inhaler will provide a metered amount of the aerosol formulation, for administration of a measured dose of the drug.

The medicaments, when it is desirable to deliver them systemically, may be formulated for parenteral administration by injection, *e.g.,* by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the medicaments may be in powder form for constitution with a suitable vehicle, *e.g.*, sterile pyrogen-free water, before use.

The medicaments may also be formulated in rectal or vaginal compositions such as suppositories or retention enemas, *e.g.,* containing conventional suppository bases such as cocoa butter or other glycerides.

The medicaments also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The medicaments also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The medicaments are suitable for use in a variety of drug delivery systems. For a brief review of methods for drug delivery, see Langer, Science 249:1527-1533, 1990, which is incorporated herein by reference.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

The medicaments optionally include a pharmaceutically-acceptable carrier. The term pharmaceutically-acceptable carrier means one or more compatible solid or liquid filler, diluents or encapsulating substances which are suitable for administration to a human or other vertebrate animal. The term carrier denotes an organic or inorganic ingredient, natural or synthetic, with which the active components are combined to facilitate the application.

The invention is further illustrated by the following Examples, which in no way are limiting. The entire contents of all of the references (including literature references, issued patents, published patent applications, and co-pending patent applications) cited throughout this application are hereby expressly incorporated by reference.

### Examples:

### Example 1: Induction of Antigen-Specific Immune Responses using Immune stimulating complexes and Oligonucleotides in a Vaccine Setting

### Introduction:

The induction of antigen-specific Th1 cell mediated immunity is highly desirable for certain conditions including (i) prophylactic vaccination against viral pathogens where sterilizing immunity is difficult to achieve due to the ability of the virus to rapidly mutate its surface proteins (*e.g.,* HIV, HCV) and (ii) therapeutic immunization against chronic viral or bacterial infections, or (iii) therapeutic immunization to treat cancer.

Th1-type immunity is associated with CD8+ cytotoxic T lymphocytes, which may act by lytic and non-lytic mechanisms. Lytic CTL secrete a chemical perforin upon meeting a cell that presents peptides from the foreign antigen (tumor or pathogen associated) on its surface by MHC Class I molecules. Perforin then forms holes in the cell membrane and kills the cell. Non-lytic CTL secrete Th1-type cytokines such as IL-12 and IFN-γ.

IFN-γ is the hallmark of Th1 type cellular responses since it is the primary cytokine secreted from CD4+ T cells to induce CD8+ CTL. As well, IFN-γ secreted by both CD4+ and CD8+ T cells is the main cytokine responsible for non-lytic control of chronic viral infections.

Th1 type CD8+ CTL are created when naïve CD8+ T cells detect antigen presented by professional antigen-presenting cells such as dendritic cells in the presence of Th1 cytokines that are secreted by stimulated CD4+ T cells that also recognize the same antigen. There are various ways to detect Th1-type CTL. A direct method is to measure their ability to lyse target cells that express the antigen and are also loaded with a radiolabeled substance, which is then detected as a way to quantify the degree of killing. This is a difficult and cumbersome assay thus it is well accepted to use indirect methods to detect Th1 T cell responses.

Indirect methods to detect CTL or Th1 cellular immunity all rely on measuring IFN-gamma responses. In one method, splenocytes or PBMC are restimulated in culture with the antigen and the amount of IFN-gamma secreted by the T-cells into the culture media is measured by ELISA assay. This is the method used in the present studies. In another method, immune cells recovered from the immunized animal (spleen cells or peripheral blood mononuclear cells = PBMC) or human (PBMC) can be sorted by FACS analysis into T cells that secrete IFN-gamma; CD4+ and CD8+ T cells can be sorted separately and counted. In a third method it is possible to estimate the number of IFN-γ secreting cells by a method known as ELISPOT.

### Materials and Methods:

Immunization of mice: All experiments were carried out using female BALB/c mice aged 6-8 weeks with 10 mice per experimental or control group. For all immunizations, mice were lightly anaesthetized with Isoflurane® (CDMV, St. Hyacinthe, QC).

Antigens: Recombinant HBsAg (ay subtype, Seradyne, Indianapolis, IN).

Oligonucleotides: All oligonucleotides (see Table 1) were obtained from Coley Pharmaceutical GmbH, Langenfeld, Germany.

Immune stimulating complexes: ISCOMATRIX® adjuvant, herein referred to as IMX, was the immune stimulating complex used in these examples. The IMX was prepared at laboratory scale using dialysis, essentially by the method of Morein et al, 1998. Briefly, to 800µl of phosphate buffered saline (PBS) pH6.2 was added 100µl of a solution containing 17mg/ml cholesterol and 10mg/ml dipalmitoylphosphatidylcholine (DPPC) in 20% w/v Mega-10 then 100µl of 32 mg/ml ISCOPREP® saponin (CSL Limited, Parkville, Victoria, Australia) in PBS pH6.2. The solution was held at 25°C for 1 hour with gentle mixing and then dialysed extensively against PBS pH6.2. During dialysis IMX containing ISCOPREP® saponin, cholesterol and DPPC was formed.

Intramuscular immunization: Each mouse received a single intramuscular (IM) injection on days 0 and 28 using a 1.0 ml insulin syringe (Becton Dickenson, Franklin Lakes, NJ) into the left tibialis anterior (TA)muscle of 1 µg HBsAg (ay subtype, Seradyne, Indianapolis, IN) +/- CpG or non-CpG ODN (Coley Pharmaceutical GmbH, Langenfeld, Germany) ±IMX +/- alum (Al₂O₃, Alhydrogel "85," Superfos Biosector, Vedbaek, Denmark; 2.5 µl 2% Al₂O₃ per µg HBsAg to give 25 mg Al³⁺/mg HBsAg), made up to a total volume of 50 µl with phosphate buffered saline (Sigma Chemical Co., St. Louis, MO).

Subcutaneous immunization: Each mouse received a single subcutaneous (SC) injection with a 1.0 m1 insulin syringe (Becton Dickenson, Franklin Lakes, NJ) into the lower back of 1 µg HBsAg (ay subtype, Seradyne, Indianapolis, IN) +/- CpG or non-CpG ODN, ±IMX +/- alum (Al₂O₃, Alhydrogel "85," Superfos Biosector, Vedbaek, Denmark; 2.5 µl 2% Al₂O₃ per µgHBsAg to give 25 mg Al³⁺/mg HBsAg)., made up to a total volume of 100 µl with phosphate buffered saline (Sigma Chemical Co., St. Louis, MO).

Collection of plasma: Plasma was recovered from mice at various times after immunization by retro-orbital bleeding and stored at -20° C until assayed.

### Evaluation of immune responses:

HBsAg-specific IgG (anti-HBs): Antigen-specific antibodies in the mouse plasma were detected and quantified by end-point dilution ELISA assay (in triplicate) for individual animals as described previously (Davis *et al.,* 1998). Briefly, 96-well polystyrene plates (Corning) coated overnight (RT) with HBsAg particles (100 µl of 1 µg/ml HBsAg in 0.05 M sodium carbonate-bicarbonate buffer, pH 9.6) were incubated with the plasma for 1 hr at 37 °C. Captured antibodies were then detected with horseradish peroxidase (HRP)-conjugated goat anti-mouse IgG, IgG1, or IgG2a (1:4000 in PBS-Tween, 10% FCS: 100 µl/well; Southern Biotechnology Inc., Birmingham, AL), followed by addition of o-phenylenediamine dihydrochloride solution (OPD, Sigma), 100 µl/well, for 30 min at RT in the dark. The reaction was stopped by the addition of 2M H₂SO₄, µl/well. Each bar represents the group geometric mean (± SEM) of the ELISA end-point dilution titer for the specified antibodies in plasma taken 4 weeks after final immunization. Titers were defined as the highest plasma dilution resulting in an absorbance value two times that of non-immune plasma with a cut-off value of 0.05.

Interferon-gamma (IFN-γ) secretion: TFN-γ secretion was measured following antigen re-stimulation of splenocytes from immunized animals. Spleen cell suspensions were prepared and adjusted to a final concentration of 5 X 10⁶ cells per ml in RPMI 1640 (Life Technologies, Grand Island, NY) tissue culture medium supplemented with 2% normal mouse serum (Cedarlane Laboratories, Ontario, Canada), penicillin-streptomycin solution (final concentration of 1000 U/ml and 1 mg/ml respectively; Sigma, Irvine, UK), and 5 × 10⁻⁵ M β-mercaptoethanol (Sigma) (Complete RPMI 1640). Splenocyte suspension was plated onto 96-well U-bottom tissue culture plates (100 µl/well) along with 100 µl of each stimulant diluted to appropriate concentrations in Complete RPMI 1640. The stimulant used was HBsAg at 5.0 and 2.5 µg/ml. Concanavalin A (10 µg/ml, Sigma) was used as a positive control and cells cultured with media alone were used as negative controls. Each splenocyte sample was plated in triplicate and the cells were incubated in a humidified 5% CO₂ incubator at 37°C for 48 and 72 hr. At the end of the incubation period, the 96-well plates were centrifuged for 5 min at 1200 rpm and culture supernatants harvested and stored at -80°C until assayed. Commercially available assay kits (mouse IFN-γ OptEIA; PharMingen, Mississauga, ON) were used according to manufacturers instructions to assay cytokine levels in culture supernatants taken at 72 hr.

Cytotoxic T lymphocyte activity (CTL activity): Spleens were recovered under sterile conditions from mice previously immunized with HBsAg +/-immune stimulating complex +/-oligonucleotide +/- alum. Single cell suspensions were prepared and suspended in RPMI 1640 (Life Technologies, Grand Island, NY) tissue culture medium supplemented with 10% FBS (Life Technologies) and penicillin-streptomycin solution (final concentrations of 1000 U/ml and 1 mg/ml respectively) (Sigma, Irvine, U.K.) as well as 5 x 10⁻⁵ M β-mercaptoethanol (Sigma) and 3% EL-4 supernatant as a source of IL-2. Splenocytes (3 × 10⁷) were cocultured with 1 × 10⁶ syngeneic HBsAg-expressing stimulator cells (P815-preS), which had been inactivated by irradiation (20,000 rad). The cultures were maintained for 5 days in 10 ml of media in upright 25 cm² tissue culture flasks in a humidified atmosphere (5% CO₂) at 37°C and then were harvested and washed in media. These effector cells were serially diluted and cultured with 5 × 10^{3 51}Cr-labeled HBsAg-expressing targets (P815S) or control target cells (P815) at 37°C in round-bottom 96-well microtiter plates, with each sample in triplicate. After 4 h of incubation, 100 µl of supernatant was removed for radiation (gamma) counting. The percent lysis was calculated as [(experimental release - spontaneous release)/(total release - spontaneous release)] × 100. Spontaneous release was determined by incubating target cells without effector cells, and total release was determined by adding 100 µl of 2% Triton X-100 to the target cells. The percent specific lysis was calculated as follows: % lysis with P815S cells-% lysis P815 cells.

### Results and Conclusions:

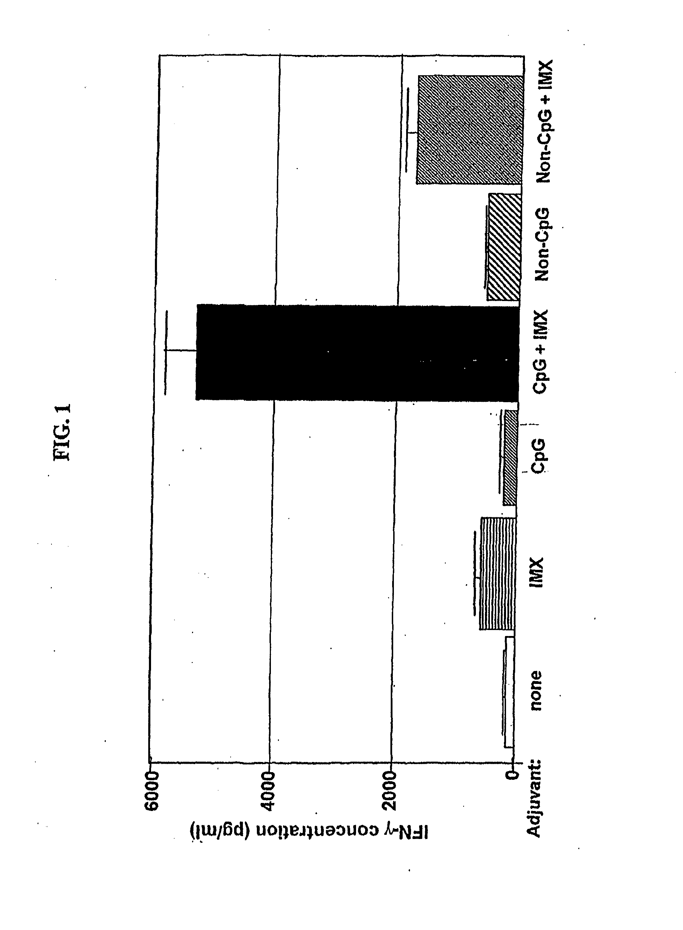

FIG. 1 is a bar graph depicting the effect of different adjuvants on interferon-gamma (IFN-g) levels measured in supernatants from splenocytes stimulated with HBsAg (2.5 or 5.0 mg/ml), wherein BALB/c mice were immunized by SC injection with HBsAg (1 µg) without or in combination with 10 mg CpG oligonucleotide sequence 7909 or non-CpG oligonucleotide sequence **2137** and/or 5µg IMX on days 1 and 28. Four weeks after boost, spleens were removed, and IFN-g measured in supernatants from splenocytes stimulated with HBsAg (2.5 or 5.0µg/ml). Bars show concentration of IFN- g (pg/ml) +/- SD in supernatants after stimulation with 5.0 mg/ml HBsAg. Equivalent results were obtained with 2.5 mg/ml stimulation (results not shown). Additional samples stimulated with media alone confirmed stimulation was antigen-specific (results not shown).

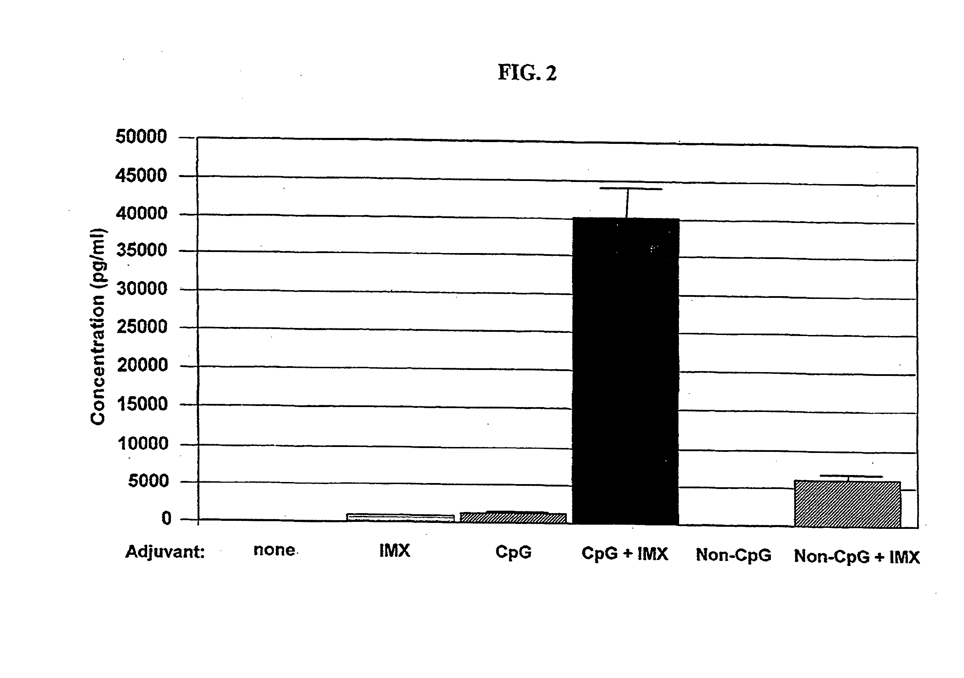

FIG. 2 is a bar graph depicting the effect of different adjuvants on interferon-gamma (IFN-g) levels measured in supernatants from splenocytes stimulated with HBsAg (2.5 or 5.0 mg/ml), wherein BALB/c mice were immunized by IM injection with HBsAg (1 µg) without or in combination with 10 mg CpG oligonucleotide sequence 7909 or non-CpG oligonucleotide sequence 2137 and/or 5µg IMX on days 1 and 28. Four weeks after boost, spleens were removed, and IFN-g measured in supernatants from splenocytes stimulated with HBsAg (2.5 or 5.0µg/ml). Bars show concentration of IFN-g (pg/ml) +/- SD in supernatants after stimulation with 5.0 mg/ml HBsAg. Equivalent results were obtained with 2.5 mg/ml stimulation (results not shown). Additional samples stimulated with media alone confirmed stimulation was antigen-specific (results not shown).

IFN-gamma secretion was observed from restimulated splenocytes after recovery from mice previously immunized via SC or IM injection with HBsAg± ODN± IMX (FIGs. 1 and 2). The response from the CpG oligonucleotide formulation (i.e., with the immune stimulating complex) and the inert oligonucleotide formulation (labeled as "non-CpG") was far greater than the additive effects of either oligonucleotide alone with IMX.

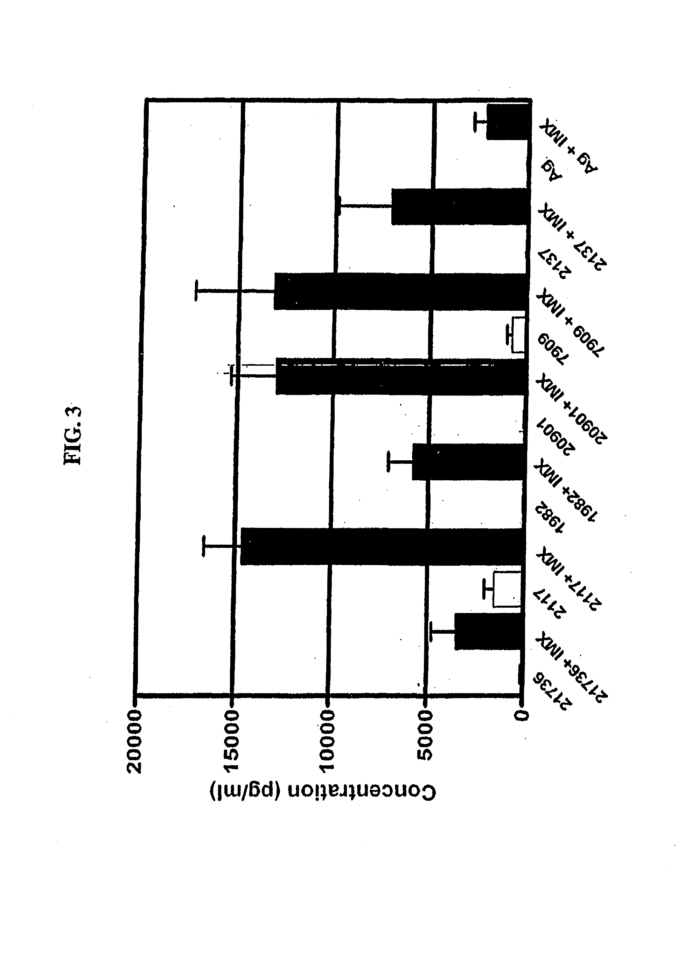

FIG. 3 is a bar graph depicting the effect of different oligonucleotides on interferon-gamma (IFN-g) levels measured in supernatants from splenocytes stimulated with HBsAg (2.5 or 5.0 mg/ml), wherein BALB/c mice were immunized by SC injection with HBsAg (1 µg) without or in combination with 10 mg CpG oligonucleotide (sequence **7909**) or non-CpG oligonucleotide (sequence **21736, 2117, 1982, 2091, or 2137**) and/or 5µg IMX on days 1 and 28. Four weeks after boost, spleens were removed, and IFN-g measured in supernatants from splenocytes stimulated with HBsAg (2.5 or 5.0µg/ml). Bars show concentration of IFN-g (pg/ml) +/- SD in supernatants after stimulation with 5.0 mg/ml HBsAg. Equivalent results were obtained with 2.5 mg/ml stimulation (results not shown). Additional samples stimulated with media alone confirmed stimulation was antigen-specific (results not shown).

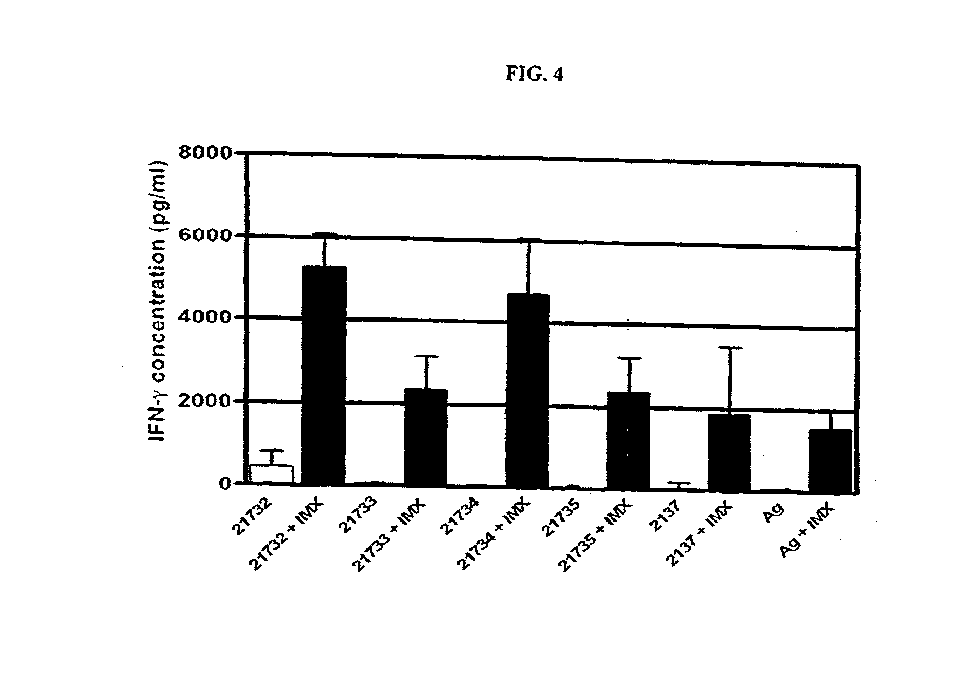

FIG. 4 is a bar graph depicting the effect of different oligonucleotides on interferon-gamma (TFN-g) levels measured in supernatants from splenocytes stimulated with HBsAg (2.5 or 5.0 mg/ml), wherein BALB/c mice were immunized by SC injection with HBsAg (1 µg) without or in combination with 10 mg non-CpG oligonucleotide (sequence **21732, 21733, 21734, 21735, or 2137**) and/or 5µg IMX on days 1 and 28. Four weeks after boost, spleens were removed, and IFN-g measured in supernatants from splenocytes stimulated with HBsAg (2.5 or 5.0µg/ml). Bars show concentration of IFN-g (pg/ml) +/- SD in supernatants after stimulation with 5.0 mg/ml HBsAg. Equivalent results were obtained with 2.5 mg/ml stimulation (results not shown). Additional samples stimulated with media alone confirmed stimulation was antigen-specific (results not shown).

FIGs. 3 and 4 clearly show that even inert oligonucleotides (*e.g.,* 1982 and 2137) can induce synergistic levels of IFN-gamma when formulated with immune stimulating complexes in the context of antigen administration. Even greater effects are observed for known immunostimulatory oligonucleotides (*e.g.,* 7909).

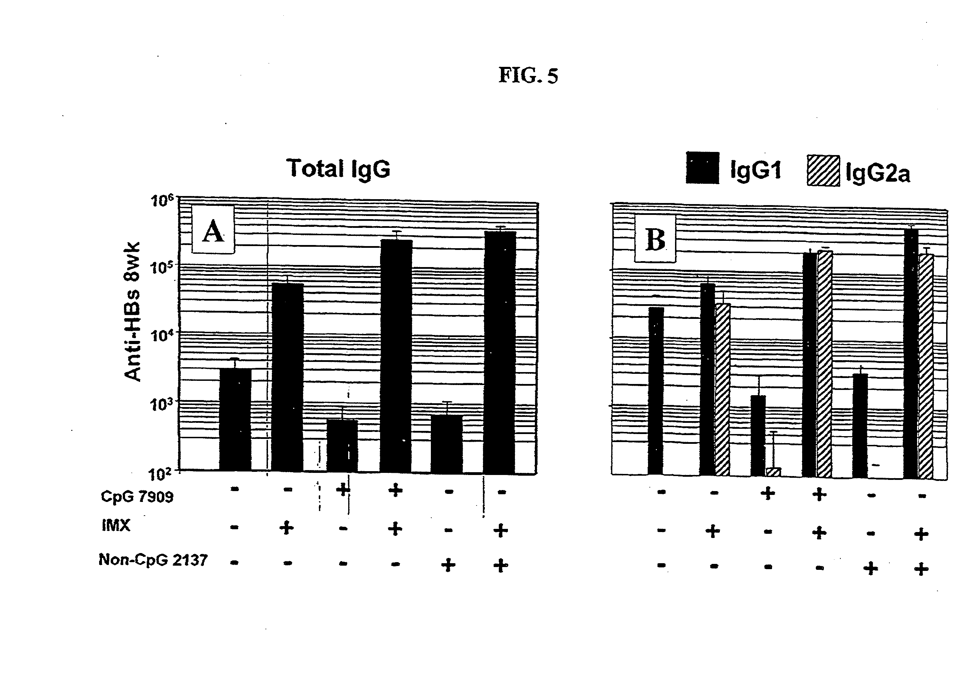

FIG. 5 is a graph depicting the effect of different adjuvants on total IgG titers ofanti-HBs, wherein BALB/c mice were immunized by SC injection with HBsAg (1 µg) without or in combination with 10 mg CpG oligonucleotide sequence 7909 or non-CpG oligonucleotide sequence 2137 and/or 5µg IMX on days 1 and 28. Four weeks after boost, animals were bled and plasma collected and anti-HBs levels determined by ELISA. Bars show anti-HBs group geometric mean titer +/- SEM for total IgG (panel A) or IgG2a and IgG1 (panel B).

As seen with IFN-gamma induction, the oligonucleotide/immune stimulating complex/antigen formulations whether comprising immunostimulatory oligonucleotides or inert oligonucleotides are capable of inducing Th1-biased antigen-specific immune responses, as evidenced by production of anti-HBs total IgG and the induction of IgG2a as shown in FIG. 5.

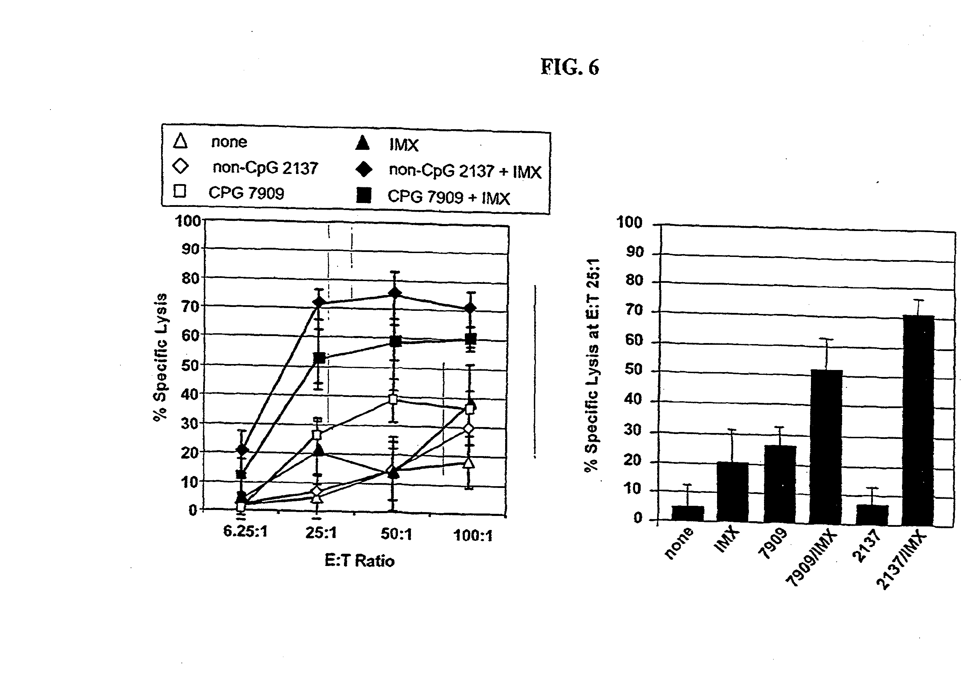

FIG. 6 is a graph depicting the effect of different adjuvants on HBsAg specific CTL response, wherein BALB/c mice were immunized by SC injection with HBsAg (1 µg) without or in combination with 10 mg CpG oligonucleotide sequence 7909 or non-CpG oligonucleotide sequence 2137 and/or 5µg IMX on days 1 and 28. Four weeks after immunization mice were killed by Halothane overdose, splenocytes isolated and HBsAg specific CTL activity determined by ⁵¹Cr release assay.

As seen with IFN-gamma and antigen-specific immunoglobulin production, oligonucleotide/immune stimulating complex/antigen formulations comprising either immunostimulatory oligonucleotides or inert oligonucleotides are capable of activating antigen-specific CTL, as shown in FIG. 6.

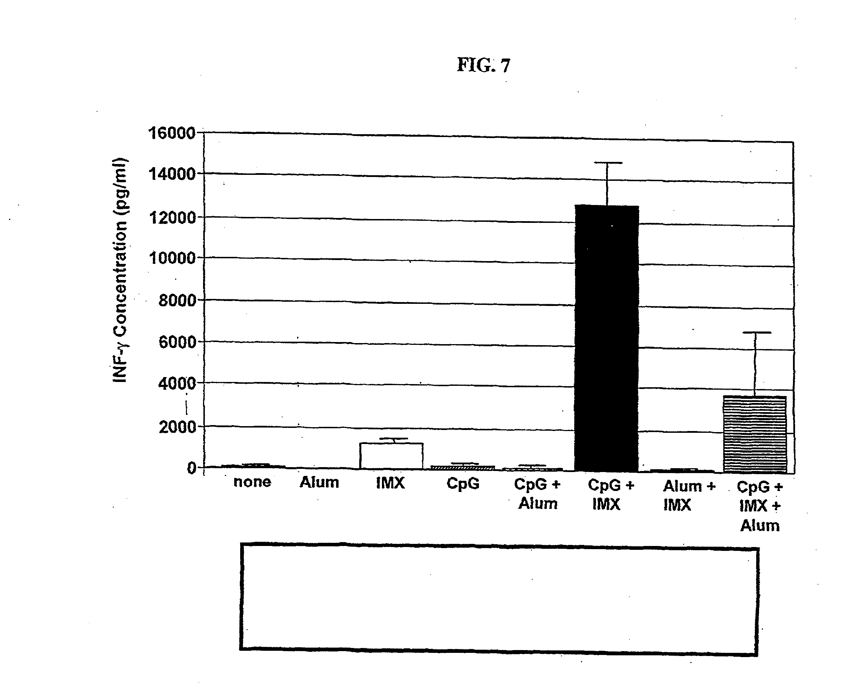

FIG. 7 is a bar graph depicting the effect of different adjuvants on interferon-gamma (IFN-g) levels measured in supernatants from splenocytes stimulated with HBsAg (2.5 or 5.0 mg/ml), wherein BALB/c mice were immunized by SC injection with HBsAg (1 µg) without or in combination with 10 mg CpG oligonucleotide sequence 7909 and/or 5µg IMX and/or alum on days 1 and 28. Four weeks after boost, spleens were removed, and IFN-g measured in supernatants from splenocytes stimulated with HBsAg (2.5 or 5.0µg/ml). Bars show concentration of IFN-g (pg/ml) +/- SD in supernatants after stimulation with 5.0 mg/ml HBsAg. Equivalent results were obtained with 2.5 mg/ml stimulation (results not shown). Additional samples stimulated with media alone confirmed stimulation was antigen specific (results not shown).

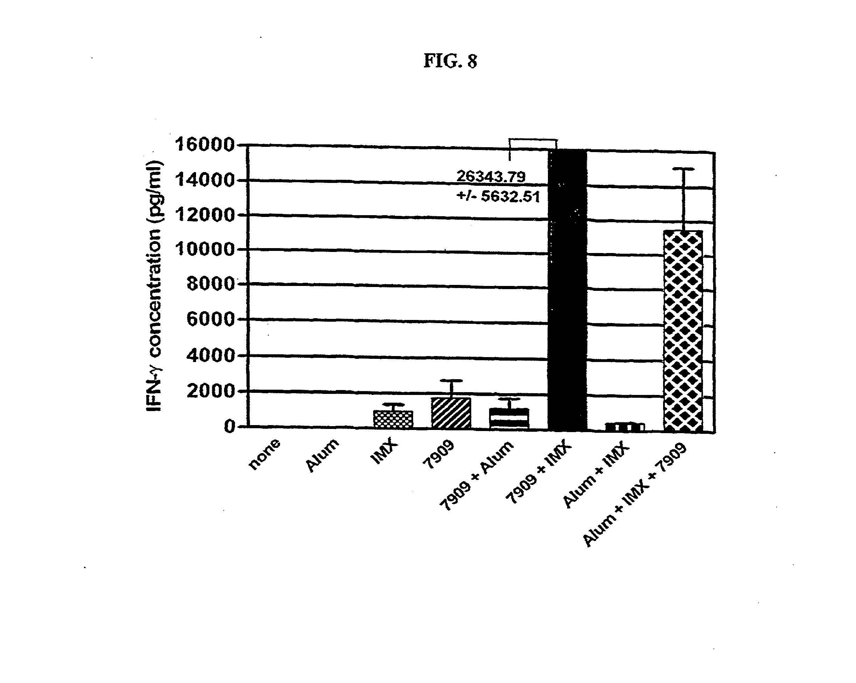

FIG. 8 is a bar graph depicting the effect of different adjuvants on interferon-gamma (IFN-g) levels measured in supernatants from splenocytes stimulated with HBsAg (2.5 or 5.0 mg/ml), wherein BALB/c mice were immunized by IM injection with HBsAg (1 µg) without or in combination with 10 mg CpG oligonucleotide sequence 7909 and/or 5µg IMX and/or alum on days 1 and 28. Four weeks after boost, spleens were removed, and IFN-g measured in supernatants from splenocytes stimulated with HBsAg (2.5 or 5.0µg/ml). Bars show concentration of IFN-g (pg/ml) +/- SD in supernatants after stimulation with 5.0 mg/ml HBsAg. Equivalent results were obtained with 2.5 mg/ml stimulation (results not shown). Additional samples stimulated with media alone confirmed stimulation was antigen specific (results not shown).

FIGs. 7 and 8 demonstrate that addition of alum to the oligonuleotide/immune stimulating complex/antigen formulation, reduces but does not eliminate the synergistic response observed with the oligonucleotide/immune stimulating complex/antigen formulations, regardless of whether administration is IM or SC.

### General Conclusion:

It is generally accepted that the virus-specific IFN-γ is a good marker for Th1 immune responses and therefore an indicator of cellular immunity. Th1 responses are widely accepted as being required in a variety of prophylactic and therapeutic vaccine settings, in particular viral infections such as HIV, HBV, HSV and CMV.

IFN-γ is a highly pleiotropic cytokine with a variety of functions. In the vaccine setting high IFN-γ responses would be beneficial in a number of ways including, but not limited to:

### Direct antiviral effect:

IFN-γ has been shown to inhibit both HBV transcription and replication in human hepatocytes (Suri et al 2001, J. Hepatol. 35:709-7)

IFN-γ production was found to correlate with the response to therapeutic immunization against chronic HBV infection (Ren et al 2003, J. Med. Virol. 71: 376-84

IFN-γ inhibits the transmission of HSV-1 from neural axon to epidermal cells by direct antiviral effects. This suggests that IFN-γ contributes to the control of HSV-1 spread and shedding in recurrent herpetic lesions. High IFN-γ expression would therefore be a valuable feature for therapeutic HSV vaccines.

### CD8 T-cell (CTL) responses:

Induction of CTL is generally dependent on the provision of CD4 T-cell help, a key component of which is Th1 Cytokines such as IFN-γ.

In the early stages of HIV infection cellular immunity (both CD4 and CD8) plays an important role in limited viral spread. However, as disease progresses and the CD4 cell count decreases, it eventually reaches a level that is not sufficient to maintain the CD8 response, which subsequently also wanes. As a consequence, viral replication proceeds "unchecked", resulting in full-blown AIDS.

As part of a therapeutic HIV vaccine the IMX/CpG/HIV antigen combination could elicit sufficient T help from the remaining CD4 T-cells to mount an effective HTV-specific CDS T-cell response. The aim of such a vaccine would be to control viraemia and restore immunocompetency.

### Cancer immunotherapy:

High levels of IFN-γ has been shown to inhibit angiogenesis and consequently suppress neoplastic growth Rankin et al Cancer Biol. Ther. 2:687-93).

As a result, it is expected that the substantially increased IFN-γ responses observed in the oligonucleotide/immune stimulating complex/antigen vaccine formulation of the present invention will confer important prophylactic and therapeutic benefits when administered to those in need.

### Example 2: Induction of Antigen-Specific Immune Responses using Immune Stimulating Complexes and Oligonucleotides in a Cancer Vaccine Setting

### Material and Methods:

Refer to Example 1 for Materials and Methods not specifically listed here.

### Cancer Mouse Models:

B16 is an experimental melanoma murine cancer model. The tumor expresses OVA antigen. Female C57B1/6 mice were vaccinated IM on days -21 and - 7. Vaccination groups were as follows: (i) OVA (50 µg) alone; (ii) OVA and CpG 7909 (25 µg); (iii) OVA and IMX (5 µg); (iv) OVA and CpG 7909 and IMX; (v) CpG 7909 (25 µg) alone; (vi) IMX (5 µg) alone; and (vii) CpG 7909 and IMX. On day 0, mice were inoculated with 5 x 10⁵ cells as the tumor challenge. On day 28, mice were sacrificed and immune assays were performed on harvested tissues.

In a second experiment, cervical carcinoma expressing HPV E6/E7 proteins was inoculated into a mouse. 1 x 10⁶ cervical cell carcinoma cells were injected SC on day 0. Treatment regimens were as follows: 25 µg CpG 7909, 5 µg IMX and/or 10 µg E6/E7 peptide SC on day 7 and weekly thereafter for 2 months.

### Results:

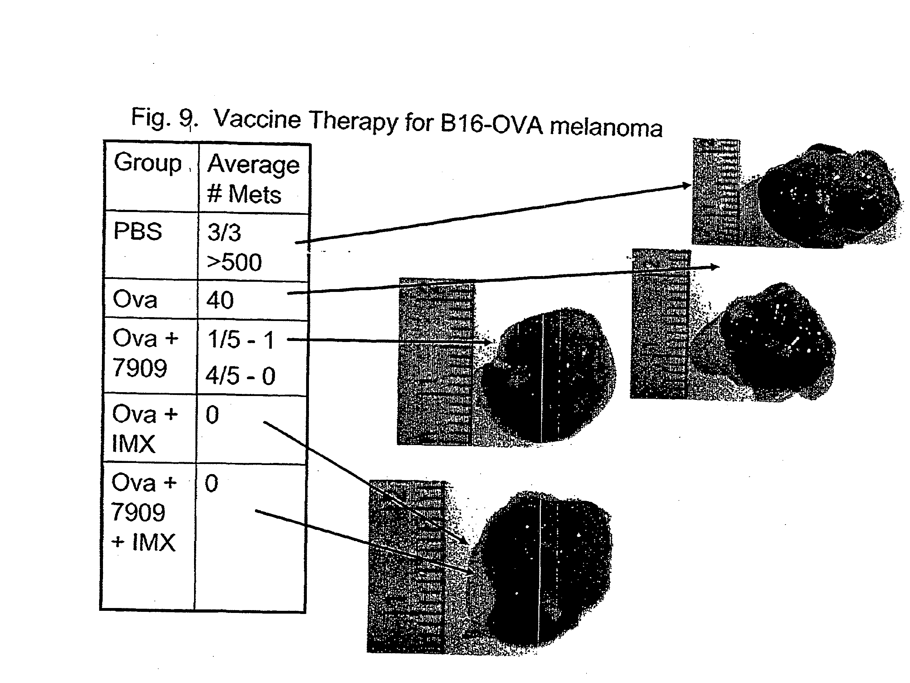

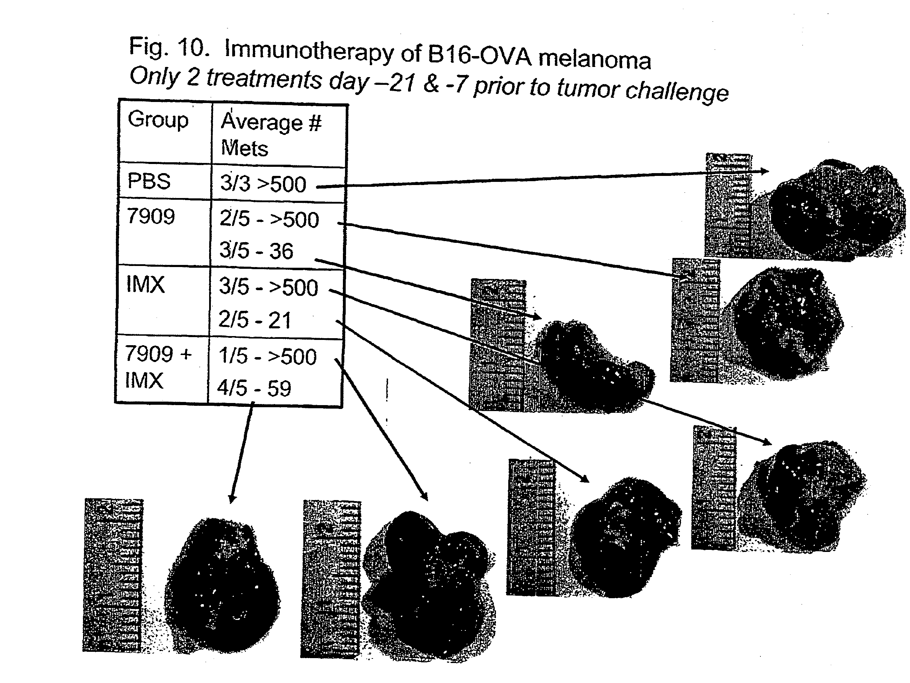

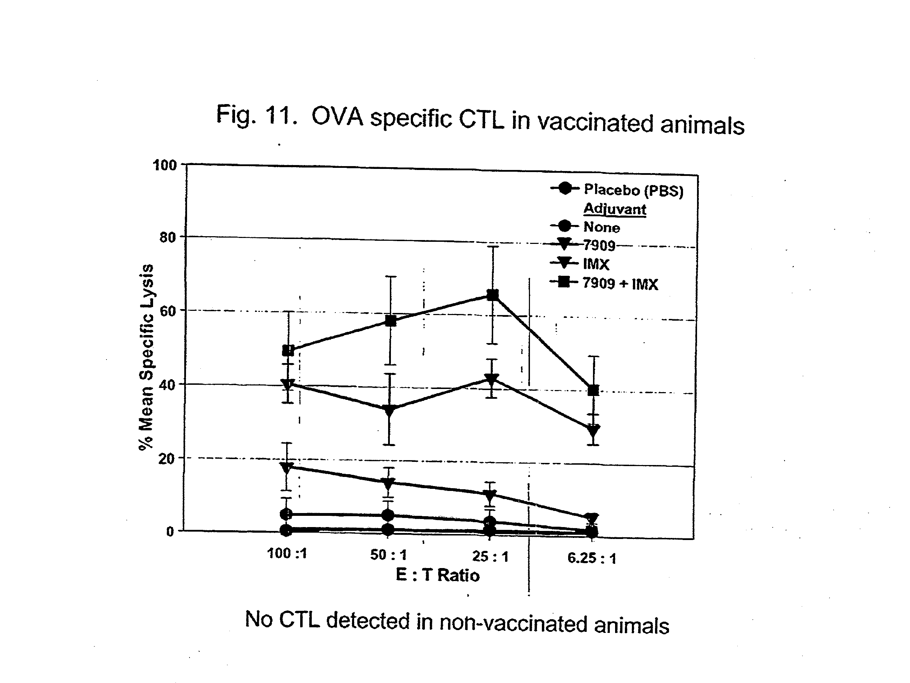

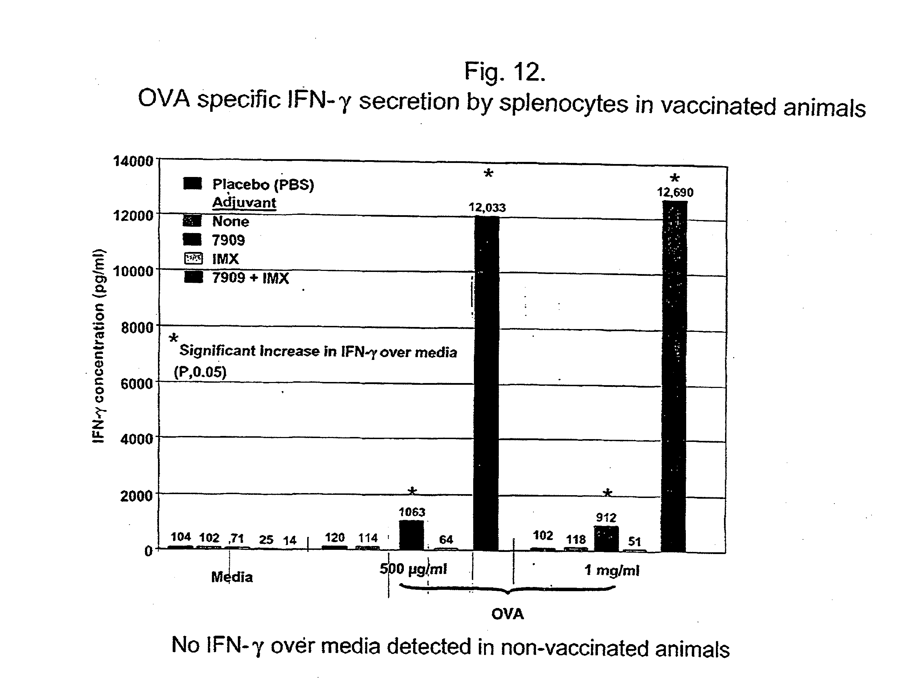

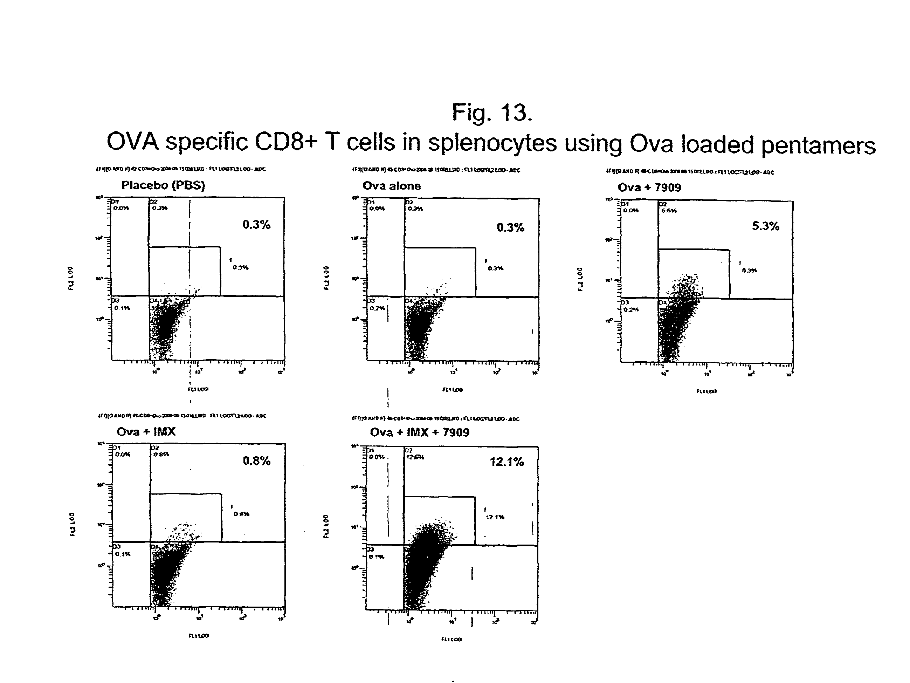

FIGS. 9-13 show the results of the B16 melanoma experiments. Synergy between CpG 7909 and IMX was demonstrated. This synergy was observed in OVA specific CMI in animals vaccinated with OVA and CpG 7909 and IMX compared to using either adjuvant alone in the melanoma model.

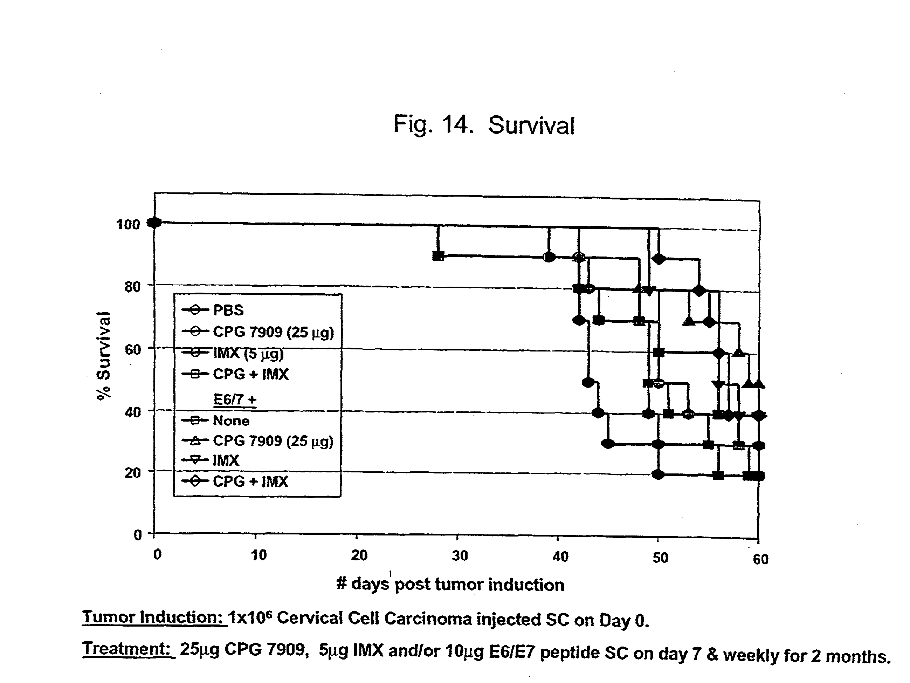

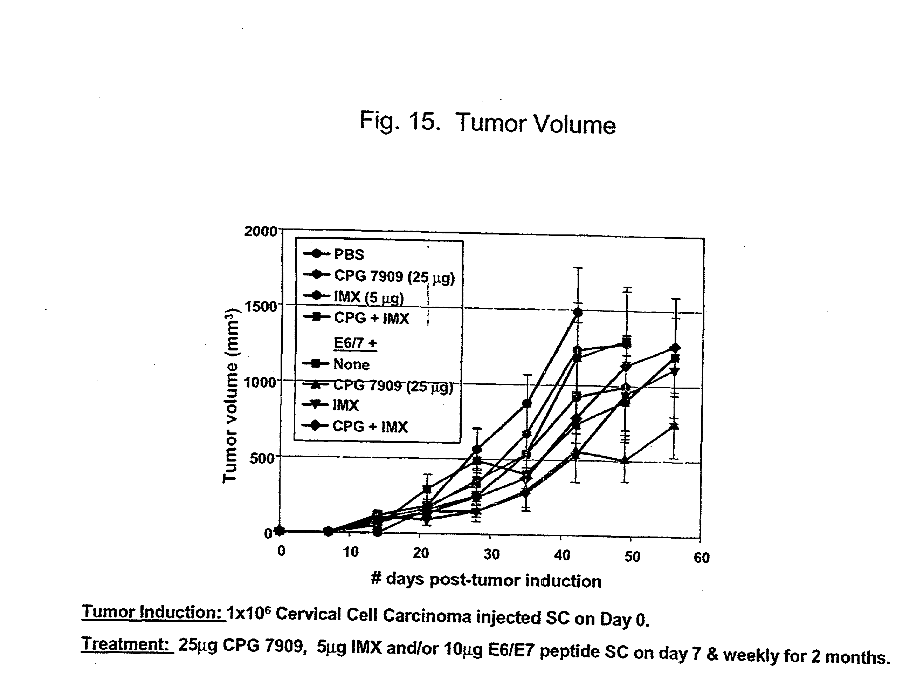

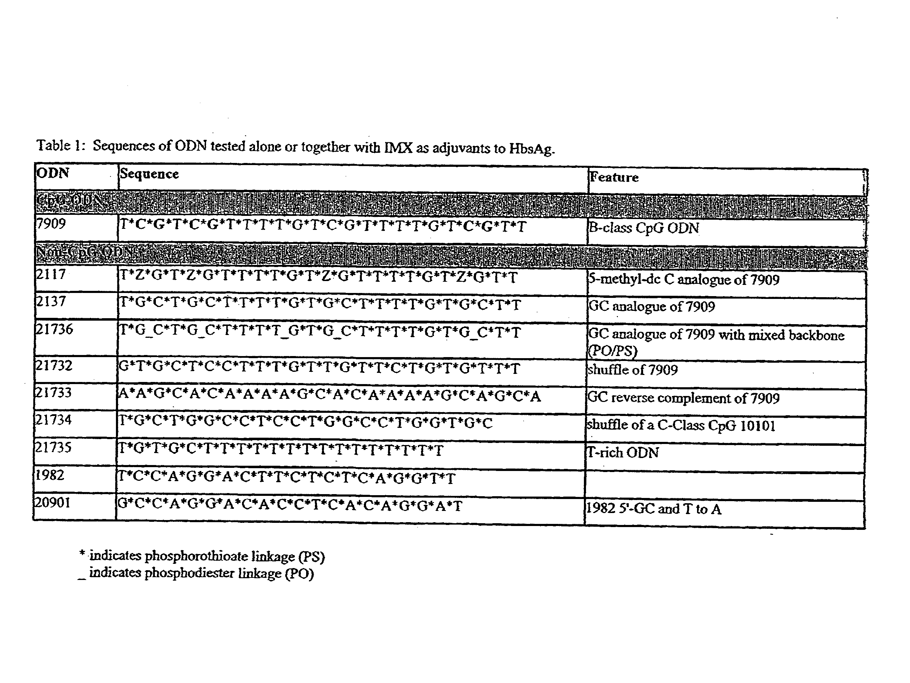

FIGS. 14-15 show the results of the cervical carcinoma experiments. Better survival and control of tumor growth in animals vaccinated with E6/E7 and CpG 7909 and IMX compared to either adjuvant alone in the C3 cervical carcinoma model.

These results suggest that suboptimal doses of either adjuvant and optionally antigen may be used in therapeutic protocols, given the overwhelming levels of IFN-gamma produced.

### Conclusions:

Virus-specific IFN-gamma is a good marker for Th1 immune responses and therefore an indicator of cellular immunity. Th1 responses are useful in a variety of prophylactic and therapeutic vaccine settings, such as for example in particular viral infections such as HIV, HBV, HSV and CMV. The levels of IFN-gamma induced in the Examples are quite surprising and indicate that immune stimulating complexes together with TLR ligands, particularly inert TLR ligands is therapeutically useful for a number of vaccine indications.

### Equivalents:

The foregoing specification is sufficient to enable one skilled in the art to practice the invention. The present invention is not to be limited in scope by examples provided, since the examples are intended as a single illustration of one aspect of the invention and other functionally equivalent embodiments are within the scope of the invention. Various modifications of the invention in addition to those shown and described herein will become apparent to those skilled in the art from the foregoing description and fall within the scope of the appended claims. The advantages and objects of the invention are not necessarily encompassed by each embodiment of the invention.

In view of the foregoing, it will be appreciated that the invention described herein *inter alia* relates to the following items:
1. A medicament, comprising (a) an oligonucleotide component, (b) an immune stimulating complex component, and (c) an antigen component, wherein said medicament induces an interferon-gamma response when administered to a vertebrate subject.
2. The medicament of item 1, wherein said interferon-gamma response induced by said medicament is greater than the interferon-gamma response induced by a second medicament that comprises (a) said oligonucleotide component and (b) said antigen component, but does not comprise an immune stimulating complex.
3. The medicament of item 1, wherein said oligonucleotide contains one or more CpG motifs.
4. The medicament of item 3, wherein said oligonucleotide is an A class CpG oligonucleotide, a B class CpG oligonucleotide or a C class CpG oligonucleotide.
Claim 5. The medicament of item 1, wherein said oligonucleotide contains one or more non-CpG motifs.
6. The medicament of item 5, wherein said oligonucleotide is a T-rich oligonucleotide, a poly-T oligonucleotide or a poly-G oligonucleotide.
7. The medicament of item 5, wherein said oligonucleotide contains at least one phosphorothioate internucleotide bridge.
8. The medicament of item 1, wherein said oligonucleotide is an inert oligonucleotide.
9. The medicament of item 1, wherein said oligonucleotide comprises a palindrome.
10. The medicament of item 1, wherein said oligonucleotide is incorporated into said immune stimulating complex.
11. The medicament of item 1, wherein said immune stimulating complex comprises saponin and sterol.
12. The medicament of item 1, wherein said antigen is a cancer antigen.
13. The medicament of item 12, wherein said antigen is cancer-specific.
14. The medicament of item 12, wherein said antigen is cancer-associated.
15. The medicament of item 1, wherein said antigen is a microbial antigen.
16. The medicament of item 1, wherein said antigen is an allergen.
17. The medicament of item 1, wherein two or more components are mixed prior to administration.
18. The medicament of item 1, wherein said oligonucleotide is an inert oligonucleotide, said immune stimulating complex comprises saponin and sterol, and said antigen is a cancer antigen.
19. A method of inducing an interferon-gamma response in a vertebrate subject, comprising the step of administering a medicament that comprises (i) an oligonucleotide component, (ii) an immune stimulating complex component, and (iii) an antigen component, to said subject, wherein said medicament induces an interferon-gamma response in said subject.
20. The method of item 19, wherein said interferon-gamma response induced by said medicament is greater than the interferon-gamma response induced by a second medicament that comprises (a) said oligonucleotide component and (b) said antigen component, but does not comprise an immune stimulating complex.
21. The method of item 19, further comprising a step of measuring the interferon-gamma response.
22. The method of item 19, wherein components (i), (ii) and (iii) are administered in combination.
23. The method of item 19, wherein components (i) and (iii) are administered separately.
24. The method of item 19, wherein said oligonucleotide contains one or more CpG motifs.
25. The method of item 24, wherein said oligonucleotide is an A class CpG oligonucleotide, a B class CpG oligonucleotide or a C class CpG oligonucleotide.
26. The method of item 19, wherein said oligonucleotide contains one or more non-CpG motifs.
27. The method of item 26, wherein said oligonucleotide is a T-rich oligonucleotide, a poly-T oligonucleotide or a poly-G oligonucleotide.
28. The method of item 26, wherein said oligonucleotide contains at least one phosphorothioate internucleotide bridge.
29. The method of item 19, wherein said oligonucleotide is an inert oligonucleotide.
30. The method of item 19, wherein said oligonucleotide comprises a palindrome.
31. The method of item 19, wherein said oligonucleotide is incorporated into said immune stimulating complex.
32. The method of item 19, wherein said immune stimulating complex comprises saponin and sterol.
33. The method of item 19, wherein said antigen is a cancer antigen.
34. The method of item 33, wherein said antigen is cancer-specific.
35. The method of item 33, wherein said antigen is cancer-associated.
36. The method of item 19, wherein said antigen is a microbial antigen.
37. The method of item 19, wherein said antigen is an allergen.
38. The method of item 19, wherein two or more components are mixed prior to administration.
39. The method of item 19, wherein said oligonucleotide is an inert oligonucleotide, said immune stimulating complex comprises saponin and sterol, and said antigen is a cancer antigen.
40. The method of item 19, wherein said medicament is administered intramuscularly or subcutaneously.
41. The method of item. 19, wherein said medicament is administered mucosally.

## Claims

1. A medicament, comprising (a) an oligonucleotide component, (b) an immune stimulating complex component, and (c) an antigen component, wherein said medicament induces an interferon-gamma response when administered to a vertebrate subject.

2. The medicament of claim 1, wherein said interferon-gamma response induced by said medicament is greater than the interferon-gamma response induced by a second medicament that comprises (a) said oligonucleotide component and (b) said antigen component, but does not comprise an immune stimulating complex.

3. The medicament of claims 1 or 2, wherein said oligonucleotide contains one or more CpG motifs.

4. The medicament of claim 3, wherein said oligonucleotide is an A class CpG oligonucleotide, a B class CpG oligonucleotide or a C class CpG oligonucleotide.

5. The medicament of any one of the preceding claims, wherein said oligonucleotide contains one or more non-CpG motifs.

6. The medicament of claim 5, wherein said oligonucleotide is a T-rich oligonucleotide, a poly-T oligonucleotide or a poly-G oligonucleotide.

7. The medicament of claim 5, wherein said oligonucleotide contains at least one phosphorothioate internucleotide bridge.

8. The medicament of any one of the preceding claims, wherein said oligonucleotide is an inert oligonucleotide.

9. The medicament of any one of the preceding claims, wherein said oligonucleotide comprises a palindrome.

10. The medicament of any one of the preceding claims, wherein said oligonucleotide is incorporated into said immune stimulating complex.

11. The medicament of any one of the preceding claims, wherein said immune stimulating complex comprises saponin and sterol.

12. The medicament of any one of the preceding claims, wherein said antigen is a cancer antigen.

13. The medicament of claim 12, wherein said antigen is cancer-specific or cancer-associated.

14. The medicament of any one of the preceding claims, wherein said antigen is a microbial antigen or an allergen.

15. The medicament of any one of the preceding claims, wherein two or more components are mixed prior to administration.

16. The medicament of any one of the preceding claims, wherein said oligonucleotide is an inert oligonucleotide, said immune stimulating complex comprises saponin and sterol, and said antigen is a cancer antigen.

17. The use of the medicament according to any one of the preceding claims for the preparation of a pharmaceutical composition for inducing an interferon-gamma response in a vertebrate subject.

18. The use of claim 17, wherein the interferon-gamma response is measured.

19. The use of claims 17 or 18, wherein components (i), (ii) and (iii) are to be administered in combination.

20. The use of any one of claims 17 to 19, wherein components (i) and (iii) are to be administered separately.

21. The use of any one of claims 17 to 20, wherein said medicament is to be administered intramuscularly, subcutaneously or mucosally.
